(19) Europäisches Patentamt / European Patent Office / Office européen des brevets

(11) **EP 1 805 477 B1**

(12) **EUROPÄISCHE PATENTSCHRIFT**

(45) Veröffentlichungstag und Bekanntmachung des Hinweises auf die Patenterteilung:
**08.04.2009 Patentblatt 2009/15**

(51) Int Cl.:
*G01B 9/02* *(2006.01)*　　*G01N 21/47* *(2006.01)*
*A61B 3/12* *(2006.01)*　　*G02B 21/00* *(2006.01)*

(21) Anmeldenummer: **05793343.4**

(22) Anmeldetag: **13.10.2005**

(86) Internationale Anmeldenummer:
**PCT/EP2005/011042**

(87) Internationale Veröffentlichungsnummer:
**WO 2006/042696 (27.04.2006 Gazette 2006/17)**

(54) **INTERFEROMETRISCHES VERFAHREN UND ANORDNUNG**

INTERFEROMETRIC METHOD AND ARRANGEMENT

ENSEMBLE ET PROCEDE INTERFEROMETRIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **20.10.2004 DE 102004052205**
**03.02.2005 DE 102005006724**
**05.09.2005 DE 102005042733**

(43) Veröffentlichungstag der Anmeldung:
**11.07.2007 Patentblatt 2007/28**

(73) Patentinhaber: **Universität Stuttgart**
**70174 Stuttgart (DE)**

(72) Erfinder:
• **KÖRNER, Klaus**
**15566 Schöneiche (DE)**
• **BERGER, Reinhard**
**73547 Lorch (DE)**
• **DROSTE, Ulrich**
**70193 Stuttgart (DE)**
• **KERWIEN, Norbert**
**73563 Mögglingen (DE)**
• **KOHLER, Christian**
**74193 Schwaigern (DE)**
• **OSTEN, Wolfgang**
**28865 Lilienthal (DE)**

• **PAPASTATHOPOULOS, Evangelos**
**71229 Leonberg (DE)**
• **PRUSS, Christof**
**70839 Gerlingen (DE)**
• **RUPRECHT, Aiko**
**22880 Wedel (DE)**
• **WIESENDANGER, Tobias**
**69231 Rauenberg (DE)**

(74) Vertreter: **Müller-Boré & Partner**
**Patentanwälte**
**Grafinger Strasse 2**
**81671 München (DE)**

(56) Entgegenhaltungen:
DE-A1- 4 405 450　　DE-A1- 10 321 895
GB-A- 2 355 310　　US-B1- 6 480 285

• TIZIANI H J ET AL: "THREE-DIMENSIONAL IMAGE SENSING BY CHROMATIC CONFOCAL MICROSCOPY" APPLIED OPTICS, OSA, OPTICAL SOCIETY OF AMERICA, WASHINGTON, DC, US, Bd. 33, Nr. 10, 1. April 1994 (1994-04-01), Seiten 1838-1843, XP000434202 ISSN: 0003-6935 in der Anmeldung erwähnt

EP 1 805 477 B1

**Beschreibung**

Die vorliegende Erfindung betrifft ein Verfahren und eine Anordnung zur konfokalen Spektral-Interferometrie, insbesondere auch zur optischen Kohärenz-Tomographie (OCT) und/oder optischen Kohärenz-Mikroskopie (OCM) von biologischen und technischen Objekten in Auf- und/oder Durch licht.

Eine zwei- und dreidimensionale Erfassung des äußeren Profils und der äußeren Form sowie auch des Tiefenprofils, also die Struktur in der Tiefe, von biologischen Objekten erfolgt beim Stand der Technik noch mit einer recht geringen Geschwindigkeit und mit einer recht geringen Auflösung in der Tiefe.

Die Gewinnung von Signalen für die zwei- und dreidimensionale Erfassung des äußeren Profils und der äußeren Form mittels konfokaler Technik von Objekten kann in recht guter Näherung beugungsbegrenzt erfolgen. Damit stellt die konfokale Technik einen tragfähigen Ansatz zur Erfassung der Geometrie, also des Abstandes einer Grenzschicht oder einer Oberfläche eines biologischen oder technischen Objekts sowie auch der Form oder des Profils desselben dar. Andererseits ist die konfokale Technik in der Regel für unbehandelte biologische Materialien in einigen Fällen nur bedingt geeignet, wenn diese eine nur sehr geringe Reflektivität aufweisen, dafür aber in der Regel im Volumen Lichtstreuer sind.

Die konfokale Technik in Verbindung mit der Interferometrie ist aus Gründen der Sensitivität dafür grundsätzlich besser geeignet. Unter dem Begriff Optische Kohärenz-Tomografie (OCT) wird im Allgemeinen die konfokale Technik in Verbindung mit der Interferometrie verstanden. Dabei wird durch den Tiefen-Scan, auch als A-Scan bezeichnet, der in der Regel im Referenzstrahlengang des Interferometers durchgeführt wird, die benötigte Gangunterschiedsvariation erzeugt.

Die Weißlicht-Interferometrie mit spektraler Auswertung, auch als Fourier-Domain OCT oder Spektral-Interferometrie bekannt, siehe auch M. W. Lindner, P. Andretzky, F. Kiesewetter und G. Häusler. Spectral Radar: Optical Coherence tomography in the Fourier Domain, in Handbook of Optical Coherence Tomography, Editoren: Bouma, Brett, E.; Tearney, Guillermo, J., Marcel Dekker, Inc., New York, Basel, 2002, ISBN-Nummer 0-8247-0558-0, [1], S. 335-345, ist wegen der vergleichsweise guten Dynamik der detektierbaren Signale ebenfalls viel besser als die ausschließlich konfokale Technik geeignet, ein größeres Objektvolumen in der Tiefe auszulesen. Das Problem stellt die vergleichsweise geringe NA des Fokussier-Objektivs dar.

Ein weiteres Problem bei der Optischen Kohärenz-Tomografie (OCT) oder auch bei der optischen Kohärenz-Mikroskopie (OCM) stellt die sphärische Aberration im Objektvolumen dar, die bei hochaperturigen Fokussier-Objektiven den nutzbaren Tiefenbereich im Volumen stark begrenzen kann, siehe hierzu auch Stephen R. Chinn und Eric A. Swanson: "Optical Coherence Tomography for High-Density Data Storage" in [1] Kapitel 14, S. 408. Hier wird dargestellt, daß ein Festobjektiv mit einer numerischen Apertur von 0,55 bei einer Wellenlänge von 780 nm trotz Nachfokussierung wegen der sphärischen Aberration nur in einem Tiefenbereich von etwa +/-100 $\mu$m um die mittlere Fokusposition von 1,2 mm genutzt werden kann, ohne daß eine Signatdegradation durch Wellenfrontdeformationen auftritt. Dabei könnte bezüglich der Sensitivität ein konfokal-interferometrisches Verfahren bei geeigneter Ausführung des optischen Tasters durchaus einen Tiefenbereich von 1 mm im Objektvolumen erfassen. Dies erfordert dann jedoch zur Kompensation der sphärischen Aberration eine aktive Optik, welche auch als dynamische optische Kompensation bekannt ist. Diese aktive Optik erhöht die Komplexität des Systems jedoch sehr stark und verschlechtert die zeitliche Dynamik desselben erheblich.

Der bekannte Ansatz der optischen Kohärenz-Mikroskopie (OCM) ermöglicht mittels konfokal-interferometrischer Verfahren wegen der vergleichsweise hohen numerischen Apertur der eingesetzten Optik in der Regel nur das Auslesen eines in der Tiefe recht begrenzten Bereiches, auf welche die elektromagnetische Strahlung zur Erfassung gerade fokussiert wird. Damit ist kein simultanes Erfassen von Informationen aus einem größeren Tiefenbereich möglich.

Ansätze zur chromatisch-konfokalen Mikroskopie wurden bereits von H. J. Tiziani und H.-M. Uhde im Fachartikel Three-dimensional image sensing by chromatic confocal microscopy in Applied Optics, Vol. 33, No. 1. April 1994, S. 1838 bis 1843 dargestellt. Mit diesem Ansatz kann auf den mechanischen Tiefen-Scan verzichtet werden.

Das sequenzielle Aufnehmen von Daten aus verschiedenen Tiefen des Objektraumes mittels Durchfokussierung spielt bei der mikroskopischen Weißlicht-Interferometrie bekannterweise eine funktionstragende Rolle., wie dies beispielsweise in den nachfolgend aufgeführten Dokumenten

-    Balasubramanian N: Optical system for surface topography measurement. US Patent. No. 4.340.306 (1982),

-    Kino GS, Chim S: Mirau correlation microscope. Appl. Opt. 29 (1990) 3775-3783,

-    Byron SL, Timothy CS: Profilometry with a coherence scanning microscope. Appl. Opt. 29 (1990) 3784-3788,

-    Dresel Th, Häusler G, Venzke H: Three-dimensional sensing of rough surfaces by coherence radar. Appl. Opt. 31 (1992) 919-925,

- Deck L, de Groot P: High-speed noncontact profiler based on scanning white-light interferometry. Appl. Opt. 33 (1994) 7334-7338 und

- Windecker R, Haible P, Tiziani H J: Fast coherence scanning interferometry for measuring smooth, rough and spherical surfaces. J. Opt. Soc. Am 42 (1995) 2059-2069 weiter ausgeführt ist.

Es ist jedoch bei der mikroskopischen Weißlicht-Interferometrie von Vorteil, die Informationen über das Objekt ohne das Bewegen von mechanischen Teilen zu gewinnen. Ein erster Ansatz findet sich bei Hege G: Speckleverfahren zur Abstandsmessung. Dissertation, in Berichte aus dem Institut für Technische Optik. Vol. 4. 1984, S. 20-25. Jedoch kann es bei Objekten mit einer größeren Tiefenausdehnung Probleme mit der Schärfetiefe bei hochaperturiger Beobachtung geben.

In der Veröffentlichung "Dispersive interferometric profilometer" von J. Schwider und L. Zhou in Opt. Lett. Vol. 19. No 13, 1994 wird ein interferometrisches System vorgeschlagen, das ein Interferometer und ein Spektrometer verknüpft. Das Interferenz-Signal wird dabei mit Hilfe eines Gitters spektral aufgespalten. Jedoch ist hierbei der Tiefenmessbereich durch die numerische Apertur begrenzt.

Eine andere Möglichkeit bei der mikroskopischen Weißlichtinterferometrie ohne das Bewegen von mechanischen Teilen zu fokussieren, wurde mit der Wavelength-todepth-encoding-Technik von G. Li, P.-Ch. Sun, P. C. Lin und Y. Feinman in Optics Letters 15. Okt. 2000, Vol. 25, No. 20, S. 1505 bis 1507 mit einer diffraktiven Linse im Objektstrahlengang in Verbindung mit einem durchstimmbaren Laser vorgeschlagen. Hierbei ist der verwendete Messaufbau noch insgesamt recht aufwendig. Weiterhin gibt es jedoch noch Verbesserungspotenzial, da durch die Variation der Brennweite eines Objektivs im Objektstrahlengang zur Durchfokussierung sich auch der Abbildungsmaßstab ändert, da ein Farbvergrößerungsfehler bei der Abbildung des Objektes besteht. Dies kann für die hochauflösende 3D-Topometrie ein Problem darstellen.

In der Veröffentlichung von Peter Hlubina: "Measuring distances and displacements using dispersive white-light spectral interferometry" in SPIE 5144, S. 628-636 wird eine Anordnung zur Messung von Abständen dargestellt. Dabei wird durch Dispersion in einem Arm eines Weißlichtinterferometers, wobei der optische Gangunterschied ungleich null ist, ein spektral auswertbares Signal erzeugt. Jedoch ist mit einem derartigen Ansatz keine hohe laterale Auflösung erreichbar.

Ein weiterer Ansatz zur Vermeidung des mechanischen Scans wurde bereits in C. Bosbach, F. Deprieux, T. Pfeifer, B. Michelt: Proc. SPIE 4900 (2002) 408-415 gezeigt. Dies erfolgt jedoch mit einem recht komplexen Aufbau unter Verwendung eines zweiten Interferometers mit einem Stufenspiegel. Der Objektabstand kann jedoch nur innerhalb der wellenoptischen Schärfentiefe des Sensorkopfes, die durch die numerische Apertur des Objektivs desselben bestimmt ist, ermittelt werden und ist somit insbesondere für eine hohe numerische Apertur sehr begrenzt. Eine hohe numerische Apertur ist jedoch für eine hohe laterale Auflösung bei der Messung eines Objektes unverzichtbar.

In der Veröffentlichung "Accurate fiber-optic sensor for measurement of the distance based on white-light interferometry with dispersion" von Pavel Pavlicek und Gerd Häusler in ICO Tokyo, Paper-Nr. 15B3-1 vom 15.7.2004 wird eine Anordnung beschrieben, bei der in einer Faser im Referenzarm eines Interferometers mittels Dispersion ein über der Wellenzahl intensitätsmoduliertes Signal erzeugt wird. Der Objektabstand kann jedoch auch hier nur innerhalb der wellenoptischen Schärfentiefe des Sensorkopfes, die durch die numerische Apertur des Objektivs desselben bestimmt ist, ermittelt werden und ist somit insbesondere für eine hohe numerische Apertur sehr begrenzt. Die konfokale Diskriminierung des Objektstrahlenbündels erfolgt hierbei mittels des Endes der Faser im Objektstrahlengang des Faseroptik-Interferometers.

In der Veröffentlichung "Fiber-optic in-focus OCT" von Masato Ohmi, Akihiro Kakimoto, und Masamitsu Haruna in ICO Tokyo, Paper-Nr. 15BD-3 vom 13.7.2004 wird eine Anordnung beschrieben, bei der die Fokussierung für die optische Kohärenz-Tomografie (Optical coherence tomography, OCT) an biologischen Proben durchgeführt wird. Hierbei ist jedoch ein mechanisches Scannen sowohl im Referenzarm des Interferometers als auch im Objektarm erforderlich. Besonders das Scannen im Objektarm führt zu einem vergleichsweise großen Bauvolumen des Sensorkopfes und verhindert damit Applikationen in kleinen Kavitäten. Die konfokale Diskriminierung des Objektstrahlenbündels erfolgt auch hierbei mittels des Endes der Faser im Objektstrahlengang des Faseroptik-Interferometers.

Im Fachaufsatz "Spectral interference Mirau microscope" von Mehta, D., S.; Saito, S.; Hinosugi, H.; Takeda, M. und Kurokawa, T. in Applied Optics, Vol. 42, No. 7, 1. März 2003, Seiten 1296 bis 1305 wird ein Verfahren beschrieben, bei dem zu Lasten der lateralen Auflösung die Meßfeldgröße erhöht wird. Es wird ein relativ großer Tiefenmeßbereich mit Submikrometer-Tiefenauflösung erreicht. In der beschriebenen Anordnung müssen zur Realisierung oder Meßfunktion akusto-optische, durchstimmbare Filter eingesetzt werden, die jedoch recht kostenaufwendig sind. Dies stellt somit keine, technische Lösung für einen kostengünstigen Sensor dar.

Im Fachaufsatz "Spatio-spectral digital holography for full-field tomografic imaging with adaptive focusing" von M. Pawlowski, Y. Sakano, Y. Miyamoto, M. Takeda und K. Obayashi in Proc. Of SPIE Vol. 5531, Seiten 121 bis 126 wird ein holografisches Verfahren beschrieben, bei dem Objektinformationen außerhalb der Fokustiefe bei der mikroskopischen Abbildung adaptiv numerisch rekonstruiert werden, indem monochromatische Wellen propagiert werden. Es ist für die 3D-Erfassung eines Objekts kein mechanisches Scannen erforderlich. Auch in dieser Anordnung müssen zur Realisierung der Meßfunktion ebenfalls akusto-optische, durchstimmbare Filter eingesetzt werden.

In der Offenlegungsschrift DE 10321895 A1 "Interferometrischer Sensor zur chromatischen Objekt-Tiefenabtastung" -von K. Körner und W. Osten ist bei einem Spektral-Interferometer in der Fourier-Ebene des zugehörigen Abbildungssystems ein spektral brechkraftvariables Element eingeführt, welches die gewünschte chromatische Längsaufspaltung oder Tiefenaufspaltung des Objektbündels im Objektraum erzeugt. Es wird dort auch die Anwendung eines Mirau-Interferometers mit Kompensation der chromatischen Tiefenaufspaltung mittels chromatischer Brechkraft im Referenzstrahlengang beschrieben. Die mechanische Zugänglichkeit der Fourier-Ebene ist insbesondere bei hochaperturigen Mikroskopobjektiven, die für Mirau-Interferometer eingesetzt werden, jedoch nicht immer gegeben. Wesentlich ist jedoch, daß beim Stand der Technik bei einem Mirau-Interferometer mit einer numerischen Apertur von beispielsweise 0,55 nur schwer eine beugungsbegrenzte Abbildung sowohl für Referenz- als auch für Objektbündel erreicht werden kann, wenn sowohl eine chromatische Tiefenaufspaltung im Objektraum von beispielsweise 20 $\mu$m, als auch ein optischer Gangunterschied im Interferometer von beispielsweise nur 5 $\mu$m bis 50 $\mu$m, erreicht werden soll, um eine, für die problemlose Auswertung hinreichend kleine Ortsfrequenz der Müllerschen Streifen zu erzielen.

In der Veröffentlichung von E. Papastathopoulos, K. Körner und W. Osten "Chromatic Confocal Spectral Interferometry", Proceedings of Fringe 2005, Editor W. Osten, Stuttgart, Springerverlag 2005, wobei dieser Fachaufsatz am 11. 9. 2005 veröffentlicht werden wird, ist das Verfahren der Spektral-Interferometrie mit chromatischer Tiefenaufspaltung des Objektbündels im Objektraum am Beispiel eines Linnik-Interferometers dargestellt. Das Linnik-Interferometer ermöglicht durch die Anwendung eines zweiten Abbildungssystems für den Referenzstrahlengang die Einstellung eines hinreichend kleinen optischen Gangunterschiedes, einschließlich des Gangunterschiedes null, indem das zweite Abbildungssystem gemeinsam mit dem Referenzspiegel zur Justierung verschoben wird. Dabei kann die im Ergebnis dieser Verschiebung im Feld dann auftretende optische Gangunterschiedsvariation durch Haidinger-Ringe bei Bedarf numerisch korrigiert werden. Das Linnik-Interferometer ist jedoch prinzipbedingt recht komplex, großvolumig und schwer zu justieren und somit eher für Applikationen im Meßlabor geeignet.

GB 2 355 310 A offenbart ein opto-elektronisches Instrument zum Abstandmessen und/oder zum Messen einer Dicke eines transparenten Objekts, wobei hierzu eine Lichtquelle mit kurzer Kohärenzlänge und breitem Spektrum verwendet wird, sowie ein Photodetektor und eine Vielzahl optischer Elemente, welche ausgelegt sind, das Licht zu dem vermessenden Objekt hinzuführen und das reflektierte Licht in den Photodetektor einzuführen. Hierbei wird anhand von Strahlteilern das Licht in Meßstrahl und Referenzstrahl unterteilt und beide Strahlen dem Photodetektor zugeführt.

US 6 480 285 B1 offenbart eine Vorrichtung bzw. ein Verfahren zum Herstellen eines Abbilds eines Bereichs einer Substanz bzw. eines Gegenstands, in welchem eine Information gespeichert ist, wobei unterschieden wird zwischen einem Abbild, das fokussiert ist und einem Abbild, das nicht fokussiert ist, um somit Fehler in der Bilderstellung zu vermeiden bzw. zu verringern.

Eine Aufgabe der Erfindung betrifft die Erhöhung der lateralen Messgenauigkeit bei einem großen Tiefenmessbereich insbesondere bei der mikroskopischen Weißlicht-Interferometrie oder der Kohärenz-Tomografie (Optical coherence tomography, OCT) für biologische Proben mit einem vergleichsweise kostengünstigen und kompakten Sensoraufbau, ohne dass im Objektraum ein mechanisches Scannen zur Bestimmung des Abstandes oder des Profils, einschließlich des Tiefenprofils, des Objekts erfolgen muss.

Weiterhin soll der Abstand von lateral bewegten Objekten detektiert sowie auch Defekte auf technischen Oberflächen, wie Risse und Einschnürungen, aufgrund der Änderung der Objekttiefe im Bereich eines Defektes extrem schnell erkannt werden.

Eine weitere Aufgabe besteht darin, durch die Anwendung der Erfindung eine extreme Miniaturisierung des Sensorkopfes zu ermöglichen, auch um den Sensor für den Einsatz in der minimal-invasiven Medizin anwendbar zu machen, beispielsweise, um auch in biologischen Kavitäten die dreidimensionale Mikroform von biologischen Proben messen zu können. Letzteres kann es erforderlich machen, dass der Sensor von einem Patienten auch schluckbar ausgeführt wird. Eine weitere Applikation stellt das dreidimensionale Messen der Topografie in technischen Hohlräumen wie Innenzylinder oder Bohrungen dar.

Damit ist also im besonderen die erfinderische Aufgabe zu lösen, beim optischen Antasten der Objektoberfläche in verschiedenen Tiefen des Objektraumes optische Signale aus diesen Tiefen ohne das mechanische Bewegen von Komponenten - insbesondere im Objektraum- zu erzeugen, da diese Bewegungsmittel in der Regel ein erhebliches Bauvolumen aufweisen und der Miniaturisierung des Sensors entgegenstehen. Die mit dem erfinderischen Sensor beim Messen gewonnenen optischen Signale sollen mittels Zeiten- oder Flächenkameraerfasst werden und bei Bedarf an hoher Genauigkeit und Eignung des Messobjektes auch eine Auswertung der Phaseninformation gestatten.

Da vor allem die verfügbare Lichtenergie die Meßgeschwindigkeit begrenzt, besteht die Aufgabe, die verfügbare Lichtenergie optimal beim erfinderischen Verfahren zu nutzen.

Damit ist also die erfinderische Aufgabe zu lösen, in einem Tiefenbereich oder auch in verschieden tiefen Volumenbereichen gleichzeitig optische Merkmale, wie beispielsweise Reflexionsgrad oder Lichtstreuung, sehr schnell, räumlich aufgelöst und mit hoher Zuverlässigkeit zu erfassen. Dies soll auch bei Materialien möglich sein, die trübe Medien darstellen, also wegen der Streuung des Lichts nur sehr wenig Licht in den optischen Tastkopf gelangen lassen.

So besteht die erfinderische Aufgabe, im optischen Tastkopf auf einen mechanisch generierten Tiefen-Scan, den A-Scan, zu verzichten, da dieser eine unakzeptable Scan-Zeit erforderlich macht. Es soll jedoch dennoch in einem größeren Tiefenbereich gemessen werden können.

Weiterhin ist also auch die erfinderische Aufgabe zu lösen, eine große Robustheit im Aufnahmevorgang dadurch zu erreichen, daß der optische Tastkopf, zum Zeitpunkt der Aufnahme auch bei einer gewissen Fehllage desselben in der Tiefe, beispielsweise durch Fehleinstellungen, Vibrationen beim Erfassen und bei unerwünschten Patientenbewegungen bei der Diagnose noch vergleichsweise zuverlässig Signalwerte liefern kann.

Damit ist also im Besonderen auch die erfinderische Aufgabe zu lösen, beim Erfassen eines Objekts, welches auch ein trübes Medium darstellen kann, auch in einer Tiefe von beispielsweise einigen 100 $\mu$m oder in verschieden tiefen Volumenbereichen gleichzeitig optische Merkmale sehr schnell räumlich zu erfassen.

Das Verfahren soll eine große Flexibilität aufweisen, um an die jeweilige Aufgabe optimal angepaßt arbeiten zu können. Dagegen sollen Manipulationen am Tastkopf bis auf den gegebenenfalls auszuführenden Lateral-Scan im Interesse einer hohen Meßgeschwindigkeit eher unterbleiben können.

Die verallgemeinerte Aufgabe der Erfindung lautet also: Es soll von Details einer Objektoberfläche und/oder von Details des Objektinneren jeweils eine (z) oder zwei (x, z) oder auch drei Raumkoordinate/n (x, y, z) bestimmt sowie jeweils die zugehörige Lichtintensität in einem definierten Spektralbereich möglichst schnell erfaßt werden. Die Lichtintensität wird zum einen von der Größe der Reflektivität, bzw. der Streuung des erfaßten Details bestimmt, andererseits von allen optischen Komponenten und allen Details des gesamten Lichtweges, der dem Detail im Lichtfluss vor- und nachgeordnet ist.

Die obigen Aufgaben werden durch das Verfahren gemäß dem unabhängigen Anspruch 1 und die Anordnung gemäß dem unabhängigen Anspruch 16 gelöst. Bevorzugte Ausführungsvarianten bzw. bevorzugte Ausführungsformen sind Gegenstand der abhängigen Ansprüche.

Vorteilhafterweise können für die gewerbliche Nutzung Objekte, insbesondere auch biologische und technische Objekte, vergleichsweise schnell in ihrer äußeren Form oder ihres äußeren Profils und/oder auch zumindest teilweise in ihrer Dreidimensionalität im Inneren des Objektvolumens, also in einem Bereich unter der Oberfläche des Objekts, vermessen werden.

Hierbei sind weiterhin vorteilhafterweise möglichst hochaufgelöste 3D-Erfassen von eher mikroskopisch kleinen Objekten, also eine Auflösung in den drei Raumkoordinaten nahe an der Begrenzung durch die Beugung elektromagnetischer Wellen möglich, wobei ein vergleichsweise ausgedehnter Tiefenbereich ebenfalls erfaßt wird. Es können im Extremfall - beim Einsatz hochaperturiger Optik und beispielsweise bei Anwendung der Immersionstechnik - Auflösungen auch in der Größenordnung der verwendeten Wellenlänge in den drei Raumkoordinaten erzeugt werden.

Insbesondere können die zu vermessenden Objekte auch translucent sein. Dabei kann es sich auch um technische Wertstoffe wie Kunststoffe handeln, die beispielsweise faserverstärkt sind oder Füllstoffe in verschiedenen Partikelgrößen aufweisen. Dabei steht die Erfassung der zwei- oder auch dreidimensionalen Verteilung dieser Inhaltsstoffe im Basismaterial im Vordergrund.

Ein weiterer Vorteil besteht darin, Schichtdicken zu messen, beispielsweise von Lackschichten, optischen Schichten oder auch von Ölfilmen sowie auch die Topografie von unter der Schicht liegenden Oberflächen. Auch die räumliche Verteilung von Partikeln in der Schicht selbst kann von Interesse sein.

Dabei kann mit dem erfinderischen Verfahren ein möglichst großer Datenfluß mit dem optischen Tastkopf erreicht werden.

Das erfinderische Verfahren kann auch in der Präventivmedizin eingesetzt werden, beispielsweise zur Früherkennung von Krankheiten. Um möglichst viel, Lichtenergie vom Objekt gewinnen und damit auch schnell messen zu können, soll im optischen Tastkopf vorzugsweise der Einsatz recht hochaperturiger Fokussieroptik, beispielsweise mit einer numerischen Apertur von 0,5, durch die Anwendung der Erfindung möglich sein.

Ein weiterer Vorteil besteht auch darin, für diese Messung einen kompakten optischen Tastkopf einsetzen zu können.

Ferner besteht ein Vorteil auch darin, mit dem optischen Tastkopf eine vergleichsweise große Robustheit gegenüber mechanischen Einflüssen - wie auch Stößen - beim Erfassen von Objekten, insbesondere auch von Biomaterialien, zu erreichen.

Ein weiterer Vorteil besteht insbesondere darin, vergleichsweise geringe Herstellungskosten sowie eine hohe Produktlebensdauer für den optischen Tastkopf zu erreichen.

Insbesondere besteht ein Vorteil darin, am Patienten bei einem chirurgischen Eingriff die Messungen in möglichst kurzer Zeit durchzuführen.

Vorteilhafterweise kann die Erfindung zur spektralen Zweistrahl-Interferometrie mit chromatischer Tiefenaufspaltung für vielfältige Aufgaben in der interferometrischen Technik insbesondere für Prüfungs- und Detektionsaufgaben eingesetzt werden. Insbesondere werden folgende Applikationen für die Erfindung vorgeschlagen: Optische Coherence Tomography (OCT), Optische Coherence Microscopy (OCM) für biologische und technischen Anwendungen, Erfassung des Tiefenprofils, des Brechzahlprofils, der optischen Dicke, der Schichtdicke sowie auch der Reflektivität eines Objekts. Weiterhin kann die erfinderische Lösung auch zur optischen Datenauslesung genutzt werden, indem die Größe der Intensität der von den Pits und Lands einer datentragenden Schicht eines optischen Datenträgers zurückkommenden Strahlung detektiert wird.

Weiterhin vorteilhafterweise kann durch Anwendung der Erfindung der Abstand von lateral bewegten Objekten detektiert werden sowie auch Defekte auf technischen Oberflächen, wie Risse, Einschnürungen und Mikrolöcher in Schweißnähten, aufgrund der Änderung der Objekttiefe im Bereich eines Defektes schnell erkannt werden.

Die Erfindung kann vorteilhafterweise auch für die Erfassung des Abstandes, des Profils, der Mikroform oder des Brechungsindexes von biologischen Proben, beispielsweise in biologischen Kavitäten eingesetzt werden. Dabei kann auch mit Immersionstechniken gearbeitet werden.

Weitere Vorteile der Erfindung werden nachfolgend dargestellt:

Vorteilhafterweise wird die Flexibilität hinsichtlich der Positionierung der Tiefe des nutzbaren Tiefenmeßbereiches im Objektraum erhöht als auch die Tiefe des nutzbaren Tiefenmeßbereiches vergrößert, insbesondere bei Verwendung von einem Abbildungssystem mit einer vergleichsweisen hohen numerischen Apertur, beispielsweise im Bereich von 0,4 bis 0,8.

Insbesondere kann auch ein einfacher, vergleichsweise robuster und kostengünstiger Ein-Punkt-Sensor aufgebaut werden können, ohne daß im Objektraum ein mechanisches Scannen erfolgen muß. Dabei können insbesondere auch sehr kostengünstige, fasergekoppelte Spektrometer und fasergekoppelte Superluminineszenz-Dioden zum Einsatz kommen.

Durch die Anwendung der erfinderischen Lehre ermöglicht eine wesentliche Miniaturisierung des Sensorkopfes im Vergleich zum Linnik-Interferometer und macht auch um beispielsweise den Sensor für den Einsatz in der minimalinvasiven Medizin oder in der Bohrlochinspektion anwendbar.

Vorteilhafterweise werden die mit dem Sensor beim Messen gewonnenen optischen Signale mittels Strahlungsdetektor oder gerastertem Strahlungsdetektor wie eine Zeilen- oder eine Flächenkamera erfaßt. Anstelle eines Spektrometers kann auch eine durchstimmbare Lichtquelle verwendet werden.

Weiterhin vorteilhafterweise werden beim optischen Antasten der Objektoberfläche in verschiedenen Tiefen des Objektraumes optische Signale aus diesen Tiefen ohne das mechanische Bewegen von Komponenten - insbesondere im Objektraum - gewonnen werden. Dabei kann eine zumindest näherungsweise beugungsbegrenzte laterale Abbildung des Objekts durch ein Abbildungssystem mit vergleichsweise hoher numerischer Apertur erfolgen, welches auch von Referenzstrahlung durchsetzt wird. Es kann also eine erfinderische Anordnung ohne Anwendung eines zweiten Abbildungssystems für Referenzstrahlung aufgebaut werden.

Ein weiterer Vorteil besteht darin, für spektrale Zweistrahl-Interferometer mit chromatischer Tiefenaufspaltung zur Vergrößerung des Tiefenmeßbereiches mit einem Abbildungssystem für das Objekt, wobei das Abbildungssystem für spektral breitbandige Strahlung ausgelegt ist und welches gleichzeitig auch von Referenzstrahlung durchsetzt wird, die Einstellung eines hinreichend kleinen optischen Gangunterschiedes zu ermöglichen. Hierbei ist auch der optische Gangunterschiedes null eingeschlossen. Dies kann auch bei vergleichsweise großer chromatischer Tiefenaufspaltung erreicht werden, insbesondere auch für ein Mirau-Interferometer, aber auch beispielsweise für ein Michelson-, Twyman-Green- oder ein Mach-Zehnder-Interferometer angewendet werden können.

Weiterhin vorteilhafterweise ist der Abstand $z_i$ eines scharf abgebildeten Objektpunktes $P_i$ im Tiefenmeßbereich Dz_c, der durch chromatische Tiefenaufspaltung im Abbildungsstrahlengang für Objektbündel gegeben ist, von der Fläche (G) gleichen optischen Gangunterschieds im Objektraum so einzustellen, daß ein hinreichend kleiner optischer Gangunterschied im Zweistrahl-Interferometer besteht, der zu Müllerschen Streifen in einem Spektrometer führt, die hinsichtlich ihrer Frequenz über der Wellenlänge technisch gut auswertbar sind.

Andererseits können beim Wellenlängen-Scan mittels einer zeitlich durchstimmbaren, monochromatischen Strahlungsquelle elektromagnetische Interferenzmodulationen über der Zeit erzeugt werden, die eine hinreichend kleine und damit technisch gut auswertbare Frequenz aufweisen. Dies kann insbesondere für ein Mirau-Interferometer gelten, welches, sich durch seine Einfachheit, sein vergleichsweise geringes Volumen und auch seine hohe Stabilität und Robustheit bei vielen Applikationen bereits einen festen Platz erobert hat. Dabei wird sich der Trend zur Anwendung des Mirau-Interferometers als optischer, kompakter Sensor in der Zukunft wohl noch verstärken.

Ein Aspekt der vorliegenden Erfindung betrifft ein Verfahren zur konfokalen Spektral-Interferometrie, insbesondere auch zur Erfassung des Abstandes, des Profils und der Form und auch zur Optischen Kohärenz-Tomografie (OCT) und/oder optischen Kohärenz-Mikroskopie (OCM) von biologischen und technischen Objekten oder Objektdetails in Auf- und/oder Durchlicht mit

- entweder mindestens einer Multiwellenlängen-Quelle elektromagnetischer Strahlung (1, 1a), wobei bei Nutzung der Multiwellenlängen-Quelle dem gerasterten Empfänger (111a) ein dispersives Spektrometer (100) vorgeordnet ist,

- oder mindestens einer Wellenlängen-durchstimmbaren Quelle elektromagnetischer Strahlung und mit mikroskopischer Abbildung der Objektoberfläche und/oder des Inneren des Objektsvolumens mittels Fokussier-Systems auf einen gerasterten Empfänger (111a) elektromagnetischer Strahlung zur Detektion der interferierenden elektromagnetischen Strahlung,
gekennzeichnet dadurch,
daß die chromatisch-konfokale Technik mit der spektralen Zweistrahl-Interferometrie verfahrensmäßig verbunden wird, wobei sowohl eine vorbestimmte chromatische Tiefenaufspaltung der elektromagnetischen Strahlung im Fokussier-System erfolgt als auch

- entweder bei Nutzung der Multiwellenlängen-Quelle eine Spektralanalyse der detektierten interferierenden elektromagnetischen Strahlung mittels dispersivem Spektrometer durchgeführt wird, so daß ein Spektrum zur Verfügung steht,

- oder bei Nutzung der Wellenlängen-durchstimmbaren Quelle eine Wellenlängendurchstimmung vorbestimmt durchgeführt wird und die interferierende elektromagnetische Strahlung bei der Wellenlängendurchstimmung detektiert wird, so daß über der Zeit ein Spektrum aufgenommen wird,

und aus dem Spektrum über die Kenntnis der vorbestimmten Tiefenaufspaltung im chromatisch-konfokaten System und die zumindest näherungsweise Kenntnis des Brechungsindexes die z-Position eines jeden Objektdetails bestimmt wird. Weitere Ausgestaltungen der Erfindung können umfassen : Verfahren zur konfokalen Spektral-Interferometrie gemäß der Erfindung,
wobei bei Vorhandensein eines reflektierenden oder lichtstreuenden Bereiches, also einem Objektdetail, im Objektvolumen (208) im Abtastvorgang mindestens ein Wavelet über der spektralen Achse des Spektrometers (100) erzeugt wird, dessen Halbwertsbreite durch die Dimensionierung der chromatischen Tiefenaufspaltung vorbestimmt ist, und die Anzahl der Perioden unter der Einhüllenden des Wavelets, die durch die Dimensionierung von optischem Gangunterschied und von der Gangunterschiedsänderung über der Wellenlänge vorbestimmt ist, einstellbar gemacht ist.

Verfahren zur konfokalen Spektral-Interferometrie gemäß einer bevorzugten Ausführungsform der Erfindung wobei vorzugsweise das Verfahren bei einem optischen Gangunterschied Dx ungleich null im Interferometer durchgeführt wird.

Verfahren zur konfokalen Spektral-Interferometrie gemäß einer bevorzugten Ausführungsform der Erfindung wobei sich vorzugsweise durch die chromatische Längsaberration, beziehungsweise die chromatische Tiefenaufspaltung, die Foki in unterschiedlichen Tiefen des Objektvolumens (208) ausbilden, wobei für jeden Fokus jeder beliebigen Wellenlänge im genutzten Spektralbereich, die sphärische Aberration durch eine vorbestimmte Einstellung im Fokussier-System ein Minimum aufweist, so daß für den Fokusfleck in jeder berücksichtigten Tiefe im Objektvolumen (208) in guter Näherung eine beugungsbegrenzte Abbildung besteht.

Verfahren zur konfokalen Spektral-Interferometrie gemäß einer bevorzugten Ausführungsform der Erfindung wobei vorzugsweise die Signalamplitude von Wavelets ausgewertet wird.

Verfahren zur konfokaten Spektral-Interferometrie gemäß einer bevorzugten Ausführungsform der Erfindung wobei vorzugsweise der Schwerpunkt der Einhüllenden von Wavelets ausgewertet wird.

Verfahren zur konfokalen Spektral-Interferometrie gemäß einer bevorzugten Ausführungsform der Erfindung, wobei vorzugsweise die Größe des Maximums der Einhüllenden des Wavelets ausgewertet wird.

Verfahren zur konfokalen Spektral-Interferometrie gemäß einer bevorzugten Ausführungsform der Erfindung wobei vorzugsweise die Phase des Wavelets ausgewertet wird.

Verfahren zur konfokalen Spektral-Interferometrie gemäß einer bevorzugten Ausführungsform der Erfindung, wobei vorzugsweise in mindestens einem Arm des Interferometers durch Dispersion und /oder Diffraktion der optische Gangunterschied Dx vorbestimmt wellenlängenabhängig gemacht ist.

Verfahren zur konfokalen Spektral-Interferometrie gemäß einer bevorzugten Ausführungsform der Erfindung wobei vorzugsweise zur Eliminierung der tiefenabhängigen sphärischen Aberration eine vorbestimmte wellenlängenabhängige Wellenfrontformung mittels Diffraktion oder Refraktion und Dispersion oder einer Kombination derselben durchgeführt wird.

Verfahren zur konfokalen Spektral-Interferometrie gemäß einer bevorzugten Ausführungsform der Erfindung wobei vorzugsweise eine achromatische Phasenschiebung erzeugt wird und die achromatische Phasenschiebung mehrfach erzeugt wird und nach jeder achromatischen Phasenschiebung mindestens eine weitere Aufnahme mittels des Empfängers elektromagnetischer Strahlung durchgeführt wird.

Verfahren zur konfokalen Spekfral-Interferometrie gemäß einer bevorzugten Ausführungsform der Erfindung , wobei vorzugsweise der Grad der chromatischen Tiefenaufspaltung vorbestimmt veränderbar gemacht ist.

Ein weiterer Aspekt der vorliegenden Erfindung betrifft eine Anordnung zur konfokalen Spektral-Interferometrie mit einem spektralen Zweistrahl-Interferometer, insbesondere auch zur Erfassung des Abstandes, des Profils und der Form und auch zur Optischen Kohärenz-Tomografie und/oder -Mikroskopie von biologischen und technischen Objekten in Auf- und/oder Durchlicht wobei

- die Anordnung eine Multiwellenlängen-Quelle (1, 1a) elektromagnetischer Strahlung oder einer Vielzahl von Multiwellenlängen-Quellen aufweist und wobei

- die Anordnung ein Fokussier-System auf einem gerasterten Empfänger (111a) elektromagnetischer Strahlung aufweist, welches zu einer mikroskopischen Abbildung der Objektoberfläche und/oder des Inneren des Objektvolumens ausgelegt ist, wobei dem gerasterten Empfänger (111a) ein dispersives Spektrometer (100) vorgeordnet ist,

gekennzeichnet dadurch,
daß dem spektralen Zweistrahl-Interferometer eine chromatisch-konfokale Anordnung im Abbildungsstrahlengang des Fokussier-Systems zur Objektbeleuchtung und Objektdetektion zugeordnet ist, und das Interferometer als Common-path-Interferometer ausgebildet ist. Ausgestaltungen der Erfindung können sein:

Anordnung zur konfokalen Spektral-Interferometrie gemäß einer bevorzugten Ausführungsform der Erfindung wobei vorzugsweise im Fokussier-System mindestens eine diffraktiv-optische Zonenlinse (5, 55, 105) angeordnet ist.

Anordnung zur konfokalen Spektral-Interferometrie gemäß einer bevorzugten Ausführungsform der Erfindung, wobei vorzugsweise die diffraktiv-optische Zonenlinse (5, 55, 105) in der Pupille des Fokussier-Systems angeordnet ist, welche sich in der Fourier-Ebene des Fokussier-Objektivs (6, 12, 12b) befindet.

Anordnung zur konfokalen Spektral-Interferometrie gemäß einer bevorzugten Ausführungsform der Erfindung, wobei vorzugsweise das Interferometer als fasergekoppeltes Interferometer ausgebildet ist.

Anordnung zur konfokalen Spektral-Interferometrie gemäß einer bevorzugten Ausführungsform der Erfindung wobei vorzugsweise das Interferometer als Linnik-Interferometer ausgebildet ist.

Anordnung zur konfokale Spektral-Interferometrie gemäß einer bevorzugten Ausführungsform der Erfindung, wobei vorzugsweise das Interferometer als Common-path-Interferometer ausgebildet ist.

Anordnung zur konfokalen Spektral-Interferometrie gemäß einer bevorzugten Ausführungsform der Erfindung, wobei vorzugsweise das Interferometer als Mirau-Interferometer ausgebildet ist.

Anordnung zur konfokalen Spektral-Interferometrie gemäß einer bevorzugten Ausführungsform der Erfindung, wobei vorzugsweise das Interferometer als Fizeau-Interferometer ausgebildet ist.

Anordnung zur konfokalen Spektral-Interferometrie gemäß einer bevorzugten Ausführungsform der Erfindung, wobei vorzugsweise dem Interferometer mindestens ein fasergekoppeltes Zeilen-Spektrometer (100) nachgeordnet ist.

Anordnung zur konfokalen Spektral-Interferometrie gemäß einer bevorzugten Ausführungsform der Erfindung, wobei vorzugsweise der Retroreflektor als Tripelspiegelreflektor (215) ausgebildet ist.

Anordnung zur konfokalen Spektral-Interferometrie gemäß einer bevorzugten Ausführungsform der Erfindung wobei vorzugsweise mindestens ein optisches Element im Referenzstrahlengang mit dispersivem Material angeordnet ist.

Anordnung zur konfokalen Spekfral-Interferometrie gemäß einer bevorzugten Ausführungsform der Erfindung, wobei vorzugsweise das optische Element mit dispersivem Material im kollimierten Referenzstrahlengang ausgebildet ist.

Anordnung zur konfokalen Spektral-Interferometrie gemäß einer bevorzugten Ausführungsform der Erfindung, wobei vorzugsweise im kollimierten Referenzstrahlengang ein Diffraktionsmodul 118 mit Liniengitterm angeordnet ist.

Anordnung zur konfokalen Spektral-Interferometrie gemäß einer bevorzugten Ausführungsform der Erfindung, wobei vorzugsweise das Diffraktionsmodul (118) mittels vier baugleicher und sämtlich parallel zueinander angeordneter Phasengitter (122, 123, 124, 125, 122a, 123a, 124a, 124a) aufgebaut ist.

Anordnung zur konfokalen Spektral-Interferometrie gemäß einer bevorzugten Ausführungsform der Erfindung wobei vorzugsweise das Interferometer als Mach-Zehnder-Interferometer ausgebildet ist.

Anordnung zur konfokalen Spektral-Interferometrie gemäß einer bevorzugten Ausführungsform der Erfindung wobei vorzugsweise im Interferometer sowohl der optische Gangunterschied mittels Retroreflektors als auch die optische Gangunterschiedsänderung über der Wellenlänge mittels verschiebbarer diffraktiver (122, 123, 124. 125, 122a, 123a, 124a, 125a) oder refraktiver Mittel (217a, 217b) vorbestimmt einstellbar gemacht sind.

Anordnung zur konfokalen Spektral-Interferometrie gemäß einer bevorzugten Ausführungsform der Erfindung, wobei vorzugsweise zur Eliminierung der, tiefenabhängigen sphärischen Aberration durch eine wellenlängenabhängige Wellenfrontformung diffraktive oder refraktive optische Elemente angeordnet sind oder eine Kombination derselben im chromatisch tiefenaufspaltenden Fokussier-System angeordnet ist.

Anordnung zur konfokalen Spektral-Interferometrie gemäß einer bevorzugten Ausführungsform der Erfindung, wobei vorzugsweise zur Erzeugung einer achromatischen Phasenschiebung diffraktive oder refraktive optische Elemente angeordnet sind oder eine Kombination derselben im chromatisch tiefenaufspaltenden Fokussier-System angeordnet ist.

Anordnung zur konfokalen Spektral-Interferometrie gemäß einer bevorzugten Ausführungsform der Erfindung, wobei vorzugsweise dem Fokussier-System eine achromatische adaptive Optik zugeordnet ist.

Anordnung zur konfokalen Spektral-Interferometrie gemäß einer bevorzugten Ausführungsform der Erfindung, wobei vorzugsweise die adaptive Optik als ein in das Fokussier-System integrierter, vorbestimmt steuerbarer Membranspiegel ausgebildet ist.

Anordnung zur konfokalen Spektral-Interferometrie gemäß einer bevorzugten Ausführungsform der Erfindung, wobei vorzugsweise dem Fokussier-System in der Fourier-Ebene des Fokussier-Objektivs des Fokussier-Systems oder in einer zu dieser Fourier-Ebene optisch konjugierten Ebene ein Wölb-oder Hohlspiegel zugeordnet ist, wobei die laterale Lage der optischen Achse desselben vorbestimmt steuerbar gemacht ist.

Anordnung zur konfokalen Spektral-Interferometrie gemäß einer bevorzugten Ausführungsform der Erfindung, wobei vorzugsweise der Wölb-oder Hohlspiegel als vorbestimmt steuerbares achromatisches Spiegel-Array ausgebildet ist.

Eine bevorzugte Ausführungsform der Erfindung betrifft ein Interferometrisches Multispektral-Verfahren zur hochdynamischen Objekt-Tiefenabtastung oder -Profilerfassung mit einem Zweistrahl-Interferometer mit einer punktförmigen Multiwellenlängen-Quelle elektromagnetischer Strahlung oder einer Vielzahl von punktförmigen Multiwellenlängen-Quellen am Eingang des Zweistrahl-Interferometers zur Objektbeleuchtung und mit mikroskopischer Abbildung des Objekts auf einen Empfänger elektromagnetischer Strahlung, wobei im Zweistrahl-Interferometer mittels bündelbegrenzender Mittel eine konfokale Diskriminierung erzeugt wird, wobei sich die bündelbegrenzenden Mittel zumindest näherungsweise in einer optisch konjugierten Ebene der mindestens einen der punktförmigen Multiwellenlängen-Quelle elektromagnetischer Strahlung am Eingang des Zweistrahl-Interferometers befinden und im Zweistrahl-Interferometer ein Referenzstrahlenbündel und ein Objektstrahlenbündel elektromagnetischer Strahlung erzeugt wird, gekennzeichnet dadurch,

daß im Zweistrahl-Interferometer ein diffraktiv-optisches Zonenelement (DOZE) angeordnet ist, mittels dessen sowohl elektromagnetische Strahlung in der nullten als auch in der ersten Beugungsordnung oder gegebenenfalls auch einer höheren Beugungsordnung N oder einer tieferen Beugungsordnung als der nullten in Transmission oder in Reflexion erzeugt wird

und daß das Referenzstrahlenbündel des Zweistrahl-Interferometers im Hin- und Rücklauf das diffraktiv-optische Zonenelement stets in der nullten Beugungsordnung passiert und dagegen das Objektstrahlenbündel im Hin- und Rücklauf in Bezug zum Objekt das diffraktiv-optische Zonenelement jeweils in der ersten Beugungsordnung oder gegebenenfalls jeweils einer höheren Beugungsordnung N passiert, wodurch im Objektraum durch Beugung der elektromagnetischen Strahlung eine chromatische - also eine über der Wellenlänge entstehende - Längsaufspaltung des Objektbündels in Foki erfolgt

und daß die elektromagnetische, Strahlung zumindest näherungsweise aller Wellenlängen vom Referenzstrahlenbündel $R\_0\_0$, das keine Beugung am diffraktiv-optischen Zonenelement erfährt, nach dem Passieren des diffraktiv-optischen Zonenelements die bündelbegrenzenden Mittel zur konfokalen Diskriminierung durchsetzt, während nach dem diffraktiv-optischen Zonenelement für das vom Objekt kommende Objektstrahlenbündel $0\_1\_1$ oder $O\_N\_N$ nur die Anteile die begrenzenden Mittel zur konfokalen Diskriminierung am Interferometerausgang oder im Zweistrahl-Interferometer passieren, die zumindest näherungsweise scharf auf das Objekt abgebildet waren

und daß zwischen den konfokal diskriminierten Strahlungsanteilen des Referenz-und des Objektstrahlenbündels zumindest näherungsweise Zweistrahl-Interferenz stattfindet

und daß die interferierende elektromagnetische Strahlung anschließend zur Analyse in ein Spektrometer oder in ein weiteres Interferometer geführt und auf dem Empfänger elektromagnetischer Strahlung abgebildet wird, wobei die Empfängerebene sich zumindest näherungsweise in einer optisch konjugierten Ebene zu mindestens einer der punktförmigen Multiwellenlängen-Quellen am Eingang des Zweistrahl-Interferometers befindet

und daß aus den mittels Spektrometer spektroskopisch analysiert oder einem weiteren Interferometer interferometrisch analysiert erzeugten optischen Signalen der Abstand oder das Profil des Objekts mittels bekannter Algorithmen zur Wavelet-oder Interferogramm-Analyse bestimmt wird.

Multispektrales interferometrisches Verfahren zur hochdynamischen Objekttiefenabtastung oder-Profilerfassung gemäß einer bevorzugten Ausführungsform der Erfindung wobei, das Verfahren mit einem objektabbildenden Fizeau-Interferometer mit einer teildurchlässigen Referenzspiegelfläche und mit einem zu prüfenden Objekt (8) durchgeführt wird,

und das Fizeau-Interferometer mit einem Abbildungsstrahlengang für das zu prüfende Objekt (8) und einem Punktempfänger, einem gerasterten Zeilenempfänger oder einem gerasterten Flächenempfänger für elektromagnetische Strahlung ausgebildet ist

und im Hinlauf des Lichtes von der Lichtquelle (1) zum Objekt aus dem Eingangsstrahlenbündel mittels Diffraktion in Reflexion oder Transmission an einem statischen oder dynamischen, diffraktiv-optischen Zonenelement (DOZE) (5, 105) ein gebeugtes Strahlenbündel O_N (N=1, 2, 3...oder -1, -2, -3...) in der ersten oder einer höheren Beugungsordnung N (N=1, 2, 3) oder niedrigeren Beugungsordnung N als der nullten (N=-1, -2, -3) entsteht, wobei das gebeugte Strahlenbündel O_N (N=1, 2, 3...oder -1, -2, -3...) die teildurchlässige Referenzspiegelfläche (7) durchsetzt, und

wobei das gebeugte Strahlenbündel O_N (N=1, 2, 3...oder -1, -2, -3...) nach Fokussierung auf das Objekt (8) im Objektraum chromatisch längs aufgespalten ist, so daß in Abhängigkeit von der Wellenlänge des Lichts eine Reihe oder eine Vielzahl von Reihen von Fokuspunkten in der Tiefe ausgebildet wird,

und nach Reflexion des Teilstrahlenbündels O_N (N=1, 2, 3... oder -1, -2, -3...) am Objekt (8) ein Objekt-Teilstrahlenbündel vom rückreflektierten Strahlenbündel durch Beugung am diffraktiv-optischen Zonenelement in derselben Ordnung wie im Hinlauf, nämlich O_N_N (N=1, 2, 3... oder -1, -2, -3...) gebildet wird, so daß das Objektteilstrahlenbündel O_N_N (N=1, 2, 3... oder -1, -2, -3...) nach dem Passieren des diffraktiv-optischen Zonenelements (5) wieder im Strahlengang zurückläuft,

und aus dem Eingangsstrahlenbündel im Hinlauf auf die Referenzspiegelfläche (7) auch noch ein Teilstrahlenbündel, welches im Weiteren als Referenzstrahlenbündel R_0 dient, durch Beugung in der nullten Ordnung am diffraktiv-optischen Zonenelement (5, 105) gebildet wird und im Rücklauf von der Referenzspiegelfläche (7a), auf welche das Referenzstrahlenbündel R_0 fokussiert wird,

durch Beugung auch genau wieder in der nullten Ordnung am diffraktiv-optischen Zonenelement (5, 105) ein Strahlenbündel R_0_0 gebildet wird, so daß das Referenzteilstrahlenbündel R_0_0 nach dem Passieren des diffraktiv-optischen Zonenelements wieder im Strahlengang zurückläuft,

und zwischen dem Objekt-Teilstrahlenbündel O_N_N (N=1, 2, 3... oder -1, -2, -3...) und dem Referenzstrahlenbündel R_0_0 der optische Gangunterschied ungleich null gemacht ist, wenn auf einen Objektpunkt fokussiert ist,

und das Objekt-Teilstrahlenbündel O_N_N (N=1, 2, 3... oder -1, -2, -3...) und das Referenzstrahlenbündel R_0_0 nach dem diffraktiv-optischen Zonenelement (5, 105) parallel verlaufen und zur Interferenz kommen und anschließend die beiden miteinander interferierenden Teilstrahlenbündel in den Detektionsstrahlengang eingekoppelt werden.

Multispektrales interferometrisches Verfahren zur hochdynamischen Objekttiefenabtastung oder -Profilerfassung gemäß einer bevorzugten Ausführungsform der Erfindung, wobei vorzugsweise das multispektrale interferometrische Verfahren zur hochdynamischen Objekttiefenabtastung und - profilmessung mit einem Mirau-Interferometer ausgeführt wird.

Multispektrales interferometrisches Verfahren zur hochdynamischen Objekttiefenabtastung oder -Profilerfassung, wobei vorzugsweise das multispektrale interferometrische Verfahren zur hochdynamischen Objekttiefenabtastung und - profilmessung mit einem Michelson-Interferometer ausgeführt wird.

Multispektrales interferometrisches Verfahren zur hochdynamischen Objekttiefenabtastung oder -Profilerfassung gemäß eines bevorzugten Ausführungsform der Erfindung wobei vorzugsweise eine konfokale Diskriminierung des Lichts der interferierenden Teilstrahlenbündel durchgeführt wird und die konfokal diskriminierten interferierenden Bündel O_N_N (N=1, 2, 3... oder -1, -2, -3...) und R_0_0 zur spektroskopischen Analyse eine Lateralaufspaltung mittels Dispersion oder Diffraktion erfahren und eine FFT des Intensitätsverlaufs erfolgt, um aus dem sich mittels FFT ergebenden Wertes nach Kalibrierung die Tiefenposition eines Objektpunktes zu bestimmen,

oder wobei vorzugsweise die interferierenden Bündel O_N_N (N=1, 2, 3... oder -1, - 2, -3...) und R_0_0 eine weitere Interferenzbildung mittels scannendem Zweistrahl-Interferometer (AZI) zur optischen Analyse, also mit zeitlichem Variieren des optischen Gangunterschieds, erfahren und der im scannenden Zweistrahl-Interferometer beim Scan durchfahrene optische Gangunterschied mindestens einmal genau auch dem im Interferometer zwischen Objekt-Teilstrahlenbündel O_N_N (N=1, 2, 3...oder -1, -2, -3...) und Referenzstrahlenbündel R_0_0 bestehenden optischen Gangunterschied entspricht, so daß beim Variieren des optischen Gangunterschieds ein moduliertes periodisches Signal erzeugt wird und mittels Punktempfänger, gerastertem Zeilenempfänger oder gerastertem Flächenempfänger (11, 111) über der Zeit detektiert wird

oder wobei vorzugsweise die interferierenden Bündel O_N_N (N=1, 2, 3... oder -1, - 2, -3...) und R_0_0 eine weitere Interferenzbildung mittels statischem Zweistrahl-Interferometer zur optischen Analyse, erfahren

also einem Zweistrahl-Interferometer mit lateraler Variation des optischen Gangunterschieds, obei die Orte unterschiedlichen Gangunterschieds Pixeln einer Zeilen- oder Flächenkamera (11, 111) zugeordnet sind,

und wobei vorzugsweise der im Zweistrahl-Interferometer auftretende optische Gangunterschied dem im Interferometer zwischen Objekt-Teilstrahlenbündel O_N_N (N=1, 2, 3... oder -1, -2, -3...) und Referenzstrahlenbündel R-0-0 bestehenden optischen Gangunterschied in mindestens einem Element der Zeilen- oder Flächenkamera (11, 111) zumindest näherungsweise entspricht

oder wobei vorzugsweise im Fall der Anwendung einer Wellenlängen-durchstimmbaren Quelle elektromagnetischer Strahlung beim Scan der Wellenlänge ein optisches moduliertes periodisches Signal erzeugt wird und mittels Punktempfänger, gerastertem Zeilenempfänger oder gerastertem Flächenempfänger (11, 111) über der Zeit detektiert wird und dieses über der Wellenlänge ausgewertet wird.

Beispielsweise kann das multispektrale interferometrische Verfahren zur hochdynamischen Objekttiefenabtastung oder -Profilerfassung vorzugsweise durch Beugung in der ersten Ordnung in Transmission an einer diffraktiv-optischen Phasengitter-Zonenlinse erfolgen, so daß zumindest näherungsweise nur die nullte und die erste Beugungsordnung entstehen und zwar als Referenz- und als Objekt-Teilstrahlenbündel, die sich koaxial zueinander ausbreiten.

Beispielsweise kann in dem multispektralen interferometrischen Verfahren zur hochdynamischen Objekttiefenabtastung oder -Profilerfassung vorzugsweise die diffraktiv-optische Phasengitter-Zonenlinse als zerstreuende Linse (5, 105) wirken.

Eine bevorzugte Ausführungsform der vorliegenden Erfindung betrifft ein multispektrales interferometrisches Verfahren zur hochdynamischen Objekttiefenabtastung und - Profilerfassung mit einem objektabbildenden Mirau-interferometer mit einem internen Strahlteiler, einer internen Referenzspiegelfläche (15) und mit einem zu prüfenden Objekt (8),

wobei dem Mirau-Interferometer eine spektral breitbandige oder Wellenlängendurchstimmbare Quelle elektromagnetischer Strahlung vorgeordnet ist, im Weiteren

als Lichtquelle (1) bezeichnet, welche ein Eingangsstrahlenbündel für das Mirau-Interferometer erzeugt,

also das Mirau-Interferometer mit einem Abbildungsstrahlengang für das zu prüfende Objekt (8) und mindestens einem einzigen Punktempfänger, einem gerastertem Zeilenempfänger oder einem gerastertem Flächenempfänger (11, 111) für elektromagnetische Strahlung ausgebildet ist,

gekennzeichnet dadurch,

daß im Hinlauf des Lichtes auf dem Weg zum Objekt (8) nach der letzten Linse des Mirau-Interferometers nach dem Strahlteiler des Mirau-Interferometers aus dem diesen Strahlteiler passierenden Teilstrahlenbündel mittels Diffraktion in Transmission an einer statischen oder dynamischen, diffraktiv-optischen Zonenlinse (5a) ein gebeugtes Strahlenbündel O_N (N=1, 2, 3... oder -1, -2, -3...) in der ersten oder einer höheren Beugungsordnung N (N=1, 2, 3) oder einer niedrigeren Beugungsordnung N als der nullten (N=-1, -2,-3) N entsteht, welches nach Fokussierung auf das Objekt (8) im Objektraum chromatisch längs aufgespalten ist, so daß in Abhängigkeit von der Wellenlänge des Lichts eine Reihe oder eine Vielzahl von Reihen von Fokuspunkten in der Tiefe ausgebildet wird, und nach Reflexion des Teilstrählenbündels O_N (N=1, 2, 3... oder -1,-2,-3...) am Objekt (8) ein Objekt-Teilstrahlenbündel vom rückreflektierten Strahlenbündel durch Beugung an der diffraktiv-optischen Zonenlinse (5a) in derselben Ordnung wie im Hinlauf, nämlich O_N_N (N=1, 2, 3... oder -1, -2, -3...) gebildet wird, so daß das Objektteilstrahlenbündel O_N_N (N=1, 2, 3... oder -1, -2, -3...) nach dem Passieren der diffraktiv-optischen Zonenlinse (5a) wieder im Strahlengang zurückläuft,

und aus dem Eingangsstrahlenbündel im Hinlauf auf die Fläche des Strahlteilers, dessen Strahlteilerfläche (7) parallel zu der diffraktiv-optischen Zonenlinse (5a) liegt, auch noch ein Teilstrahlenbündel,

welches im Weiteren als Referenzstrahlenbündel R dient,

gebildet wird und im Rücklauf von der Referenzspiegelfläche (15), auf welche das Referenzstrahlenbündel R fokussiert wird,

und daß das Referenzteilstrahlenbündel R nach dem Passieren des Strahlteilers in Reflexion wieder im Strahlengang zurückläuft,

und zwischen dem Objekt-Teilstrahlenbündel O_N_N (N=1, 2, 3... oder -1, -2, -3...) und dem Referenzstrahlenbündel R der optische Gangunterschied ungleich null gemacht ist, wenn auf einen Objektpunkt fokussiert ist,

und daß das Objekt-Teilstrahlenbündel O_N_N (N=1, 2, 3... oder -1, -2, -3...) und das Referenzstrahlenbündel R nach der diffraktiv-optischen Zonenlinse (5a) parallel verlaufen und zur Interferenz kommen und anschließend mittels Teilung der Amplitude der Wellenfronten die beiden miteinander interferierenden Teilstrahlenbündel in den Detektionsstrahlengang eingekoppelt werden

und eine konfokale Diskriminierung des Lichts der interferierenden Teilstrahlenbündel durchgeführt wird

und die interferierenden Bündel Objekt-Teilstrahlenbündel O_N_N (N=1, 2, 3... oder -1, -2, -3...) und Referenzstrahlenbündel R zur spektroskopischen Analyse eine Lateralaufspaltung mittels Dispersion oder Diffraktion erfahren und eine FFT des Intensitätsverlaufs durchgeführt wird, um aus dem sich mittels FFT ergebenden Wertes nach Kalibrierung die Tiefenposition eines Objektpunktes zu bestimmen,

oder daß die interferierenden Bündel Objekt-Teilstrahlenbündel O_N_N (N=1, 2, 3... oder -1, -2, -3...) und R eine weitere Interferenzbildung mittels scannendem Zweistrahl-Interferometer, also mit zeitlichem Variieren des optischen Gangunterschieds zur optischen Analyse, erfahren und der im scannenden Zweistrahl-Interferometer beim Scan durchfahrene optische Gangunterschied mindestens einmal genau auch dem im interferometer zwischen Objekt-Teilstrahlenbündel O_N_N (N=1, 2, 3... oder -1, -2, -3...) und Referenzstrahlenbündel R bestehenden optischen Gangunterschied entspricht, so daß beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird

oder daß die interferierenden Bündel O_N_N (N=1, 2, 3... oder -1, -2, -3...) und R eine weitere Interferenzbildung mittels statischem Zweistrahl-Interferometer zur optischen Analyse, erfahren

also einem Zweistrahl-Interferometer mit lateraler Variation des optischen Gangunterschieds, wobei die Orte unterschiedlichen Gangunterschieds Pixeln einer Zeilen- oder Flächenkamera zugeordnet sind

und daß der im Zweistrahl-Interferometer auftretende optische Gangunterschied dem im Mirau-Interferometer zwischen Objekt-Teilstrahlenbündel O_N_N (N=1, 2, 3... oder -1, -2, -3...) und Referenzstrahlenbündel R bestehenden optischen Gangunterschied in mindestens einem Element der Zeilen- oder Flächenkamera zumindest näherungsweise entspricht

oder daß im Fall der Anwendung einer Wellenlängen-durchstimmbaren Quelle elektromagnetischer Strahlung beim Scan der Wellenlänge ein moduliertes periodisches optisches Signal erzeugt wird und mittels Punktempfänger über der Zeit detektiert wird und dieses über der Wellenlänge ausgewertet wird.

Eine bevorzugte Ausführungsform der vorliegenden Erfindung betrifft ein multispektrales interferometrisches Verfahren zur hochdynamischen Objekttiefenabtastung oder - Profilerfassung mit einem fasergekoppelten Michelson-Interferometer mit einem Strahlteiler, der als x-Koppler (33) ausgebildet ist, und mit einem Referenzspiegel (15a) oder mit einer Referenzspiegelfläche und mit einem zu prüfenden Objekt (8),

wobei dem fasergekoppelten Michelson-Interferometer eine spektral breitbandige oder Wellenlängen-durchstimmbare Quelle elektromagnetischer Strahlung vorgeordnet ist, im Weiteren als Lichtquelle (1) bezeichnet, welche ein Eingangsstrahlenbündel für das Michelson-Interferometer erzeugt,

also das fasergekoppelte Michelson-Interferometer im Objektstrahlengang mit einem Abbildungsstrahlengang für das zu prüfende Objekt (8) und mindestens einem einzigen Punktempfänger, einem gerastertem Zeilenempfänger oder einem gerastertem Flächenempfänger (11, 111) für elektromagnetische Strahlung ausgebildet ist,

gekennzeichnet dadurch,

daß im Hinlauf des Lichtes auf dem Weg zum Objekt nach dem x-Koppler (33) des Michelson-Interferometers mittels Diffraktion in Transmission an einer statischen oder einer dynamischen, diffraktiv-optischen Zonenlinse (DOZL) (5) ein gebeugtes Strahlenbündel O_N (N=1, 2, 3... oder -1, -2, -3...) in der ersten oder einer höheren Beugungsordnung N (N=1, 2, 3) oder niedrigeren Beugungsordnung N als der nullten (N=-1, -2, -3) entsteht und dieses gebeugte Strahlenbündel O_N (N=1, 2, 3... oder -1, -2, -3...) nach Fokussierung auf das Objekt (8) im Objektraum chromatisch längs aufgespalten ist, und nach Reflexion des Teilstrahlenbündels O_N (N=1, 2, 3... oder -1, -2, -3...) am Objekt (8) ein Objekt-Teilstrahlenbündel vom rückreflektierten Strahlenbündel durch Beugung an der diffraktiv-optischen Zonenlinse in derselben Ordnung wie im Hinlauf, nämlich O_N_N (N=1, 2, 3... oder -1, -2, -3...) gebildet wird, so daß das Objektteilstrahlenbündel O_N_N (N=1, 2, 3... oder -1, -2, -3...) nach dem Passieren der diffraktiv-optischen Zonenlinse (5) wieder im Strahlengang zurückläuft, konfokal mittels Faserende diskriminiert wird, anschließend den x-Koppler (33) passiert und in eine Auskoppelfaser (9) gelangt

und daß das Referenzbündel R im Hinlauf durch Teilung am x-Koppler (33) entsteht und am Ende der Faser direkt an einer Referenzspiegelfläche (7b) oder am Ende einer der Faser nachgeordneten Fokussieroptik an einer Referenzspiegelfläche reflektiert wird

und daß das in der Faser zurücklaufende Licht den x-Koppler (33) passiert und ebenfalls in die Auskoppelfaser (9) gelangt, wo das Licht aus dem Objektstrahlengang mit dem aus dem Referenzstrahlengang sich überlagert und zur Interferenz kommt

und daß die interferierenden Bündel O_N_N (N=1, 2, 3... oder -1, -2, -3...) und R dabei zur spektroskopischen Analyse eine Lateralaufspaltung mittels Dispersion oder Diffraktion erfahren

und daß eine FFT des Intensitätsverlaufs erfolgen kann, um aus dem sich mittels FFT ergebenden Wertes nach Kalibrierung die Tiefenposition eines Objektpunktes P zu bestimmen

oder daß die interferierenden Bündel O_N_N (N=1, 2, 3... oder -1, -2, -3...) und R eine weitere Interferenzbildung mittels scannendem Zweistrahl-Interferometer, also mit zeitlichem Variieren des optischen Gangunterschieds zur optischen Analyse, erfahren, wobei der im scannenden Zweistrahl-Interferometer beim Scan durchfahrene optische Gangunterschied mindestens einmal genau auch dem im Michelson-Interferometer zwischen Objekt-Teilstrahlenbündel O_N_N (N=1, 2, 3... oder -1, -2, -3...) und Referenzstrahlenbündel R bestehenden optischen Gangunterschied entspricht, so daß beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird

oder daß die interferierenden Bündel Objekt-Teilstrahlenbündel O_N_N (N=1, 2, 3... oder -1, -2, -3...) und Referenzstrahlenbündel R eine weitere Interferenzbildung mittels statischem Zweistrahl-Interferometer zur Objekt-Teilstrahlenbündel optischen Analyse erfahren, also einem Zweistrahl-Interferometer mit lateraler Variation des optischen Gangunterschieds

oder daß im Fall der Anwendung einer Wellenlängen-durchstimmbaren Quelle elektromagnetischer Strahlung beim Scan der Wellenlänge ein moduliertes periodisches optisches Signal erzeugt wird und mittels Punktempfänger über der Zeit detektiert wird und dieses über der Wellenlänge ausgewertet wird.

Eine bevorzugte Ausführungsform der vorliegenden Erfindung betrifft eine multispektrale interferometrische Anordnung zur hochdynamischen Objekttiefenabtastung oder - Profilerfassung mit einem objektabbildenden Zweistrahl-Interferometer mit einer Referenzspiegelfläche (7) und mit einem zu prüfenden Objekt (8),

wobei dem Zweistrahl-Interferometer eine spektral breitbandige oder Wellenlängen-durchstimmbare Quelle elektromagnetischer Strahlung vorgeordnet ist, im weiteren als Lichtquelle (1) bezeichnet, welche ein Eingangsstrahlenbündel für das Zweistrahl-Interferometer erzeugt,

also das Zweistrahl-Interferometer mit einem Abbildungsstrahlengang für das zu prüfende Objekt (8) und einem Punktempfänger, einem gerasterten Zeilenempfänger oder einem gerasterten Flächenempfänger für elektromagnetische Strahlung (11, 111) ausgebildet ist,

gekennzeichnet dadurch,

daß das Zweistrahl-Interferometer mit einer diffraktiv-optischen Zonenlinse (5, 105) und mittels in Richtung des Verlaufs, des Eingangsstrahlenbündels nachgeordneter abbildender Linse (12) oder Objektiv (12a) mit Referenzspiegelfläche (7) mit oder ohne Strahlteilerschicht (7a, 107a) des Zweistrahl-Interferometers ausgebildet ist.

Gemäß einer bevorzugten Ausführungsform der multispektralen interferometrischen Anordnung zur hochdynamischen Objekttiefenabtastung oder -Profilerfassung ist vorzugsweise dem Zweistrahl-Interferometer ein Spektrometer (10) nachgeordnet.

Gemäß einer bevorzugten Ausführungsform der multispektralen interferometrischen Anordnung zur hochdynamischen Objekttiefenabtastung oder -Profilerfassung, ist vorzugsweise dem objektabbildenden Zweistrahl-Interferometer ein Zweistrahl-Interferometer nachgeordnet ist, welches eine optische Analyse des Interferenzlichts am Ausgang des objektabbildenden Zweistrahl-Interferometers ermöglicht.

Multispektrale interferometrische Anordnung zur hochdynamischen Objekttiefenabtastung gemäß einem Beispiel, wobei vorzugsweise das objektabbildende Zweistrahl-Interferometer als Fizeau-Interferometer mit mikroskopischer Abbildung des Objekts ausgebildet ist, wobei eine diffraktiv-optische Phasengitter-Zonen-Linse (5, 105) im Fizeau-Interferometer angeordnet ist, die bevorzugt sowohl Licht in der nullten und in der ersten Beugungsordnung erzeugt.

Multispektrale interferometrische Anordnung zur hochdynamischen Objekttiefenabtastung oder -Profilerfassung gemäß einem Beispiel, wobei vorzugsweise die abbildende Linse als Grinlinse (6, 106) mit Frontfläche als Referenzspiegelfläche (7) mit Strahlteilerschicht (7a, 107a) oder ohne Strahlteilerschicht des Zweistrahl-Interferometers ausgebildet ist.

Multispektrale interferometrische Anordnung zur hochdynamischen Objekttiefenabtastung oder -Profilerfassung gemäß einem Beispiel, wobei vorzugsweise die diffraktiv-optische Phasengitter-Zonen-Linse (5, 105) in der Brennebene der abbildenden Linse (12) oder dem Objektiv (12a) mit Referenzspiegelfläche (7) oder der Grinlinse (6, 106) mit Frontfläche als Referenzspiegelfläche (7) angeordnet ist, die dem Objekt abgewandt ist.

Multispektrale interferometrische Anordnung zur hochdynamischen Objekttiefenabtastung oder -Profilerfassung gemäß einem Beispiel, wobei vorzugsweise das objektabbildende Zweistrahl-Interferometer als Mirau-Interferometer ausgebildet ist, wobei zwischen der Strahlteilerplatte (13) mit Strahlteilerschicht (7a) des Mirau-Interferometers und dem Objekt (8) das diffraktiv-optische Zonenelement (5a) angeordnet ist.

Multispektrale interferometrische Anordnung zur hochdynamischen Objekttiefenabtastung oder -Profilerfassung gemäß einem Beispiel, wobei vorzugsweise das diffraktiv-optische Zonenelement als transmissive, diffraktive Zonenlinse (5, 5a, 105) ausgebildet ist.

Multispektrale interferometrische Anordnung zur hochdynamischen Objekttiefenabtastung oder -Profilerfassung gemäß einem Beispiel, wobei vorzugsweise die diffraktive Zonenlinse (5, 5a, 105) so ausgebildet ist, daß diese ihre optische Wirkung vorwiegend in der ersten Beugungsordnung entfaltet.

Multispektrale interferometrische Anordnung zur hochdynamischen Objekttiefenabtastung oder -Profilerfassung gemäß einem Beispiel, wobei vorzugsweise die diffraktive Zonenlinse als lichtzerstreuende Zonenlinse (5, 5a, 105) ausgebildet ist.

Multispektrale interferometrische Anordnung zur hochdynamischen Objekttiefenabtastung oder -Profilerfassung gemäß einem Beispiel, wobei vorzugsweise die Strahlteilerplatte (13) mit Strahlteilerschicht (7a) und die diffraktive Zonenlinse (5a) zu einer Kittgruppe (13a) vereinigt sind, wobei die Strahlteilerschicht (7a) innerhalb der Kittgruppe (13a) angeordnet ist und die Substratdicken und die Substratwerkstoffe der Strahlteilerplatte (13) und der diffraktiven Zonenlinse (5a) gleich gemacht sind.

Ein Beispiel betrifft eine multispektrale interferometrische Anordnung zur hochdynamischen Objekttiefenabtastung oder - Profilerfassung mit einem fasergekoppelten Michelson-Interferometer mit einem Strahlteiler und mit einem Referenzspiegel (15a) und mit einem zu prüfenden Objekt (8),

wobei dem fasergekoppelten Michelson-Interferometer eine spektral breitbandige oder Wellenlängen-durchstimmbare Quelle elektromagnetischer Strahlung vorgeordnet ist, im weiteren als Lichtquelle (1) bezeichnet, welche ein Eingangsstrahlenbündel für das Michelson-Interferometer erzeugt,

also das fasergekoppelte Michelson-Interferometer mit einem Abbildungsstrahlengang für das zu prüfende Objekt (8) und einem Punktempfänger, einem gerasterten Zeilenempfänger oder einem gerasterten Flächenempfänger für elektromagnetische Strahlung (11, 111) ausgebildet ist,

gekennzeichnet dadurch,

daß dem Strahlteiler des fasergekoppelten Michelson-Interferometers eine statische oder dynamische, diffraktiv-optische Zonenlinse (5) in Lichtrichtung des Eingangsstrahlenbündels im Abbildungsstrahlengang für das zu prüfende Objekt (8) nachgeordnet ist, wobei die diffraktiv-optische Zonenlinse (5) für eine einzige Beugungsordnung ausgebildet ist und daß der Strahlteiler als x-Koppler (33) ausgebildet ist.

Multispektrale interferometrische Anordnung zur hochdynamischen Objekttiefenabtastung oder -Profilerfassung gemäß eimem Beispiel, wobei vorzugsweise dem fasergekoppelten Michelson-Interferometer ein Spektrometer (10) nachgeordnet ist.

Multispektrale interferometrische Anordnung zur hochdynamischen Objekttiefenabtastung oder -Profilerfassung, wobei vorzugsweise dem fasergekoppelten Michelson-Interferometer ein Zweistrahl-Interferometer nachgeordnet ist, welches eine optische Analyse des Interferenzlichts am Ausgang des Michelson-Interferometers ermöglicht.

Ein Beispiel betrifft ein Verfahren zur spektralen Zweistrahl-Interferometrie mit chromatischer Tiefenaufspaltung im Objektstrahlengang durch eine chromatische Brechkraft zur Prüfung von einem Objekt (312b, 312c) oder zur Auslesung eines optischen Datenträgers, der dann das Objekt (312a) darstellt, mit einem Zweistrahl-Interferometer mit einem Referenzstrahlengang und einem Referenzreflektor sowie mit einem Objektstrahlengang, der von mindestens einem Objektteilstrahlenbündel durchlaufen wird, mit zumindest teilweiser Abbildung des Objekts (312a, 312b, 312c) durch ein Abbildungssystem (306a, 306b) mit einer vergleichsweise großen Brechkraft, welches die Hauptbrechkraft in der Anordnung darstellt, wobei auch Referenzstrahlung das Abbildungssystem durchsetzt, und mit Anwendung einer Lichtquelle (1 a, 1 b, 1 c) und eines gerasterten Detektors (315, 315a) für elektromagnetische Strahlung sowie eines Spektrometers (314, 314a) oder einer wellenlängendurchstimmbaren Quelle elektromagnetischer Strahlung (1 c), gekennzeichnet dadurch,

a) daß entweder eine zumindest näherungsweise achromatische Nebenbrechkraft in den Referenzstrahlengang durch ein strahlenfokussierendes oder strahlendivergierendes Referenzelement oder Referenzsystem eingeführt wird, wobei die Nebenbrechkraft hier als Referenz-Nebenbrechkraft bezeichnet wird und wobei die achromatische Referenz-Nebenbrechkraft das gleiche Vorzeichen wie die chromatische Brechkraft aufweist.

b) oder daß eine zumindest näherungsweise achromatische Nebenbrechkraft in den Objektstrahlengang durch ein strahlenfokussierendes oder strahlendivergierendes Element oder System eingeführt wird, wobei die Nebenbrechkraft hier als Objekt-Nebenbrechkraft bezeichnet wird und wobei die achromatische Objekt-Nebenbrechkraft das umgekehrte Vorzeichen wie die chromatische Brechkraft aufweist.

c) oder daß sowohl eine zumindest näherungsweise achromatischen Nebenbrechkraft in den Referenzstrahlengang durch ein strahlenfokussierendes oder strahlendivergierendes Referenzelement oder Referenzsystem als auch eine zumindest näherungsweise achromatische Nebenbrechkraft im Objektstrahlengang durch ein strahlenfokussierendes oder strahlendivergierendes Element oder System eingeführt wird.

Verfahren zur spektralen Zweistrahl-Interferometrie mit chromatischer Tiefenaufspaltung im Objektstrahlengang durch eine chromatische Brechkraft gemäß einem Beispiel, wobei vorzugsweise die chromatische Brechkraft im Objektstrahlengang durch die Referenz-Nebenbrechkraft zumindest teilkompensiert oder aber überkompensiert wird.

Verfahren zur spektralen Zweistrahl-Interferometrie mit chromatischer Tiefenaufspaltung im Objektstrahlengang durch eine chromatische Brechkraft gemäß einem Beispiel, wobei vorzugsweise durch die Objekt-Nebenbrechkraft die chromatische Längsaberration im Objektstrahlengang durch mindestens ein strahlenfokussierendes oder strahlendivergierendes und zumindest näherungsweise achromatisches Element oder System zumindest teilkompensiert oder aber überkompensiert wird.

Verfahren zur spektralen Zweistrahl-Interferometrie mit chromatischer Tiefenaufspaltung im Objektstrahlengang durch eine chromatische Brechkraft gemäß einem Beispiel, wobei vorzugsweise durch eine chromatische Ausbildung des gemeinsamen Abbildungssystems mit angepaßter chromatischer Queraberration die chromatische Queraberration für das Objektteilstrahlenbündel, die im Objektstrahlengang unerwünschterweise vorhanden ist, zumindest näherungsweise zu null gemacht wird.

Ein Beispiel betrifft eine spektrale Zweistrahl-Interferometer-Anordnung mit chromatischer Tiefenaufspaltung im Objektstrahlengang durch eine chromatische Brechkraft mittels mindestens eines strahlenfokussierenden oder strahlendivergierenden chromatischen Elements oder Systems mit chromatischem Element zur Prüfung von einem Objekt oder zur Auslesung eines optischen Datenträgers, der dann das Objekt darstellt, mit einer Lichtquelle (301, 301 a) und mit einem gerastertem Detektor (315, 315a) elektromagnetischer Strahlung, einer Strahlteilerfläche (309) und einem Spektrometer (314,314a) und mit Objekt- und Referenzstrahlengang und einem Referenzreflektor (310) in demselben sowie einem Abbildungssystem für das Objekt, welches die Hauptbrechkraft des optischen Systems darstellt, gekennzeichnet dadurch,

a) daß entweder im Referenzstrahlengang eine zumindest näherungsweise achromatische, strahlenfokussierende oder strahlendivergierende Linse, oder Linsengruppe oder Spiegel, oder Spiegellinse, oder Komponente also jeweils mit positiver oder negativer Brechkraft, der Referenz-Nebenbrechkraft, angeordnet ist, angeordnet, wobei die achromatische Referenz-Nebenbrechkraft das gleiche Vorzeichen wie die chromatische Brechkraft des strahlenfokussierenden oder strahlendivergierenden chromatischen Elements oder Systems aufweist.

b) oder daß im Objektstrahlengang eine zumindest näherungsweise achromatische, strahlenfokussierende oder strahlendivergierende Linse, oder Linsengruppe, oder Spiegel, oder Spiegellinse, oder Komponente also jeweils mit positiver oder negativer Brechkraft, der Objekt-Nebenbrechkraft, angeordnet ist, angeordnet, wobei die achromatische Referenz-Nebenbrechkraft das umgekehrte Vorzeichen wie die chromatische Brechkraft des strahlenfokussierenden oder strahlendivergierenden chromatischen Elements oder Systems aufweist.

c) oder daß sowohl im Referenzstrahlengang als auch im Objektstrahlengang eine zumindest näherungsweise achromatische, strahlenfokussierende oder strahlendivergierende Linsenfläche, oder Linse, oder Linsengruppe, oder Spiegel, oder Spiegellinse, oder Komponente also jeweils mit positiver und/oder negativer Brechkraft angeordnet ist, wobei die Wirkung der resultierenden achromatischen Nebenbrechkraft den Abstand $z_i$ von einem scharf abgebildeten Objektpunkt $P_i$ im Tiefenmeßbereich zur Fläche G gleichen optischen Gangunterschiedes verkleinert.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise die Referenz-Nebenbrechkraft mindestens 30% der chromatischen Brechkraft im Objektstrahlengang für die mittlere Wellenlänge des zur zumindest teilweisen Abbildung des Objekts genutzten Spektralbereiches beträgt.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise im Objektstrahlengang das chromatische Element als mindestens ein diffraktiv-optisches, strahlenfokussierendes oder strahlendivergierendes Zonenelement (311a, 311b, 311c, 311d) ausgebildet ist und im Referenzstrahlengang mindestens eine refraktive, strahlenfokussierende oder strahlendivergierende Linsenfläche (308a, 308b) oder Linse oder auch eine Spiegelfläche angeordnet sind.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise im Objektstrahlengang das chromatische Element mindestens ein diffraktiv-optisches, strahlenfokussierendes oder strahlendivergierendes Zonenelement (311a, 311b, 311c, 311d) und im Referenzstrahlengang mindestens ein strahlenfokussierender oder strahlendivergierender Achromat angeordnet sind.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise vom diffraktiv-optischen, strahlenfokussierenden oder strahlendivergierenden Zonenelement (311a, 311b, 311c, 311d) im Objektstrahlengang die Strahlformungseigenschaften in der positiven oder der negativen ersten Beugungsordnung oder in einer höheren positiven oder negativen Beugungsordnung jeweils zur Abbildung des Objekts mitgenutzt werden. So kann auch durch die chromatische Brechkraft die numerische Apertur der Anordnung noch etwas erhöht werden.

Spektrale Zweistrahl-Interterometer-Anordnung mit chromatischer Tiefenaufspaltung im Objektstrahlengang durch eine chromatische Brechkraft gemäß einem Beispiel, wobei vorzugsweise die Zweistrahlinterferometer-Anordnung als Mirau-Interferometer mit mindestens einem diffraktiv-optischen, strahlenfokussierenden oder strahlendivergierenden Zonenelement (311a, 311b, 311c, 311d) im Objektstrahlengang ausgebildet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise das Zonenelement als Phasen-Zonen-Linse (311a, 311b, 311c, 311d) für die positive und/oder die negative erste Beugungsordnung ausgebildet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise die Phasen-Zonen-Linse (311a) strahlenfokussierend ausgebildet ist und dieser mindestens eine refraktive Linsenfläche (308b) oder Linse mit strahlendivergierender Wirkung, also eine Zerstreuungskomponente, zugeordnet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise der Betrag der Brechkraft der refraktiven Linsenfläche (308b) oder der Linse dem Betrag der mittleren Brechkraft der Phasen-Zonen-Linse (311 b) zumindest näherungsweise entspricht. Spektrale Zweistrahl-Interferometer-Anordnung mit chromatischer Tiefenaufspaltung im Objektstrahlengang durch eine chromatische Brechkraft gemäß einem Beispiel, wobei vorzugsweise die Strahlteilerfläche (309) mit einer Krümmung ausgebildet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise mindestens eine refraktive Planparallelplatte im Referenz- und/oder im Objektstrahlengang zur Anpassung der Dispersion in der optischen Anordnung angeordnet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise mindestens eine Phasen-Zonenplatte im Referenz- und/oder Objektstrahlengang zur Anpassung der Dispersion in der optischen Anordnung angeordnet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise das Abbildungssystem für das Objekt mit mindestens einer Grinlinse (306b) ausgebildet ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise im Abbildungssystem mindestens ein refraktives Element mit chromatischer Queraberration angeordnet ist, durch das die chromatische Queraberration für das Objektteilstrahlenbündel, die im Objektstrahlengang unerwünschterweise vorhanden ist, zumindest näherungsweise zu null gemacht ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise im Abbildungssystem mindestens ein diffraktives Zonenelement mit chromatischer Queraberration angeordnet ist, durch das die chromatische Queraberration für das Objektteilstrahlenbündel, die im Objektstrahlengang unerwünschterweise vorhanden ist, zumindest näherungsweise zu null gemacht ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise in einem Mirau-Interferometer ein Hybridelement mittels zweiseitigem Substrat angeordnet ist, bei dem auf der dem Abbildungssystem zugeordneten Substratseite eine teildurchlässige Spiegelschicht angeordnet ist, die als Strahlteilerfläche (309) ausgebildet ist, wobei diese Substratseite konkav (308b) ausgebildet ist, und auf der anderen Substratseite eine Phasen-Zonen-Struktur (311a) mit strahlfokussierender Wirkung in der ersten Beugungsordnung aufgebracht ist oder wobei die dem Abbildungssystem zugeordneten Substratseite konvex (8a) ausgebildet ist, und auf der anderen Substratseite eine Phasen- Zonen-Struktur mit strahldivergierender Wirkung in der ersten Beugungsordnung aufgebracht ist.

Spektrale Zweistrahl-Interferometer-Anordnung mit chromatischer Tiefenaufspaltung im Objektstrahlengang durch eine chromatische Brechkraft gemäß einem Beispiel, wobei vorzugsweise die Zweistrahl-Interferometer-Anordnung als Twyman-Green-, Michelson- oder Mach-Zehnder-Anordnung ausgebildet ist.

Bevorzugte Ausführungsformen der vorliegenden Erfindung werden anhand nachfolgender Zeichnungen beispielhaft beschrieben, wobei sowohl die Ausführungsformen beliebig untereinander kombiniert werden können als auch einzelne Merkmale der Ausführungsformen beliebig mit weiteren Ausführungsformen kombiniert werden können.

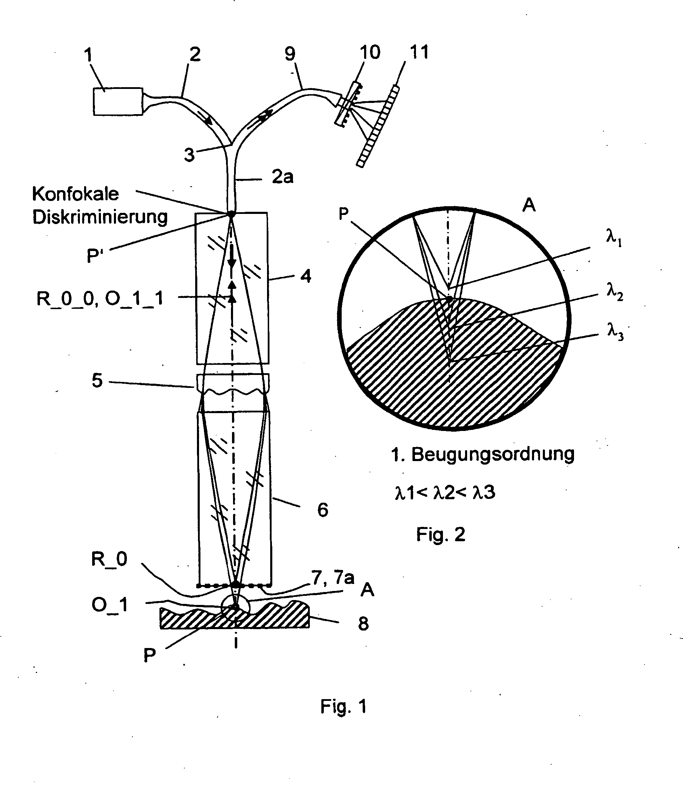

In **Figur 1** wird die Wirkung einer diffraktiv-optischen Zonenlinse 5 (DOZL) als brechkraftvariable Komponente in einer Common-path-Interferometeranordnung zur Tiefenmessung eines Objekts 8 dargestellt. Die Common-path-Interferometeranordnung kann dabei auch mit einer Präzisionsführung gekoppelt sein, um das Profil eines Objektes 8 messen zu können.

Das von einer Weißlichtquelle 1 ausgehende Licht gelangt über eine Lichtleitfaser 2 und eine y-Weiche 3 über eine Grinlinse 4 zur Kollimierung des Lichtbündels auf eine diffraktiv-optische Zonenlinse 5 mit in Abhängigkeit von der Lichtwellenlänge variabler negativer Brechkraft, so dass diese als Zerstreuungslinse wirkt.

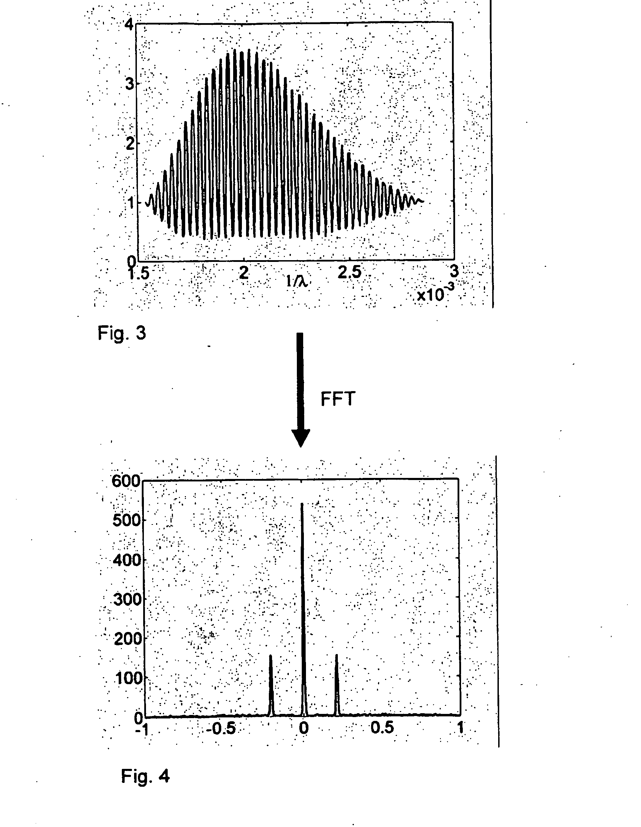

Das Lichtbündel wird an der diffraktiv-optischen Zonenlinse 5 in der nullten Beugungsordnung in einen Referenz-R und in der ersten Beugungsordnung in einen eher schwach divergierenden Objektstrahl O_1 aufgespalten. Dabei ist die nachfolgende Grinlinse 6 in ihrem geometrisch-optischen Aufbau so abgestimmt, dass die Frontfläche 7 der Grinlinse 6, die als Referenzteilspiegel mit Strahlteilerschicht 7a wirkt, im Fokus des Bündels der nullten Beugungsordnung der diffraktiv-optischen Zonenlinse 5 liegt. Das an der Frontfläche 7 mit Strahlteilerschicht 7a reflektierte Teillichtbündel dient als Referenzbündel R. Das in der 1. Beugungsordnung die diffraktiv-optische Zonenlinse 5 passierende Teillichtbündel O_1 durchsetzt die Frontfläche 7 der Grinlinse 6 und die Strahlteilerschicht 7a, welche die Referenzspiegelfläche darstellt, und gelangt auf das Objekt 8. Durch die brechkraftvariable Wirkung der diffraktiv-optischen Zonenlinse 5 erfolgt in Abhängigkeit von der Lichtwellenlänge die Fokussierung des Objektstrahlenbündels O_1 in unterschiedlichen Tiefen des Objektraumes. Dies ist in der Figur 2 dargestellt. Dabei stellt das Fokusbild die Abbildung des Endes der Monomodefaser 1 dar. Das aus dem Objektraum vom Fokusbild rückreflektierte und rückgestreute Licht passiert gemeinsam mit dem Referenzbündel wieder die Grinlinse 6. Das aus dem Objektraum vom Fokusbild rückreflektierte und rückgestreute Licht passiert die diffraktiv-optische Zonenlinse 5 wieder in der ersten Beugungsordnung und wird zum Objektstrahlenbündel O_1_1. Das an der Frontfläche 7, die als Referenzspiegelfläche mit Strahlteilerschicht 7a wirkt, fokussierte Licht wird zum Teil reflektiert und passiert die diffraktiv-optische Zonenlinse 5 wieder in der nullten Beugungsordnung und wird zum Referenzstrahlenbündel R_0_0. So bestehen zwei interferierende Bündel O_1_1 und R_0_0, die auf das Ende der Monomodefaser 2 mittels Grinlinse 4 wieder fokussiert werden und in diese Monomodefaser 2 eintreten, wobei hier insbesondere für das Objektstrahlenbündel O_1_1 eine konfokale Diskriminierung erfolgt. Nach dem Passieren der y-Weiche 3 gelangt das interferierende Licht über die Auskoppelfaser 9 auf ein Beugungsgitter 10 auf eine hochempfindliche Zeilenkamera 11, die ein Spektrum registriert. Dabei ist das Spektrum über der Wellenzahl moduliert - je nach den Bedingungen für konstruktive und destruktive Interferenz in Abhängigkeit von der Wellenlänge und dem optischen Gangunterschied am Objektpunkt - und bildet über der Wellenzahl ein Wavelet, s. a. **Figur 3.**

Das entstehende Signal / wird dabei mit folgender Formel mit der Modulation m berechnet:

$$I(\lambda_i) = I_{Ref} + I_{Obj}(\lambda_i) + 2\sqrt{I_{Ref} \cdot I_{Obj}(\lambda_i)} \cdot m \cdot \cos\left(2\pi \cdot \frac{2\Delta z}{\lambda_i} + \varphi_0\right). \qquad (1)$$

Der Abstand 2Δz kennzeichnet den Wegunterschied der interferierenden Wellenpakete (0. Ordnung und 1. Ordnung). Figur 3 zeigt das Signal über der Wellenzahl, dem reziproken Wert der Wellenlänge, bei einem Abstand 2Δz von 30 μm.

Der Einfluss der konfokalen Blende auf das Signal wird über folgende Formel bestimmt:

$$I = \frac{\sin\left(\frac{u}{2}\right)}{\frac{u}{2}} \quad \text{mit} \quad u = \frac{2\pi}{\lambda}NA^2 v \qquad (2)$$

Mit NA wird die numerische Apertur des Lichtkegels an der Blende bezeichnet. Das Argument *v* entspricht hier der Wellenlängenachse. Zur Berechnung des konfokalen Signals wurde eine mittlere Wellenlänge $\overline{\lambda}$ von 500 nm angenommen. Dabei ist für das entstehende Interferenzsignal zu beachten, dass nur das Objektstrahlenbündel eine konfokale Diskriminierung über der Wellenlänge am Ende der Lichtleitfaser 2a erfährt, da Lichtanteile, die im Objektraum nicht gut fokussiert sind, nicht oder nur stark abgeschwächt in die Lichtleitfaser 2a eintreten können. Das Referenzstrahlenbündel tritt zumindest näherungsweise für alle Lichtwellenlängen gleich gut fokussiert in die Lichtleitfaser 2a ein. Damit ergibt sich auch für das Interferenzsignal eine Einhüllende über der Wellenlänge bzw. der Wellenzahl.

Mit dieser Anordnung kann der Abstand eines Objektpunktes mit hoher Geschwindigkeit und Genauigkeit durch Auswertung des Spektrums ermittelt werden. Dabei ist die spektrale Auflösung so hoch zu wählen, dass innerhalb eines Spektralelements - durch das nicht mehr auf einem Photoelement oder Pixel aufgelöste Spektralintervall - die Kohärenzlänge größer als der optische Gangunterschied im Interferometer ist. Dieser optische Gangunterschied beträgt einige 10 μm bis zu einigen 100 μm und kann je nach der spektralen Auflösung und photometrischer Empfindlichkeit des verwendeten Spektrometers größer oder kleiner gewählt werden.

Diese Sensoranordnung ermöglicht die Applikation in Nano-Messmaschinen zur Abstandskontrolle und bei lateraler Bewegung des Sensors zum Objekt auch zur Profilmessung desselben.

Andererseits kann mit dieser Anordnung auch Optische Kohärenz-Tomografie (optical coherence tomography, OCT) an biologischen Proben betrieben werden.

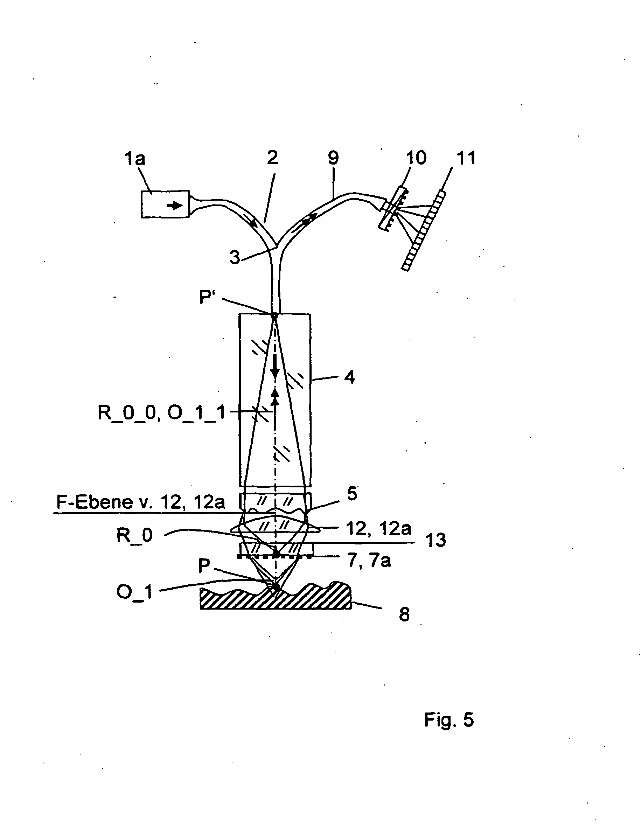

In **Figur 5** wird eine Anordnung entsprechend Figur 1 beschrieben. Dabei ist hier der Einsatz eines Kontinuums-Weißlichtlasers als Lichtquelle 1a sehr von Vorteil. Hier kommt jedoch anstelle der Grinlinse 6 von Figur 1 eine Mikrolinse 12 oder auch ein gut chromatisch korrigiertes Mikroobjektiv 12a zum Einsatz. Der Mikrolinse 12 oder dem gut korrigierten Mikroobjektiv 12a ist eine Planparallelplatte 13 mit der Referenzspiegelfläche 7 mit der Strahlteilerschicht 7a nachgeordnet. Die Applikation der Anordnung ist hierbei die Abstandsmessung in einem Punkt eines Objekts 8. Beim Einsatz einer Reihe von Fasern 2, mehreren y-Weichen 3 und mehreren Auskoppelfasern 9 und eines Spektrometers 10 mit einem Spalt und mit einer Flächenkamera 11 kann auch eine linienhafte Profilmessung durchgeführt werden. Das Spektrometer 10 weist dabei ein hohes spektrales Auflösungsvermögen auf. Diese Sensoranordnung kann ebenfalls als universeller Punktsensor für eine Auflösung im Nanometerbereich beispielsweise in einer Nano-Messmaschine eingesetzt werden.

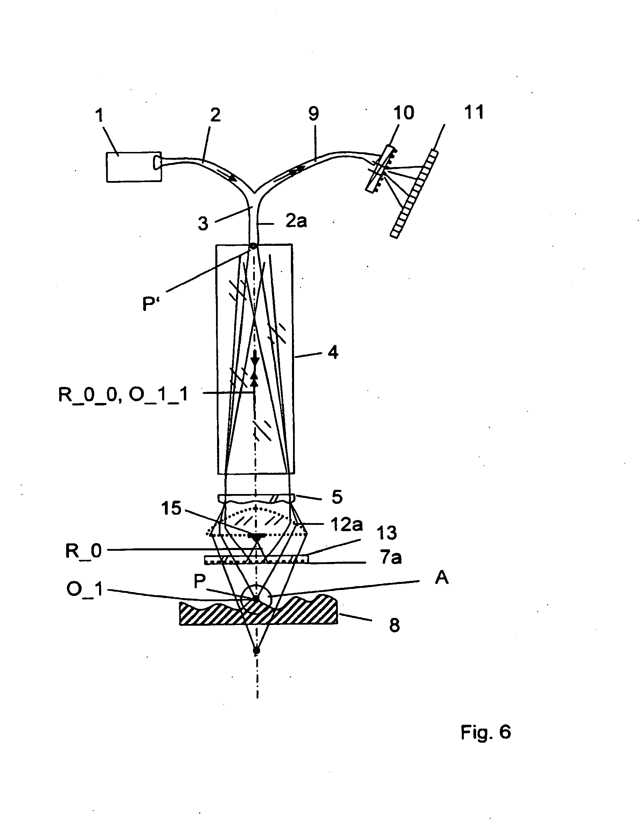

In **Figur 6** wird die Wirkung einer diffraktiv-optischen Zonenlinse 5 als brechkraftvariable Komponente in einer Common-path-Interferometeranordnung mit einem Mirau-Interferometer zur Tiefenmessung eines Objekts 8 dargestellt. Die Anordnung kann dabei auch mit einer Präzisionsführung gekoppelt sein, um das Feinprofil eines Objektes 8 messen zu können.

Das von einer Weißlichtquelle 1 ausgehende Licht, gelangt über eine Lichtleitfaser 2 und eine y-Weiche 3 über eine Grinlinse 4 zur Kollimierung des Lichtbündels auf eine diffraktiv-optische Zonenlinse 5 mit in Abhängigkeit von der Lichtwellenlänge variabler negativer Brechkraft, so dass diese als Zerstreuungslinse wirkt.

Das Lichtbündel wird an der diffraktiv-optischen Zonenlinse 5 in der nullten Beugungsordnung in einen Referenz-R_0 und in der ersten Beugungsordnung in einen schwach divergierenden Objektstrahl O_1 aufgespalten, die das Mikroobjektiv 12a passieren. Es entsteht - wie bei einem Mirau-Interferometer üblich - durch Reflexion an der Strahlteilerschicht 7a ein konvergierendes Referenzbündel R_0, wobei dieses hier aus dem Teilbündel der nullten Beugungsordnung der diffraktiv-optischen Zonenlinse 5 gebildet wird. Dieses wird am Referenzspiegel 15 reflektiert. Das an der an Strahlteilerplatte 13 mit Strahlteilerschicht 7a hindurchtretende Bündel leitet sich aus dem Teilbündel der ersten Beugungsordnung der diffraktiv-optischen Zonenlinse 5 ab und, stellt das Objektbündel O_1 dar. Das Bündel der ersten Beugungsordnung im Referenzstrahlengang und das Bündel der nullten Beugungsordnung im Objektstrahlengang werden jeweils durch konfokale Diskriminierung im Rücklauf am Eingang der Faser 2a ausgeblendet. Durch die wellenlängenabhängige Brechkraft der diffraktiven lichtzerstreuenden Zonenlinse 5 führt dies für das Objektbündel O_1 im Objektraum zu einer chromatischen Längsaufspaltung im Bereich Dz, wobei die Foki des langwelligen Lichts am Weitesten entfernt sind. So erfolgt eine Tiefenabtastung des Objektraumes. Das von einem Punkt des Objekts 8 reflektierte Licht passiert die diffraktiv-optische Zonenlinse 5 in der ersten Beugungsordnung und wird zum Teilbündel O_1_1. Das Referenzbündel R_0 durchsetzt die diffraktiv-optische Zonenlinse 5 wieder in der nullten Beugungsordnung und wird zum Teilbündel R_0_0. Nach dem Passieren des Objektivs 12a entstehen - je nach Wellenlänge - Objektbündel unterschiedlicher Wellenfrontkrümmung. Nur die zumindest näherungsweise im Punkt A fokussierten Teilbündel O_1_1 treten in das Endstück der Faser 2a gemeinsam mit dem Referenzbündel R_0_0 wieder ein, passieren die Weiche 3 in Richtung der Auskoppelfaser 9. gelangen auf ein hochempfindliches fasergekoppeltes Spektrometer 10 und auf eine hochempfindliche Zeilen kamera 11. Das entstehende Spektrum über der Wellenzahl ist in Figur 3 qualitativ dargestellt. Die Signalauswertung kann mittels FFT, s. a. **Figur 4,** erfolgen, da die Wellenzahl dem optischen Gangunterschied im Mirau-Interferometers und damit auch dem optischen Abstand (z) von der Strahlteilerschicht 7a indirekt proportional ist. Es ist aber auch möglich, die Phase des Spektrums über der Wellenzahl auszuwerten, um eine höhere z-Genauigkeit zu erreichen, beispielsweise zur Abstandsmessung mit Nanometergenauigkeit in einer Koordinatenmessmaschine. Es ist möglich eine größere Anzahl von Fasern 2a lateral in einer Linie anzuordnen, um einen Profilschnitt auswerten zu können. Das fasergekoppelte Spektrometer 10 besitzt dann einen Eingangsspalt und eine Flächenkamera 11.

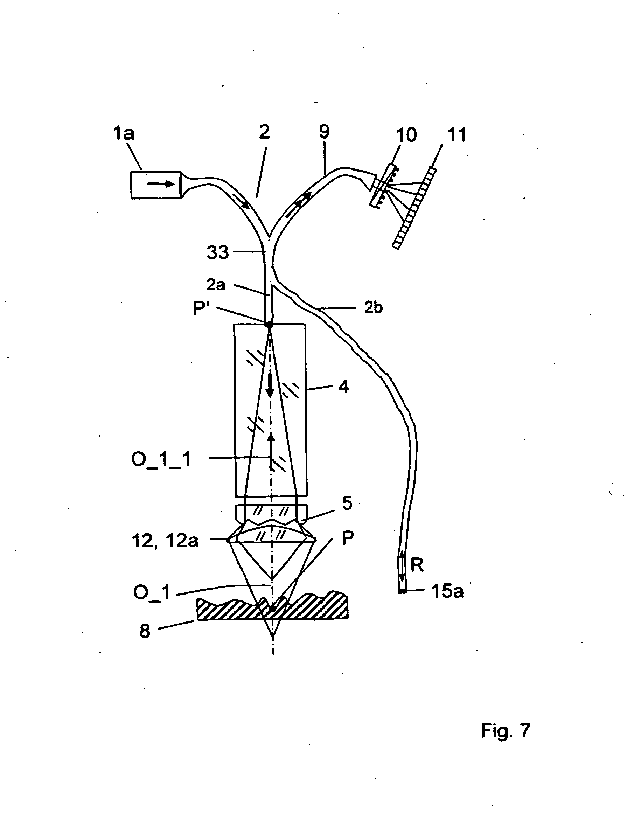

Die **Figur 7** zeigt die Wirkung einer diffraktiv-optischen Zonenlinse 5 als brechkraftvariable Komponente in einem fasergekoppelten Michelson-Interferometer zur Tiefenmessung eines Objekts 8, bzw. auch zur Erfassung von Defekten wie Einschnürungen auf einer Objektoberfläche. Das von einer Weißlichtquelle 1 ausgehende Licht, gelangt über eine Lichtleitfaser 2 und einen x-Koppler 33 über eine Grinlinse 4 zur Kollimierung des Lichtbündels auf eine diffraktiv-optische Zonenlinse 5 mit in Abhängigkeit von der Lichtwellenlänge variabler negativer Brechkraft, so dass diese als Zerstreuungslinse wirkt. Das Lichtbündel wird an der diffraktiv-optischen Zonenlinse 5 in der ersten Beugungsordnung in einen schwach divergierenden Objektstrahl O_1 aufgespalten, der das Mikroobjektiv 12a passiert und das Objektbündel O_1 dar stellt, welches das Objekt 8 beleuchtet. Durch die wellenlängenabhängige Brechkraft der diffraktiven, lichtzerstreuenden Zonenlinse 5 führt dies für das Objektbündel O_1 im Objektraum zu einer chromatischen Längsaufspaltung, wobei die Foki des langwelligen Lichts am Weitesten entfernt sind. So erfolgt eine Tiefenabtastung des Objektraumes. Das von einem Punkt P des Objekts 8, hier beispielsweise an der Stelle einer Einschnürung, reflektierte Licht passiert die diffraktiv-optische Zonenlinse 5 wieder in der ersten Beugungsordnung. Nur die zumindest näherungsweise im Punkt P fokussierten Teilbündel O_1_1 treten in das Endstück der Faser 2a wieder ein und passieren den x-Koppler 33 in Richtung der Auskoppelfaser 9. Das Referenzbündel R, welches im Referenzstrahlengang in der Faser 2b am Referenzspiegel 15a nach Reflexion entsteht, passiert den x-Koppler 33 ebenfalls in Richtung der Auskoppelfaser 9. Referenzlicht und Objektlicht gelangen nach dem Durchsetzen der Auskoppelfaser 9 auf ein hochempfindliches fasergekoppeltes Spektrometer 10 und auf eine hochempfindliche Zeilenkamera 11. Dort wird das Spektrum des interferierenden Lichts von Referenz und Objekt ausgewertet und mittels einer schnellen Fourier-Transformation (FFT) des Intensitätssignals, welches über der Wellenzahl dargestellt ist, wird die mittlere Frequenz des Intensitätssignals über der Wellenzahl als Maß für den Objektabstand bestimmt. Dabei ist die optische Länge der Faser 2b so abgestimmt, dass der optische Gangunterschied im fasergekoppelten Michelson-Interferometer nur maximal einige 100 $\mu$m beträgt, jedoch nicht null ist.

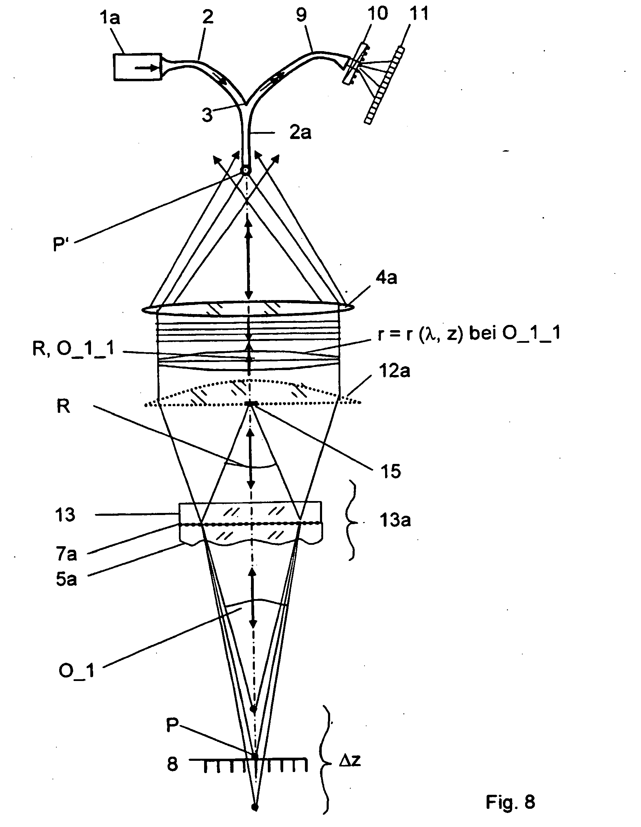

Die **Figur 8** stellt eine Anwendung für einen hochgenauen Punktsensor dar. Die Lichtquelle ist als Weißlicht-Kontinuums-Laser 1 a ausgebildet. Das Licht des Weißlicht-Kontinuums-Lasers 1a wird mit hier nicht dargestellter Fokussieroptik fokussiert und in eine Faser 2 eingekoppelt, passiert die Y-Weiche 3 und gelangt über das Faserstück 2a als divergierendes Strahlenbündel auf ein Kollimatorobjektiv 4a. Es entstehen zumindest näherungsweise plane Lichtwellen, die auf ein Objektiv 12a eines Mirau-Interferometers gelangen und mittels diesem in den Objektraum fokussiert werden. Zwischen dem Objektiv 12a des Mirau- Interferometers und dem Objektraum befindet sich die Strahlteilerplatte 13 mit Strahlteilerschicht 7a und die diffraktive lichtzerstreuende Zonenlinse 5a, die zu einer Kittgruppe 13a vereinigt sind. Es entsteht - wie bei einem Mirau-Interferometer üblich - durch Reflexion an der Strahlteilerschicht 7a ein konvergierendes Referenzbündel R, das am Referenzspiegel 15 reflektiert wird. Das an Strahlteilerplatte 13 mit Strahlteilerschicht 7a hindurchtretende Bündel passiert nun in der ersten Ordnung die diffraktive lichtzerstreuende Zonenlinse 5a. Durch die wellenlängenabhängige Brechkraft der diffraktiven lichtzerstreuenden Zonenlinse 5a führt dies im Objektraum für das Objektbündel O_1 zu einer chromatischen Längsaufspaltung im Bereich Dz, wobei die Foki des langweiligen Lichts am Weitesten entfernt von der Kittgruppe 13a verschoben sind. So erfolgt eine Tiefenabtastung des Objektraumes. Das von einem Punkt P des Objekts 8 reflektierte Licht gelangt über die diffraktive lichtzerstreuende Zonenlinse 5a, wobei hier die Transmission des Lichtes wieder in der ersten Beugungsordnung erfolgt, durch die Strahlteilerplatte 13 mit Strahlteilerschicht 7a gemeinsam mit dem Referenzbündel R in das Objektiv 12a des Mirau-Interferometers. Nach dem Passieren dieses Objektivs 12a entstehen - je nach Wellenlänge - Objektbündel unterschiedlicher Wellenfrontkrümmung. Nur die zumindest näherungsweise im Punkt P fokussierten Teilbündel treten in das Endstück der Faser 2a wieder ein, passieren die Weiche 3 in Richtung der Auskoppelfaser 9, gelangen auf ein hochempfindliches fasergekoppeltes Spektrometer 10 und auf eine hochempfindliche Zeilenkamera 11. Das entstehende Spektrum über der Wellenzahl ist in Figur 3 qualitativ dargestellt. Die Signalauswertung kann mittels FFT, s. a. Figur 4, erfolgen, da die Wellenzahl dem optischen Gangunterschied im Mirau-Interferometers und damit auch dem optischen Abstand (z) von der Strahlteilerschicht 7a indirekt proportional ist. Es ist aber auch möglich, die Phase des Spektrums über der Wellenzahl auszuwerten, um eine höhere z-Genauigkeit zu erreichen, beispielsweise zur Abstandsmessung in einer Nano-Messmaschine.

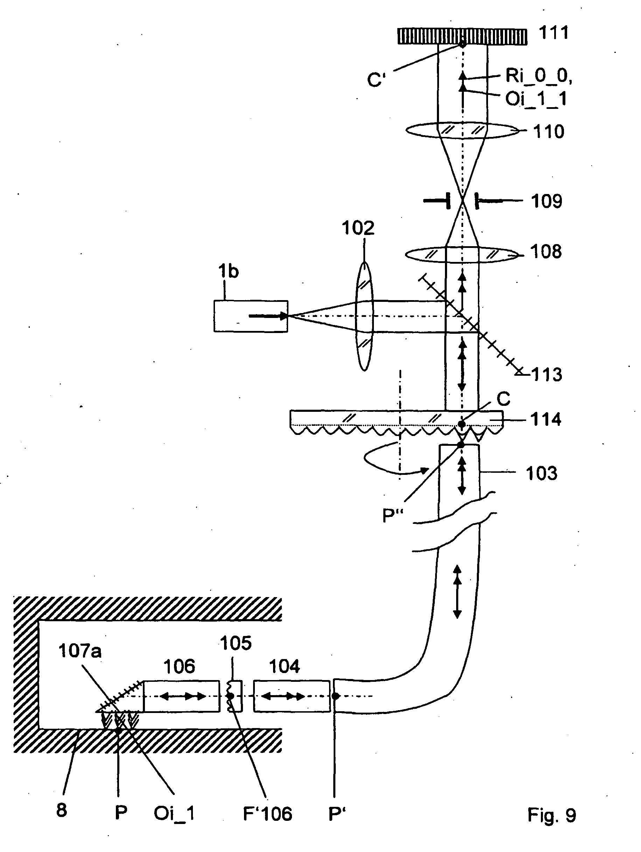

Die **Figur 9** zeigt eine Anordnung für die vorzugsweise flächige Erfassung der Mikro-Topografie. Das von einem Wellenlängen-durchstimmbaren Laser 1b ausgehende Licht wird durch einen Kollimator 102 kollimiert und am Strahlteiler 113 reflektiert und durchsetzt eine rotierende Mikrolinsenscheibe 114. Die Foki der Mikrolinsen sind scharf auf das Ende eines Bildleiters 103 abgebildet. Am Ende des Bildleiters 103 werden aus den übertragenen Fokusbildern mittels Grinlinse 104 mehrere kollimierte Bündel erzeugt, die auf eine diffraktiv-optische Zonenlinse 105 mit in Abhängigkeit von der Lichtwellenlänge variabler negativer Brechkraft gelangen, so dass diese als wellenlängenabhängig starke Zerstreuungslinse wirkt. Die Lichtbündel werden an der diffraktiv-optischen Zonenlinse 105 in der nullten Beugungsordnung in mehrere Referenzstrahlenbündel Ri_0 und in der ersten Beugungsordnung in mehrere schwach divergierende Objektstrahlenbündel Oi_1 aufgespalten. Die Referenzstrahlenbündel Ri_0 werden auf die Strahlteilerfläche 107a scharf fokussiert. Die Objektstrahlenbündel Oi_1 weisen im Objektraum eine chromatische Längsaufspaltung im Bereich Dz auf, wobei die Foki des langwelligen Lichts am Weitesten entfernt sind. So erfolgt eine Tiefenabtastung des Objektraumes mit mehreren Foki. Das vom Objektraum reflektierte Licht passiert in mehreren Bündeln wieder die diffraktiv-optische Zonenlinse 105 in der ersten Beugungsordnung. Die an der Strahlteilerfläche 107a reflektierten Referenzstrahlenbündel Ri_0 passieren wieder die diffraktiv-optische Zonenlinse 105 in der nullten Beugungsordnung. Es entstehen an der diffraktiv-optischen Zonenlinse 105 auch noch weitere Teilbündel. Jedoch werden diese im weiteren Strahlengang mittels konfokaler Diskriminierung an der Blende 109 nahezu vollständig eliminiert. Die reflektierten Referenzstrahlenbündel Ri_0_0 und die scharf auf Objektpunkte fokussierten und reflektierten Objektstrahlenbündel Oi_1_1 kommen zur Interferenz und werden mittels Grinlinse 104 auf das Ende eines Bildleiters 103 ebenfalls scharf fokussiert, passieren diesen Bildleiter 103 und durchsetzen wieder die rotierende Mikrolinsenscheibe 114. Anschließend passieren die Bündel den Strahlteiler 113 in Reflexion und werden durch ein Fokussierobjektiv 108 auf die konfokale Blende 109 fokussiert. Die konfokale Blende 109 passieren nur das Referenzstrahlenbündel Ri_0_0 und auf das Objekt 8 scharf fokussierte Anteile des Objektstrahlenbündels Oi_1_1. Hier erfolgt also die konfokale Diskriminierung der unerwünschten Teilbündel, beispielsweise das in der nullten Ordnung im Rücklauf vom Objekt an der diffraktiv-optischen Zonenlinse 105 entstehende Objektstrahlenbündel. Das die konfokale Blende 109 passierende Licht (Ri_0_0 und Oi_1_1) wird durch das Objektiv 110 kollimiert und zur Auswertung scharf auf die CMOS-Kamera 111 abgebildet. Beim Wellenlängen-Scan des Lasers 1 b entsteht in jedem Pixel ein Wavelet gemäß Figur 3, das in der bekannten Art, beispielsweise mittels FFT, ausgewertet wird, so dass sich für jeden Objektpunkt die genaue Tiefenposition ergibt.

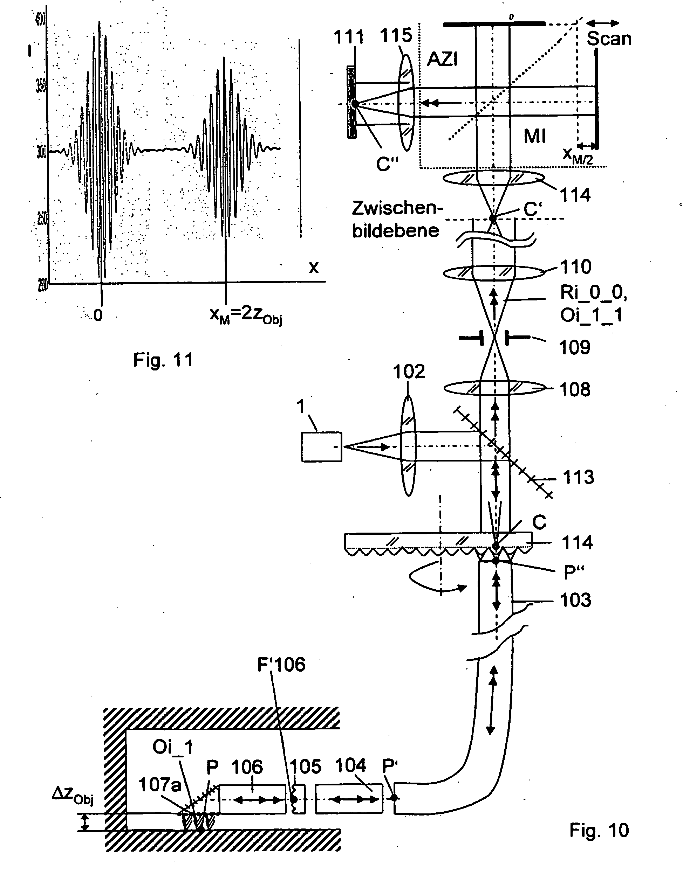

Die **Figur 10** zeigt eine Anordnung für die vorzugsweise flächige Erfassung der Mikro-Topografie. Das von einer lichtstarken, nahezu punktförmigen WeißlichtQuelle 1 ausgehende Licht wird durch einen Kollimator 102 kollimiert und am Strahlteiler 113 reflektiert und durchsetzt eine rotierende Mikrolinsenscheibe 114. Die Foki der Mikrolinsen sind scharf auf das Ende eines Bildleiters 103 abgebildet. Am Ende des Bildleiters 103 werden aus den übertragenen Fokusbildern mittels Grinlinse 104 mehrere kollimierte Bündel erzeugt, die auf eine diffraktiv-optische Zonenlinse 105 mit in Abhängigkeit von der Lichtwellenlänge variabler negativer Brechkraft gelangen, so dass diese als wellenlängenabhängig starke Zerstreuungslinse wirkt. Die Lichtbündel werden an der diffraktiv-optischen Zonenlinse 105 in der nullten Beugungsordnung in mehrere Referenzstrahlenbündel Ri_0 und in der ersten Beugungsordnung in mehrere schwach divergierende Objektstrahlenbündel Oi_1 aufgespalten. Die Referenzstrahlenbündel Ri_0 werden auf die Strahlteilerfläche 107a scharf fokussiert. Die Objektstrahlenbündel Oi_1 weisen im Objektraum eine chromatische Längsaufspaltung auf, wobei die Foki des langwelligen Lichts am Weitesten entfernt sind. So erfolgt eine Tiefenabtastung des Objektraumes mit mehreren Foki. Ein Objektpunkt P befindet sich in der Entfernung Dz von der Strahlteilerfläche 107a. Das vom Objektraum reflektierte Licht, auch das vom Punkt P, passiert in mehreren Bündeln wieder die diffraktiv-optische Zonenlinse 5 in der ersten Beugungsordnung. Die an der Strahlteilerfläche 107a reflektierten Referenzstrahlenbündel Ri_0 passieren wieder die diffraktiv-optische Zonentinse 105 in der nullten Beugungsordnung. Weitere Teilstrahlenbündel entstehen an der diffraktivoptischen Zonenlinse 105 ebenfalls. Jedoch werden diese im weiteren Strahlengang mittels konfokaler Diskriminierung an der Blende 109 nahezu vollständig eliminiert. Die reflektierten Referenzstrahlenbündel Ri_0 und die scharf auf Objektpunkte fokussierten und reflektierten Objektstrahlenbündel Oi_1 kommen zur Interferenz und werden mittels Grinlinse 104 auf das Ende eines Bildleiters 103 ebenfalls scharf fokussiert, so dass auch der Bildpunkt P' entsteht, passieren diesen Bildleiter 103 und durchsetzen wieder die rotierende Mikrolinsenscheibe 104, wo in einem Fokus der Bildpunkt P'' entsteht. Das Licht desselben leuchtet die Pupille der zugehörigen Mikrolinse mit dem Pupillenzentrum C aus. Anschließend passieren die Strahlenbündel von Referenz und Objekt den Strahlteiler 113 in Transmission und werden durch ein Fokussierobjektiv 108 auf die konfokale Blende 109 fokussiert. Hier erfolgt die bereits erwähnte konfokale Diskriminierung der unerwünschten Teilbündel, beispielsweise das in der nullten Ordnung im Rücklauf vom Objekt an der diffraktiv-optischen Zonenlinse 105 ebenfalls entstehende Objektstrahlenbündel. Das die konfokale Blende 109 passierende Licht, das sind das Referenzbündel Ri_0_0 und der auf das Objekt 8 scharf fokussierte Anteile des Objektstrahlenbündels Oi_1_1. wird durch das Objektiv 110 kollimiert und tritt in ein analysierendes Zweistrahl-Interferometer AZI vom Michelson-Typ ein. In diesem wird das Zwischenbild aus dem Common-path-Interferometer mittels Objektiv 114 nach Unendlich abgebildet. Durch Abbildung mittels Objektiv 115 entsteht schließlich auf der CMOS-Kamera 111 beim Spiegel-Scan des AZI in jedem Pixel, der einem Bildpunkt C'' entspricht, ein Weißlicht-Interferogramm, dessen mittleres Maximum bei dem optischen Gangunterschied xM liegt, wo der optische Gangunterschied $2\Delta z_{Obj}$ genau dem im Common-path-Interferometer entspricht. Es wird mittels geeigneter Auswertung des Weißlicht-Interferogramms die Lage des Mittenpeaks desselben auf wenige Nanometer genau bestimmt.

Die **Figur 11** zeigt ein - mittels der Anordnung nach Figur 10 - aus einem spektral kodierten Lichtbündel - mittels nachgeordnetem scannendem Zweistrahl-Interferometer AZI - rekonstruiertes Weißlicht-Interferogramm in der Umgebung der xM-Position für einen Objektpunkt P. Die Erzeugung von Weißlicht-Interferogrammen mittels einem - dem ersten Interferometer zur Objekterfassung nachgeordneten - statischen Michelson-Interferometer und dessen Nutzung für die Objekt-Tiefenabtastung wurde bereits in oben beschrieben. Die dazu notwendigen Auswertemethoden wurden in Applied Optics, Vol. 39, No. 8, 10. März 2000, Seite 1290 bis 1295 sowie im Fachaufsatz "Signalverarbeitung bei tiefen-scannenden 3D-Sensoren für neue industrielle Anwendungen" in der Fachzeitschrift Technisches Messen 69 (2002) 5 dargestellt. So kann für jeden Objektpunkt P oder eine Vielzahl von Objektpunkten die Tiefenposition $\Delta z_{Obj}$ genau bestimmt werden.

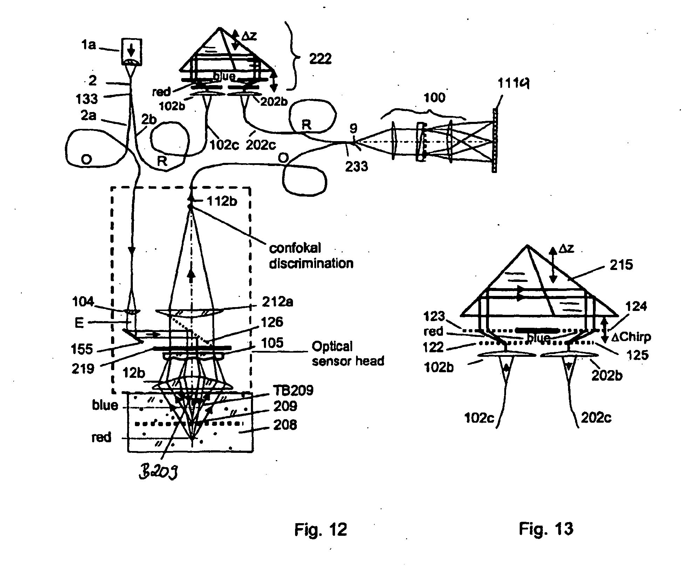

Zu **Figur 12:** Das von einem vergleichsweise starken Kontinuums-Weißlichtlaser 1a ausgehende Licht gelangt über eine Lichtleitfaser 2 und einen Y-Koppler 133, dann als Objektlicht O über ein Faserstück 2a und über eine Grinlinse 104 zur Kollimierung des Lichtbündels E, über einen Umlenkspiegel 155 und einen Strahlteiler 126, hier zur Einkopplung, in das Fokussier-System auf eine diffraktiv-optische Zonenlinse 105 mit in Abhängigkeit von der Lichtwellenlänge variabler negativer Brechkraft in der ersten Beugungsordnung, so daß diese als Zerstreuungslinse wirkt. Das chromatisch aufgespaltete Licht gelangt auf ein gut chromatisch korrigiertes Fokussier-Objektiv 12b.

Durch die wellenlängenabhängige Brechkraft der diffraktiven lichtzerstreuenden Zonenlinse 105 führt dies für das Lichtbündel nach dem Fokussier-Objektiv 12b zu einer chromatischen Längsaufspaltung, wobei hier die Foki des langwelligen Lichts am Weitesten vom Fokussier-Objektiv 12b im Objektvolumen 208 entfernt sind. Das Fokussier-System wird also von der diffraktiv-optischen Zonenlinse 105 und dem Fokussier-Objektiv 12b gebildet.

Dem optischen Abtastkopf ist zur Erzeugung einer Relativbewegung des Abtastbündels zum Objekt ein lateral arbeitender 2D-Scanner 219, also im Objektvolumen ein X-Y-Scanner zugeordnet, der hier aber nur symbolisch dargestellt wurde. Dieser 2D-Scanner 219 kann auch eine eigene, hier nicht dargestellte Abbildungsstufe beinhalten.

Das Objektdetail 209 wird von einem Fokusfleck eines Bündels einer bestimmten Wellenlänge getroffen. Auch für das Objektdetail 209 gibt es also genau ein Lichtbündel B209, welches eine genau bestimmte Wellenlänge aufweist und einen scharfen Fokus auf Objektdetail 209 ausbildet. Dieser Fokusfleck wird am Objektdetail 209 wenigstens teilweise reflektiert oder gestreut. So gelangt ein Teil des Objektlichtes als Teilbündel TB209 über das Fokussier-Objektiv 12b und über die diffraktiv-optische Zonenlinse 105, wobei das Teilbündel TB209 die Zonenlinse 105 genau in derselben ersten Beugungsordnung passiert wie im Hinlauf. Über den Strahlteiler 126 in Transmission gelangt das Teilbündel TB209 auf die Fokussierlinse 212a und auf das Ende der Faser 112b, welches die konfokale Diskriminierung realisiert. Das Fokussier-System zur Detektion wird also vom Fokussier-Objektiv 12b, der diffraktiv-optischen Zonenlinse 105 und der Fokussierlinse 212a gebildet.

Das konfokal diskriminierte Licht tritt also in die Faser 112b ein. So wird jedes Objektdetail im Objektvolumen 208 von einem Lichtbündel passender Wellenlänge optisch zumindest näherungsweise beugungsbegrenzt abgetastet und konfokal diskriminiert. Das in die Faser 112b eintretende Licht passiert den X-Koppler 233 in Richtung der Auskoppelfaser 9.

Das Referenzbündel R im Referenzstrahlengang tritt aus der Faser 2b aus, siehe **Figur 13,** erfährt eine Kollimation an der Grinlinse 102b und passiert die Phasengitter 122 und 123 jeweils in der ersten Beugungsordnung, wobei eine wellenlängenabhängige Ablenkung auftritt und sich so der optische Weg - und damit der optische Gangunterschied im Interferometer - wellenlängenabhängig ändert. Dies führt bei entsprechender Einstellung zur gewünschten Form des Wavelets. Beispielsweise kann durch Verschieben des Phasengitters 123 der optische wellenlängenabhängige optische Gangunterschied - und damit die Beeinflussung der Form des Wavelets - vergrößert oder verkleinert werden. Die dabei auch auftretende Gangunterschiedsvariation für alle Wellenlängen, also der konstante Anteil des optischen Gangunterschieds, kann bei Bedarf durch das Verschieben des Tripelspiegelreflektors 215 kompensiert werden. Der für die Signalauswertung optimale wellenlängenunabhängige Gangunterschied wird also am Tripelspiegelreflektor 215 durch zumindest näherungsweise paralleles Verschieben zur optischen Achsen der Grinlinse 102b eingestellt. So kann also der konstante Anteil und der wellenlängenabhängige Anteil des Gangunterschieds jeweils einzeln eingestellt werden, so daß sich eine für die Auswertung optimale Form und Frequenz von Wavelets ergibt.

Nach Reflexion am Tripelspiegelreflektor 215 und den beiden weiteren Phasengittern 124 und 125, die in gleicher Weise wie die beiden Phasengitter 122 und 124 wirken, und aus Symmetriegründen angeordnet sind, passiert das Referenzlicht über die Grinlinse 202b und die Faser 202c den X-Koppler 233 ebenfalls in Richtung der Auskoppelfaser 9, siehe Figur 12. Referenzlicht R und Licht O aus dem Objektvolumen 208 gelangen nach dem Durchsetzen der Auskoppelfaser 9 auf ein hochempfindliches fasergekoppeltes Spektrometer 100 und auf eine hochempfindliche Fotodiodenzeile 111a und kommen dort zur Interferenz. Mittels Fotodiodenzeile 111a wird das Spektrum des interferierenden Lichts ausgewertet.

Dabei ist der optische Gangunterschied im fasergekoppelten Interferometer mittels Tripelspiegelreflektors 215 so abgestimmt, daß dieser nur maximal einige 100 μm beträgt, jedoch nicht null ist.

Die Auswertung erfolgt, indem zum einen die Größe der Signalamplitude des Wavelets in möglichst einfacher Art bestimmt wird, da die Frequenz des Wavelets zumindest näherungsweise bekannt ist. Dies kann im einfachsten Fall durch das Auswerten von einigen Sensorelementen erfolgen, die das Wavelet so abtasten, daß jeweils Signalwerte detektiert werden, die beispielsweise eine Viertel Periode verschoben sind. Die Größe der Signalamplitude ist ein Indiz für die Stärke der Reflexion des jeweils erfaßten Objektdetails 209. Weiterhin wird, um auch die Tiefenposition dieses lichtstreuenden Objektdetails 209 bestimmen zu können, das Mittenmaximum der Einhüllenden bestimmt beispielsweise durch Auswerten des Schwerpunktes desselben. Die Auswertung kann andererseits auch mittels einer schnellen Fourier-Transformation (FFT) des Wavelets erfolgen.

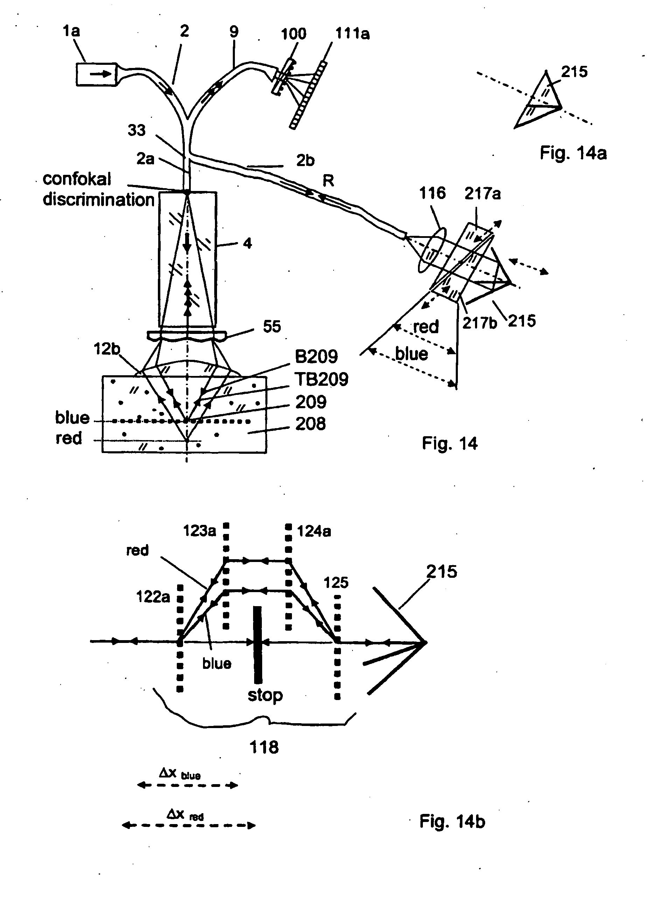

Zu **Figur 14:** Das von einem vergleichsweise starken Kontinuums-Weißlichtlaser 1a ausgehende Licht gelangt über eine Lichtleitfaser 2 und einen X-Koppler 33, als Objektteillicht über ein Faserstück 2a und über eine Grinlinse 4 zur Kollimierung des Lichtbündels auf eine diffraktiv-optische Zonenlinse 55 mit in Abhängigkeit von der Lichtwellenlänge variabler negativer Brechkraft in der ersten Beugungsordnung, so daß diese als Zerstreuungslinse wirkt. Das chromatisch aufgespaltete Licht gelangt auf ein gut chromatisch korrigiertes Fokussier-Objektiv 12b.

Durch die wellenlängenabhängige Brechkraft der diffraktiven lichtzerstreuenden Zonenlinse 55 führt dies für das Lichtbündel nach dem Fokussier-Objektiv 12b zu einer chromatischen Längsaufspaltung, wobei hier die Foki des langwelligen Lichts am Weitesten vom Fokussier-Objektiv 12b entfernt sind.

Das Objektdetail 209 wird von einem Fokusfleck eines Bündels einer bestimmten Wellenlänge getroffen. Auch für das Objektdetail 209 gibt es also genau ein Lichtbündel B_209, welches eine genau bestimmte Wellenlänge aufweist und einen scharfen Fokus auf dem Objektdetail 209 ausbildet. Dieser Fokusfleck wird am Objektdetail 209 wenigstens teilweise reflektiert oder gestreut. So gelangt ein Teil des Objektlichtes als Teilbündel TB209 über das Fokussier-Objektiv 12b und über die diffraktiv-optische Zonenlinse 55, wobei das Teilbündel TB209 die Zonenlinse 55 genau in derselben ersten Beugungsordnung passiert wie im Hinlauf. Das Licht gelangt nun über die Grinlinse 4 auf das Ende der Faser 2a, welches die konfokale Diskriminierung realisiert.

Das in die Faser 2a eintretende Licht passiert den X-Koppler 33 in Richtung der Auskoppelfaser 9. Das Referenzbündel R, welches im Referenzstrahlengang in der Faser 2b nach Austritt aus derselben und Kollimation am Kollimator 116 und nach Reflexion am Tripelspiegelreflektor 215 entsteht, passiert den X-Koppler 33 ebenfalls in Richtung der Auskoppelfaser 9.

Im Referenzarm des Interferometers sind zwei Prismen 217a und 217b angeordnet, die optisch eine Planparallelplatte im kollimierten Strahlengang bilden, deren optische Dicke durch Schiebung der Prismen 217a und 217b veränderlich gemacht ist. Wegen des größeren Brechungsindexes des Plattenmaterials bei kürzeren Wellenlängen vergrößert sich dieser entsprechend bei kürzeren Wellenlängen. Dies kann zur gezielten Beeinflussung des optischen Gangunterschieds in Abhängigkeit von der Wellenlänge im Interferometer genutzt werden. Damit ist die Möglichkeit gegeben, die Frequenz der Wavelets über der Wellenlänge gezielt zu beeinflussen und somit die unerwünscht starke Vergrößerung der Frequenz der Wavelets in Richtung kürzerer Wellenlängen erheblich zu verringern. In der **Figur 14a** ist ein Tripelprismenreflektor 215 dargestellt, der so die refraktive Platte bereits integriert hat und somit auch dispersiv ist, um eine Variation des optischen Gangunterschieds in Abhängigkeit von der Wellenlänge zu ermöglichen.

Referenzlicht und Licht aus dem Objektvolumen 208 gelangen nach dem Durchsetzen der Auskoppelfaser 9 auf ein hochempfindliches, fasergekoppeltes Spektrometer 100 - wobei hier die zugehörige Abbildungsoptik des Spektrometers nicht dargestellt wurde - und auf eine hochempfindliche Fotodiodenzeile 111a und dort zur Interferenz. Dort wird das Spektrum des interferierenden Lichts ausgewertet.

Die Auswertung erfolgt, indem die Größe der Signalamplitude des Wavelets bestimmt wird. Weiterhin wird der Schwerpunkt der Einhüllenden des Wavelets auf der Wellenlängenachse des Spektrometers bestimmt.

Dabei ist der optische Gangunterschied im fasergekoppelten Interferometer mittels Tripelspiegelreflektors 215 so abgestimmt, daß dieser nur maximal einige 100 μm beträgt, jedoch nicht null ist. Die Auswertung kann andererseits auch mittels einer schnellen Fourier-Transformation (FFT) des Wavelets erfolgen.

Die **Figur 14b** stellt die Möglichkeit dar, mittels Diffraktion an vier Liniengittern 118, die als Phasengitter in paralleler Lage zueinander und hier senkrecht zur optischen Achse, ausgebildet sind, eine Variation des optischen Gangunterschieds über der Wellenlänge zu erzeugen, wobei hier das langweiligere Licht den größeren optischen Gangunterschied aufweist. Damit kann die diffraktiv-optische Zonenlinse 55 in Figur 14 mit negativer Brechkraft ausgebildet sein, wodurch die Foki des langwelligeren Lichtes den größeren Abstand vom System aufweisen und damit auch bei Interferenz den größeren optischen Gangunterschied. Dieser größere optische Gangunterschied kann mittels der Gitteranordnung 118, bestehend aus vier Liniengittern 122a, 123a, 124a und 125a, wenigstens zum Teil kompensiert werden.

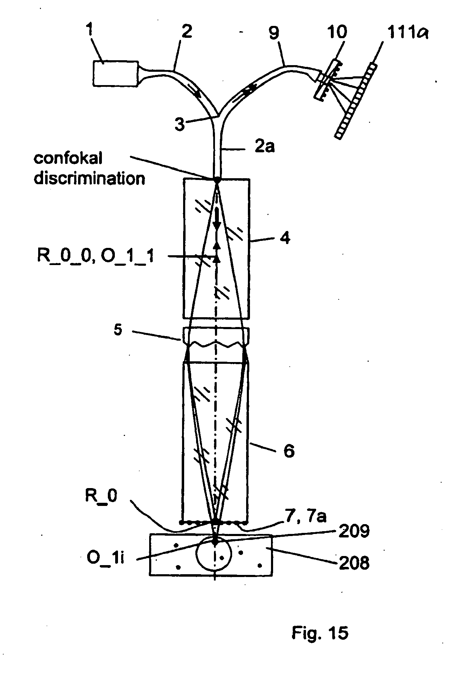

Zu **Figur 15:** Das von einer Weißlichtquelle 1 ausgehende Licht gelangt über eine Lichtleitfaser 2 und eine Y-Weiche 3 und über ein Faserstück 2a über eine Grinlinse 4 zur Kollimierung des Lichtbündels auf eine diffraktiv-optische Zonenlinse 5 mit in Abhängigkeit von der Lichtwellenlänge variabler negativer Brechkraft, so daß diese als Zerstreuungslinse wirkt.

Das Lichtbündel wird an der diffraktiv-optischen Zonenlinse 5 in der nullten Beugungsordnung in einen Referenz-R und in der ersten Beugungsordnung in mehrere schwach divergierende Objektstrahlenbündel aufgespalten, so auch in ein Objektstrahlenbündel O_1i. Dabei ist die nachfolgende Grinlinse 6 in ihrem geometrisch-optischen Aufbau so abgestimmt, daß die Frontfläche 7 der Grinlinse 6, die als Referenzteilspiegel mit Strahlteilerschicht 7a wirkt, im Fokus des Bündels der nullten Beugungsordnung der diffraktiv-optischen Zonenlinse 5 liegt. Das an der Frontfläche 7 an der Strahlteilerschicht 7a reflektierte Teillichtbündel dient als Referenzbündel R. Das in der 1. Beugungsordnung die diffraktiv-optische Zonenlinse 5 passierende Teillichtbündel O_1i durchsetzt die Frontfläche 7 der Grinlinse 6, welche die Referenzspiegelfläche darstellt, und gelangt in das Objektvolumen 208. Durch die brechkraftvariable Wirkung der diffraktiv-optischen Zonenlinse 5 erfolgt in Abhängigkeit von der Lichtwellenlänge die Fokussierung des Objektstrahlenbündels in unterschiedlichen Tiefen des Objektvolumens 208. Es besteht also eine chromatische Längsaberration oder Längsaufspaltung. Das aus dem Objektvolumens 208 vom Objektdetail 209 rückreflektierte Licht passiert gemeinsam mit dem Referenzbündel R wieder die Grinlinse 6 und passiert die diffraktiv-optische Zonenlinse 5 wieder in der ersten Beugungsordnung und wird zum Objektstrahlenbündel O_1_1i. Das an der Frontfläche 7, die als Referenzspiegelfläche wirkt, fokussierte Licht wird zum Teil reflektiert und passiert die diffraktiv-optische Zonenlinse 5 wieder in der nullten Beugungsordnung und wird zum Referenzstrahlenbündel R_0_0. So bestehen zwei interferierende Bündel O_1_1i und R_0_0, die mittels Grinlinse 4 auf das Ende der Monomodefaser 2 wieder fokussiert werden und in diese Monomodefaser 2 eintreten, wobei hier insbesondere für das Objektstrahlenbündel O_1_1i eine konfokale Diskriminierung erfolgt. Nach dem Passieren der Y-Weiche 3 gelangt das interferierende Licht über die Auskoppelfaser 9 auf ein Spektrometer 100 auf eine hochempfindliche Fotodiodenzeile 111a, die das entstehende Spektrum registriert. Dabei ist das Spektrum über der Wellenzahl moduliert - je nach den Bedingungen für konstruktive und destruktive Interferenz in Abhängigkeit von der Wellenlänge und dem optischen Gangunterschied am Objektpunkt - und bildet über der Wellenzahl mit dem reziproken Wert der Wellenlänge ein Wavelet.

Das auf der Fotodiodenzeile 111a entstehende Signal kann bei festem optischen Gangunterschied Dx in Abhängigkeit von der Wellenlänge Ii mit folgender Gleichung (3) mit der Modulation m berechnet werden:

$$I(\lambda_i) = I_{Ref} + I_{Obj}(\lambda_i) + 2\sqrt{I_{Ref} \cdot I_{Obj}(\lambda_i)} \cdot m \cdot \cos\left(2\pi \cdot \frac{\Delta x}{\lambda_i} + \varphi_0\right). \qquad (3)$$

Der Wert Δx kennzeichnet also den fest eingestellten Wegunterschied der interferierenden Wellenpakete, die aus der nullten Ordnung und der ersten Ordnung stammen, wobei der Wegunterschied Δx auch etwas von der Wellenlänge abhängen kann.

Der Einfluß der konfokalen Blende auf das Signal wird über folgende Formel (4) bestimmt:

$$I = \frac{\sin\left(\frac{u}{2}\right)}{\frac{u}{2}} \quad \text{mit} \quad u = \frac{2\pi}{\lambda} NA^2 z(\lambda) \tag{4}$$

Mit NA wird die numerische Apertur des Lichtkegels am Objekt Blende bezeichnet und z gibt die Objekthöhe an.

Im Spektrometer 100 ist die spektrale Auflösung mindestens so hoch zu wählen, daß das von einer Fotodiode der Fotodiodenzeile 111 a überdeckte Spektralintervall oder Wellenlängeninkrement $\Delta\lambda$l bei der Wellenlänge $\lambda$ klein genug ist, damit die Kohärenzlange $\lambda$, $\lambda = \lambda^2/\Delta\lambda$l, größer als der optische Gangunterschied $\Delta$x im Interferometer ist, damit Interferenz auftreten kann. Dieser optische Gangunterschied $\Delta$x beträgt bis zu mehreren 100 $\mu$m und kann je nach der spektralen Auflösung und photometrischer Empfindlichkeit des verwendeten Spektrometers größer oder kleiner gewählt werden.

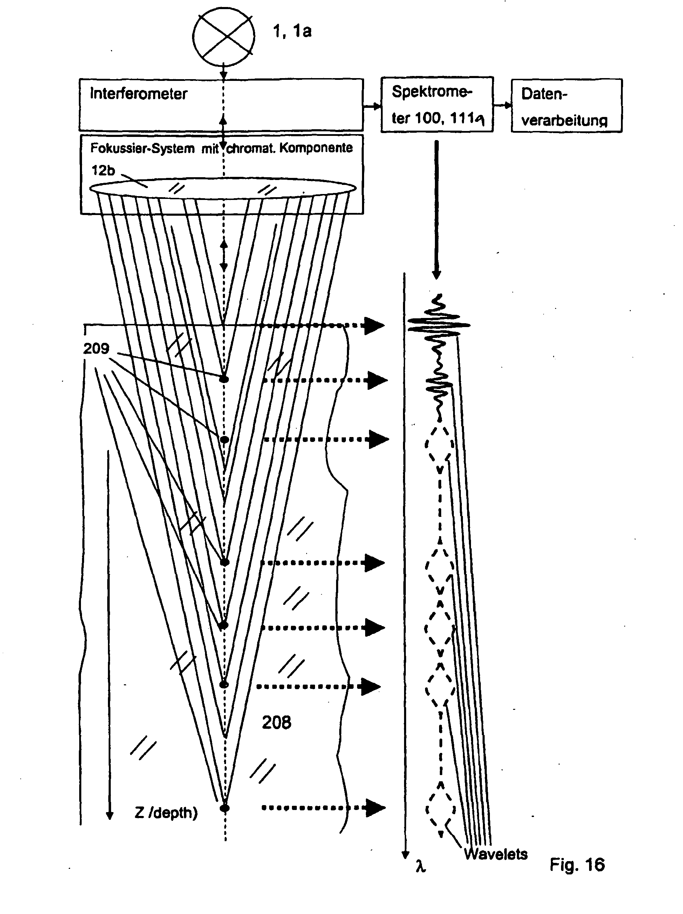

Die **Figur 16** stellt das Prinzip der chromatisch-konfokalen Spektral-Interferometrie mit integrierter chromatisch-konfokaler Tiefenaufspaltung dar. Es sind das Interferometer als Blackbox, das Fokussier-System mit integrierter chromatischer Komponente zur Tiefenaufspaltung der Foki und mit Fokussier-Objektiv 12b sowie das Objektvolumen 208 und die Blackbox der Datenverarbeitung dargestellt. Symbolisch sind mehrere Objektdetails 209 dargestellt. Auf der Wellenlängenachse, also der I-Achse, des Spektrometers 100 mit Empfänger 111a entstehen beim Vorhandensein von Objektdetails 209 im Objektvolumen 208 Wavelets. Außerdem entsteht auch ein Wavelet direkt von der Oberfläche des Objekts.

Damit ist es möglich von einem Detail einer Objektoberfläche oder von einem Detail des Objektinneren die drei Raumkoordinaten (x, y, z) sowie die Lichtintensität I für das vom Wavelet überdeckte Spektralintervall mit einer Schwerpunktwellenlänge zu erfassen, wobei die Schwerpunktwellenlänge verfahrensmäßig vorbestimmt veränderbar ist. Die Lichtintensität I im Spektralintervall wird zum einen von der Größe der Reflektivität, bzw. der Streuung des erfaßten Details bestimmt, andererseits von allen optischen Komponenten und Details des gesamten Lichtweges.

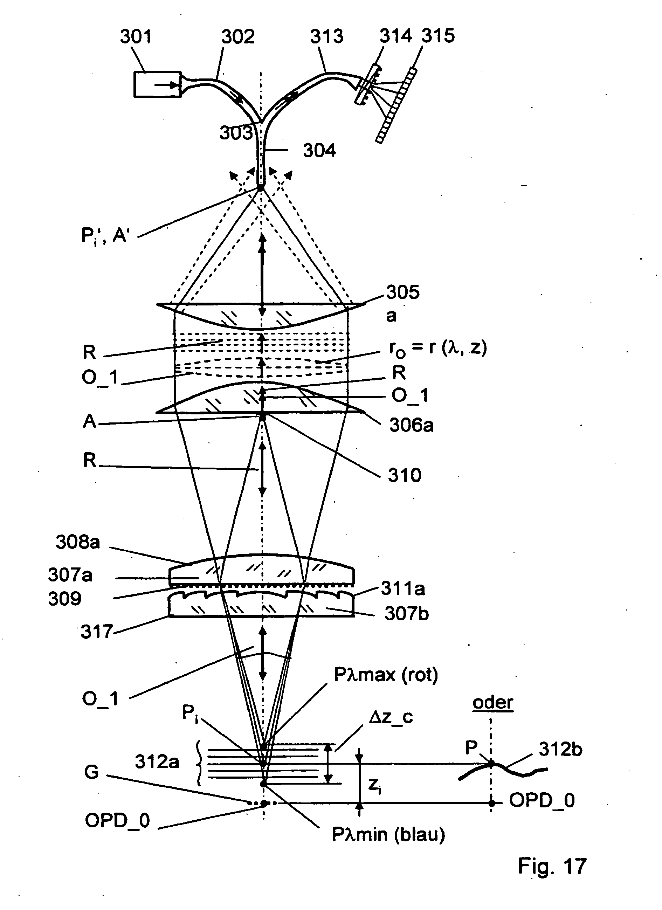

Die **Figur 17** stellt eine Anwendung für einen hochgenauen, mobilen Punktsensor dar. Dieser kann zur optischen Abtastung der Pits und Lands in verschieden tiefen Datenschichten eines mehrschichtigen Datenträgers, aber auch für die hochgenaue Abstandsmessung und Profilmessung auf einer Meßmaschine oder auch für die Prüfung eines Objekts in verschiedenen Tiefen, insbesondere in einer Reinraumumgebung, verwendet werden.

Die Lichtquelle ist als fasergekoppelte Superlumineszenz-Diode 301 ausgebildet. Das Licht derselben wird in eine Faser 302 eingekoppelt, passiert die Y-Weiche 303 und gelangt über das Faserstück 304 als divergierendes Strahlenbündel auf ein Kollimatorobjektiv 305a mit dem Brennpunkt F_5a, das zur Bündel-Kollimierung dient. Es entstehen mittels diesem zumindest näherungsweise plane Wellen, die auf ein Mikroskopobjektiv 306a, das hier das Abbildungssystem eines Mirau-Interferometers darstellt, gelangen, und somit in den Objektraum fokussiert werden. Die numerische Apertur (NA) des Mikroskopobjektivs 306a beträgt hierbei NA=0,55. Zwischen dem Mikroskopobjektiv 306a und dem Objektraum befindet sich die Strahlteilerplatte 307a mit einer konvexen Frontfläche 308a auf der äußeren Plattenseite und der Strahlteilerschicht 309, die auf der inneren Plattenseite aufgebracht ist, Auf der dem Objekt zugewandten Seite der Strahlteilerplatte 307a befindet sich eine diffraktive, sammelnde Zonenlinse 311a. Die Profiltiefe auf der diffraktiven, sammelnden Zonenlinse 311a liegt dabei im Sublambda-Bereich. Es entsteht durch Reflexion an der Strahlteilerschicht 309 ein konvergierendes Referenzbündel R, das am planen Referenzspiegel 310 reflektiert wird. Das an Strahlteilerplatte 307a mit Strahlteilerschicht 309 hindurchtretende Bündel passiert nun in der ersten Ordnung die diffraktive, sammelnde Zonenlinse 311a, wobei eine sammelnde Wirkung auftritt. Die Wirkung der Zonenlinse 311a im Objektstrahlengang wird durch die Spiegellinse, die durch die konvexe Frontfläche 308a und die Strahlteilerschicht 309 gebildet ist, teilweise kompensiert. Die Punkte A und OPD_0 stellen Orte gleichen optischen Gangunterschiedes im interferometer dar. Durch die wellenlängenabhängige Brechkraft der Zonenlinse 311a kommt es im Objektraum für das Objektbündel O_1 zu einer chromatischen Längsaufspaltung im Bereich Dz_c, wobei die Foki der kurzwelligen Strahlung am Weitesten von der Strahlteilerplatte , 307a entfernt sind ($P_{lmin}$). So erfolgt eine Tiefenabtastung des Objektraumes. Die an einem Punkt P eines mehrschichtigen Objekts 312a an einer Schicht oder bei einer anderen Applikation an einem Objekt 312b an einem Oberflächenpunkt $P_i$ reflektierte Strahlung gelangt über die diffraktive, sammelnde Zonenlinse 311a durch die Strahlteilerplatte 307a mit Strahlteilerschicht 309 und konvexer Fläche 308a gemeinsam mit dem Referenzbündel R in das Mikroskopobjektiv 306a. Dabei erfolgt hier die Transmission der Strahlung an der Zonenlinse 311a wieder in der ersten Beugungsordnung. Die Brechkraft der Spiegellinse mit der konvexen Frontfläche 308a und der Strahlteilerschicht 309 führt dazu, daß der Fokus auf dem planen Referenzspiegel 310 im Punkt A liegt und der optische Gangunterschied zwischen dem Referenz- und dem Objektstrahlenbündel im Punkt $P_l$ beispielsweise nur 35 $\mu$m beträgt.

Nach dem Passieren des Mikroskopobjektivs 306a und des Kollimatorobjektivs 305a entstehen - je nach Wellenlänge - Objektbündel unterschiedlicher Wellenfrontkrümmung. Die zumindest näherungsweise im Punkt $P_i$ fokussierten Objektstrahlenlbündel und das Referenzstrahlenbündel, welches im Punkt A fokussiert wird, treten in das Endstück der Faser 304 wieder ein, wobei $P_i'$ und A' optisch konjugierte Punkte darstellen. Die in das Endstück der Faser 304 eingekoppelten Bündel passieren die Weiche 303 in Richtung der Auskoppelfaser 313, gelangen auf ein fasergekoppeltes Spektrometer 314 und auf eine Zeilenkamera 315 und werden dort detektiert. Auf der Zeilenkamera 315 entsteht das Spektrum der Intensität über der Wellenzahl, die Müllerschen Streifen. Die Auswertung kann mittels der Auswertung der Phasenvariation über der Wellenzahl erfolgen, da die Intensität über der Wellenzahl vom optischen Gangunterschied im Interferometer und damit auch vom optischen Abstand von der Strahlteilerschicht 309 abhängig ist. Die Auswertung der Müllerschen Streifen wird in dieser Schrift nicht dargestellt.

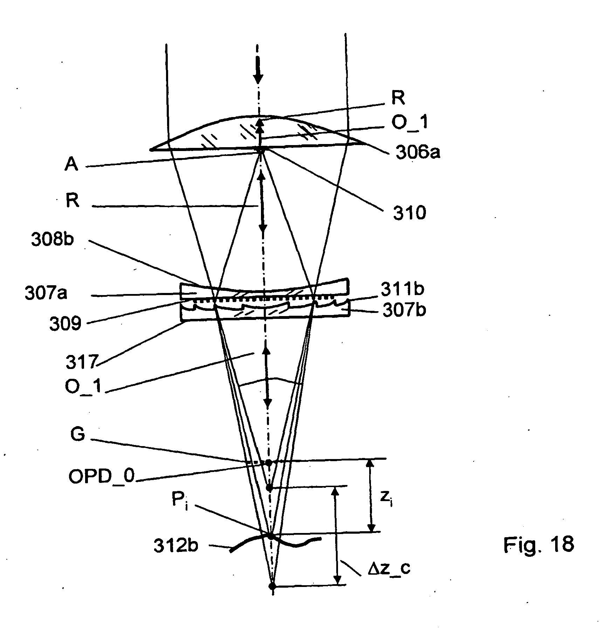

Die **Figur 18** stellt eine Anordnung mit einer Strahlteilerplatte 307a mit einer konkaven Fläche 308b und einer strahlendivergierenden Zonenlinse 311 b dar. Dadurch kann ein größerer Arbeitsabstand zu einem Meßobjekt 312a oder 312b erreicht werden.

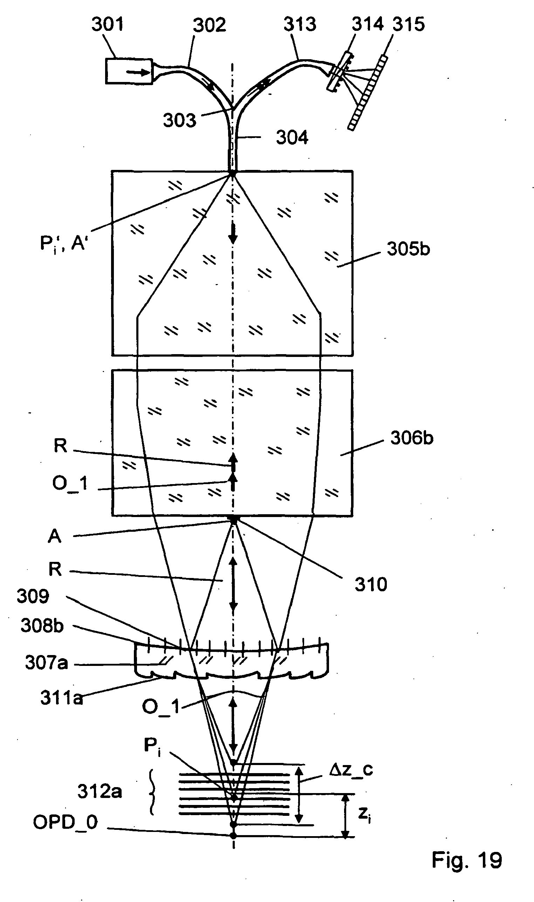

Die **Figur 19** stellt eine Anordnung für einen hochgenauen, mobilen Punktsensor mit einem Minimum an Komponenten dar. Dieser Punktsensor kann zur optischen Abtastung der Pits und Lands in verschieden tiefen Datenschichten eines mehrschichtigen Datenträgers 312a, aber auch für die Abstandsmessung und Profilmessung eines Objekts 312b auf einer Meßmaschine oder auch für die Prüfung eines Objekts 312a in verschiedenen Tiefen, insbesondere in einer Reinraumumgebung, verwendet werden.

Die Lichtquelle ist als fasergekoppelte Superlumineszenz-Diode 301 ausgebildet. Das Licht derselben 301 wird in eine Faser 302 eingekoppelt, passiert die Y-Weiche 303 und gelangt über das Faserstück 304 als divergierendes Strahlenbündel auf eine Grinlinse 305b mit dem Fokuspunkt $F\_5b$. die zur Kollimierung dient. Es entstehen zumindest näherungsweise plane Wellen, die auf eine Grinlinse 306b, die hier das Abbildungssystem eines Mirau-Interferometers darstellt, gelangen und somit in den Objektraum fokussiert werden. Zwischen der Grinlinse 306b und dem Objektraum befindet sich die Strahlteilerplatte 307a mit einer konkaven Frontfläche 308b und der Strahlteilerschicht 309, die auf die konkave Frontfläche 308b aufgebracht ist. Auf der dem Objekt zugewandten Seite der Strahlteilerplatte 307a befindet sich eine diffraktive, strahlungssammelnde Zonenlinse 311a. Es entsteht durch Reflexion an der Strahlteilerschicht 309 ein konvergierendes Referenzbündel R, das am planen Referenzspiegel 310 reflektiert wird. Das an Strahlteilerplatte 307a mit der Strahlteilerschicht 309 hindurchtretende Strahlenbündel passiert nun in der ersten Beugungsordnung die diffraktive, strahlungssammelnde Zonenlinse 311a. Die strahlungssammelnde Wirkung der Zonenlinse 311a wird durch den Spiegel, der durch die konkave Frontfläche 308b und die Strahlteilerschicht 309 gebildet ist, teilweise kompensiert. Die Punkte A und OPD_0 stellen Orte gleichen optischen Gangunterschiedes dar. Durch die wellenlängenabhängige Brechkraft der Zonenlinse 311a kommt es im Objektraum für das Objektbündel O_1 zu einer chromatischen Längsaufspaltung im Bereich $\Delta z\_c$, wobei die Foki der kurzwelligen Strahlung am Weitesten von der Strahlteilerplatte 307a entfernt sind. So erfolgt eine Tiefenabtastung des Objektraumes. Das von einem Punkt $P_i$ des mehrschichtigen Objekts 312a reflektierte Licht gelangt über die diffraktive, lichtsammelnde Zonenlinse 311a, wobei hier die Transmission des Lichtes wieder in der ersten Beugungsordnung erfolgt, durch die Strahlteilerplatte 307a mit Strahlteilerschicht 309 gemeinsam mit dem Referenzbündel R in die Grinlinse 306b. Nach dem Passieren der Grinlinsen 306b und 305b entstehen - je nach Wellenlänge - Objektbündel unterschiedlicher Wellenfrontkrümmung. Die zumindest näherungsweise im Punkt $P_i$ fokussierten Tellbündel treten in das Endstück der Faser 304 wieder ein, passieren die Weiche 303 in Richtung der Auskoppelfaser 313, gelangen auf ein fasergekoppeltes Spektrometer 314 und auf eine Zeilenkamera 315. Dort werden die Müllerschen Streifen ausgebildet und detektiert.

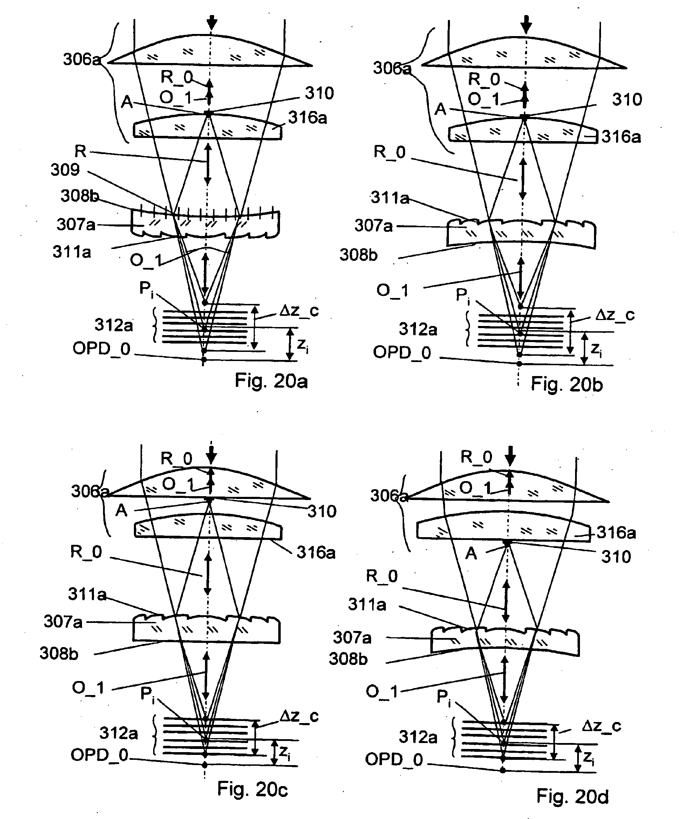

Alle **Figuren 20a** bis **20g** sind insbesondere als Punktsensoren ausgelegt, wobei jeweils auch eine konfokale Diskriminierung erfolgt. So kann Strahlung von unerwünschten Beugungsordnungen am diffraktiv-optischen Zonenelement 311a eliminiert werden, besonders wenn das diffraktiv-optische Zonenelement 311a sowohl in nullter als auch in erster Ordnung benutzt wird. Die Beleuchtung kann, wie in den Figuren 1 bis 3 dargestellt, mit den Komponenten 301 bis 305a oder 305b erreicht werden. Mit den Komponenten 304, e bis 315 kann die Detektion der Müllerschen Streifen ermöglicht werden.

Die **Figur 20a** zeigt eine Anordnung, bei der mittels Strahlteilerschicht 309 das Referenzbündel R entsteht. Die Linse 316a fokussiert das Referenzbündel R nur sehr schwach. Weiterhin ist die in der Regel geringe chromatische Längsaberration der Linse 316a im Abbildungssystem 306a bereits einkorrigiert. Der Referenzspiegel 310 ist auf der Linse 316a aufgebracht. Für das Referenzbündel wird somit ein Teil der Brechkraft des Abbildungssystems genutzt, welche die Hauptbrechkraft in der Anordnung aufbringt. Damit stellt die Brechkraft der Linse 316a die Nebenbrechkraft der Anordnung dar. Die Chromasie der Linse 316a, speziell die chromatische Längsaberration, ist dabei also durch die entgegengesetzte Chromasie des übrigen Abbildungssystems 306a kompensiert, so daß der Referenzstrahlengang hinsichtlich der Tiefenaufspaltung achromatisch ist, wodurch der Punkt A achromatisch abgebildet wird und Strahlung aller Wellenlängen der Referenzstrahtung den konfokalen Diskriminator, beispielsweise die Faser 304, passieren und zur Ausbildung der Müllerschen Streifen beitragen kann.

Die **Figur 20b** stellt eine Anordnung dar, bei der das Referenzbündel R_0 durch Beugung in der nullten Ordnung in Reflexion am diffraktiv-optischen Zonenelement 311a erzeugt wird. Die Chromasie der Linse 316a, speziell deren chromatische Längsaberration ist dabei, wie in Figur 20a beschrieben, durch die entgegen gesetzte Chromasie des übrigen Abbildungssystems 306a kompensiert.

Die **Figur 20c** stellt eine Anordnung dar, bei der das Referenzbündel R_0 ebenfalls durch Beugung in der nullten Ordnung in Reflexion am diffraktiv-optischen Zonenelement 311a erzeugt wird. Der Referenzspiegel 310 ist dabei noch tiefer im Abbildungssystem, also noch weiter von der Strahlteilung entfernt, angeordnet. Hierbei kann auch ein 90°-Prisma in den Strahlengang integriert werden, um die Innenwand einer Bohrung anzumessen.

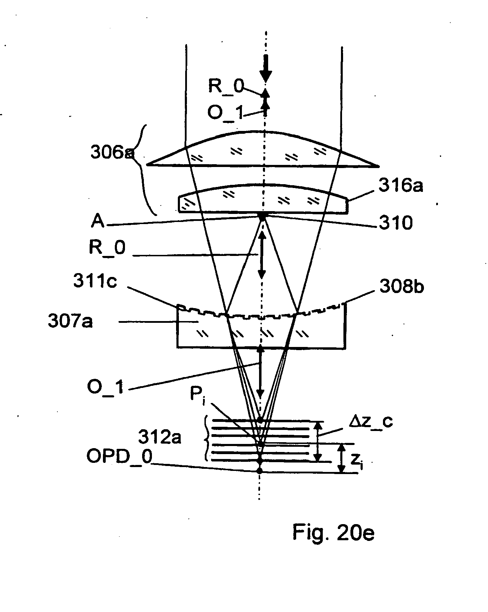

Die **Figur 20d** stellt eine Anordnung dar, bei welcher der Referenzspiegel 310 auf der Frontfläche der Linse 316a angeordnet ist. Das Referenzbündel R_0 wird ebenfalls durch Beugung in der nullten Ordnung in Reflexion am diffraktiv-optischen Zonenelement 311a erzeugt. Die teilweise Kompensation der Brechkraft des diffraktiv-optischen Zonenelements 311a erfolgt mittels der konkav gekrümmten Fläche 308b der Strahlteilerplatte 307a, wobei die Fläche 308b also refraktiv genutzt wird. In der **Figur 20e** ist eine Anordnung dargestellt, bei der die Fläche der Strahlteilerplatte 307a , die zum Abbildungssystem weist, schwach konkav gekrümmt ist. Auf dieser befindet sich das diffraktiv-optische Zonenelement 311a, welches in der ersten Beugungsordnung das Objektstrahlenbündel fokussierend beeinflußt. Das Referenzbündel R_0 wird in der nullten Ordnung in Reflexion am diffraktiv-optischen Zonenelement 311c erzeugt und erfährt durch die konkav gekrümmte Fläche 308b der Strahlteilerplatte 307a ebenfalls eine Fokussierung, so daß eine teilweise Kompensation der chromatischen Brechkraft des diffraktiv-optischen Zonenelements 311 c gegeben ist und sich somit der Abstand $Z_i$ verkleinert. Dadurch verkleinert sich im Meßbereich der Anordnung die Ortsfrequenz der detektierten Müllerschen Streifen.

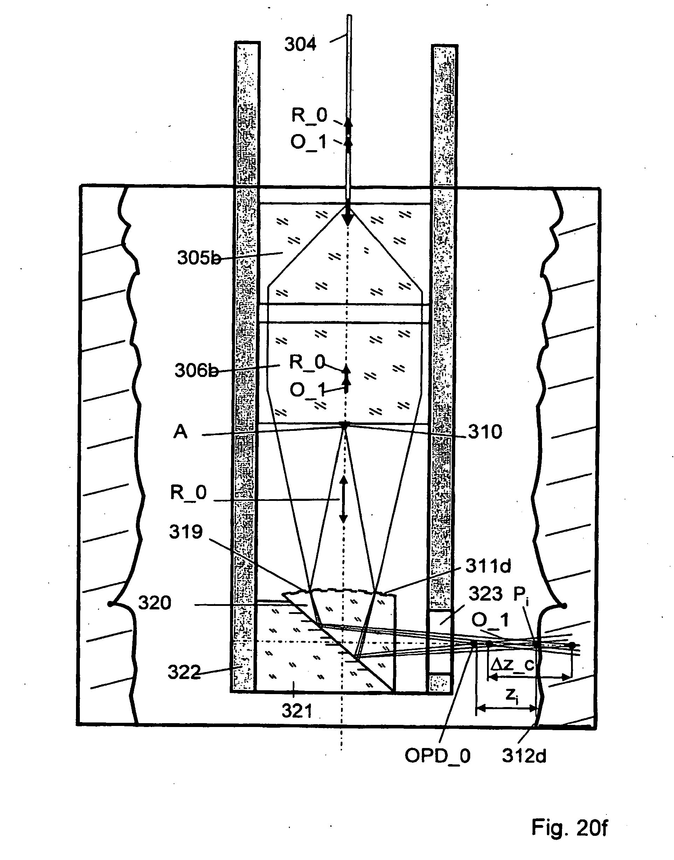

In der **Figur 20f** ist eine Anordnung für die Vermessung der Oberflächen 312d von Innenräumen dargestellt, bei der die Frontfläche 319 eines 90°-Umlenkprismas 320, die zum Abbildungssystem weist, schwach konvex gekrümmt ausgebildet ist. Auf dieser gekrümmten Frontfläche 319 befindet sich das diffraktiv-optische Zonenelement 311d, welches in der ersten Beugungsordnung das Objektstrahlenbündel in Transmission fokussierend beeinflußt. Das Referenzbündel R_0 wird dagegen in der nullten Ordnung und in Reflexion am diffraktiv-optischen Zonenelement 311a erzeugt und erfährt durch die konvex gekrümmte Frontfläche 319 des Umlenkprismas 320 eine Verringerung der Fokussierung, so daß der Weg zum Fokuspunkt A geometrisch verlängert wird. So ist eine zumindest teilweise Kompensation der Vergrößerung des optischen Gangunterschieds durch die optische Dicke des Umlenkprismas 320 im Objektstrahlengang gegeben und der Referenzstrahlengang ist achromatisch. Dadurch ergibt sich bei entsprechendem Design der beteiligten Komponenten 319, 320 311d im Meßbereich der Anordnung eine hinreichend kleine Ortsfrequenz der detektierten Müllerschen Streifen, welche optimal auswertbar ist. Das 90°-Umlenkprisma 320 wird mittels außen verrundetem Glasmontageprisma 321 in einer Zylinderfassung 322 mit Durchbruch 323 gehalten. Die Grinlinsen 305b und 306b sind mit kreisförmigem Querschnitt ausgebildet. Die Grinlinsen 305b ist fasergekoppelt.

Ein anderes Ausführungsbeispiel für eine Anordnung für die Vermessung der Oberflächen 312d von Innenräumen basiert auf der Anordnung nach Figur 20f.

Jedoch ist das Prisma 320 mit Planflächen ausgeführt. Diesem Prisma 320 sind dabei die Komponenten 307a, 307b, 308b und 311a aus Figur 18 vorgeordnet sowie eine nicht dargestellte planparallele Kompensationsplatte mit der gleichen Glasweglänge wie das Prisma zum Ausgleich des Öffnungsfehlers.

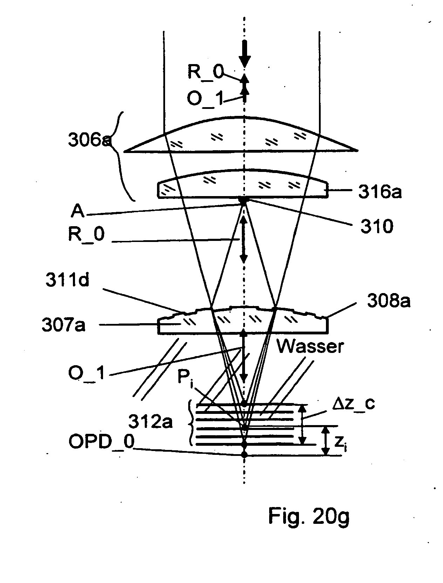

In der **Figur 20g** ist eine Anordnung dargestellt, bei der die Fläche 308a der Strahlteilerplatte 307a schwach konvex gekrümmt ist. Auf dieser befindet sich das diffraktiv-optische Zonenelement 311d, welches in der ersten Beugungsordnung das Objektstrahlenbündel fokussierend beeinflußt. Das Referenzbündel R_0 wird in der nullten Ordnung in Reflexion am diffraktiv-optischen Zonenelement 311d erzeugt und erfährt durch die konvex gekrümmte Frontfläche 319 des Umlenkprismas 320 eine Verringerung der Fokussierung des Referenzbündels R_0, so daß der Weg zum Fokuspunkt A geometrisch verlängert wird und der Referenzstrahlengang achromatisch ist. So ist eine zumindest teilweise Kompensation der Vergrößerung des optischen Gangunterschieds durch die optische Dicke eines Wasserfilms im Objekstrählengang vor dem Objekt 312d bei entsprechendem Design der beteiligten Komponenten 308a, 307a und 311d möglich. So kann eine hinreichend kleine Ortsfrequenz der detektierten Müllerschen Streifen erreicht werden, welche optimal auswertbar ist.

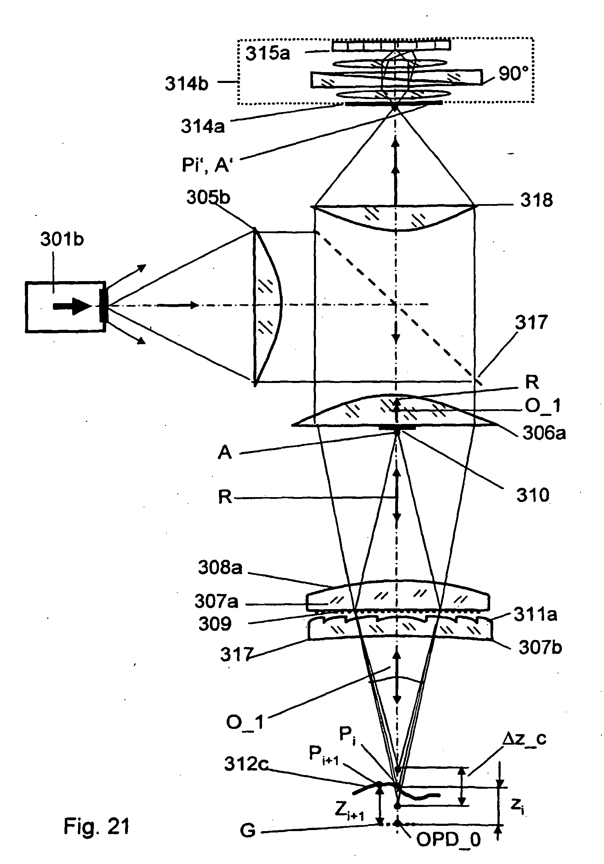

Die **Figur 21** stellt eine Anwendung für einen hochgenauen Liniensensor dar. Die Quelle elektromagnetischer Strahlung 1b ist als Weißlichtlaser, also spektral breitbandig ausgebildet. Die Strahlung derselben passiert einen Strahlteiler 317 und ein Kollimatorobjektiv 305a. Es entstehen zumindest näherungsweise plane Wellen, die auf ein Objektiv 306a eines Mirau-Interferometers gelangen und mittels diesem Objektiv 306a in den Objektraum fokussiert werden. Zwischen dem Objektiv 306a und dem Objektraum befindet sich die Strahlteilerplatte 307a mit einer konvexen Frontfläche 308a. Auf der Platte 307b sind die Strahlteilerschicht 309 und die diffraktive, strahlungssammelnde Zonenlinse 311a aufgebracht. Es entsteht durch Reflexion an der Strahlteilerschicht 309 ein konvergierendes Referenzbündel R, das am Referenzspiegel 310 reflektiert wird. Das an Strahlteilerschicht 309 hindurchtretende Strahlenbündel passiert nun in der ersten Ordnung die diffraktive lichtzerstreuende Zonenlinse 311a, wobei das Strahlenbündel O_1 entsteht und an der Zonenlinse 311a eine strahlungssammelnde Wirkung auftritt. Durch die wellenlängenabhängige Brechkraft der diffraktiven lichtzerstreuenden Zonenlinse 311a führt dies im Objektraum für das Objektbündel O_1 zu einer chromatischen Längsaufspaltung, wobei die Foki der kurzwelligen Strahlung am Weitesten von der Strahlteilerbaugruppe entfernt sind. So erfolgt eine Tiefenabtastung des Objektraumes. Das von einem Punkt $P_i$ des Objekts 312c reflektierte Licht gelangt über die Zonenlinse 311 a, wobei hier die Transmission des Lichtes wieder in der ersten Beugungsordnung erfolgt, durch die Strahlteilerschicht 309 gemeinsam mit dem Referenzbündel R in das Objektiv 306a des Mirau-Interferometers. Die Brechkraft der konkaven Frontfläche 309 führt zu einer Anpassung des optischen Gangunterschiedes zwischen dem Referenz- und dem Objektstrahlenbündel. Das Referenzstrahlenbündel R und das Objektstrahlenbündel O_1 passieren den Strahlteiler in Transmission und bilden über ein Tubusobjektiv 318 das Objekt 312c auf eine Flächenkamera 315a ab, welche Bestandteil eines Linienspektrometers 314b mit einem Eingangsspalt 314a ist. Mit dieser Anordnung ist eine Korrektur des Feldes möglich. Dabei stellt die Variation der Phase oder Frequenz im Feld kein Problem dar, da eine numerische Korrektur derselben problemlos ist.

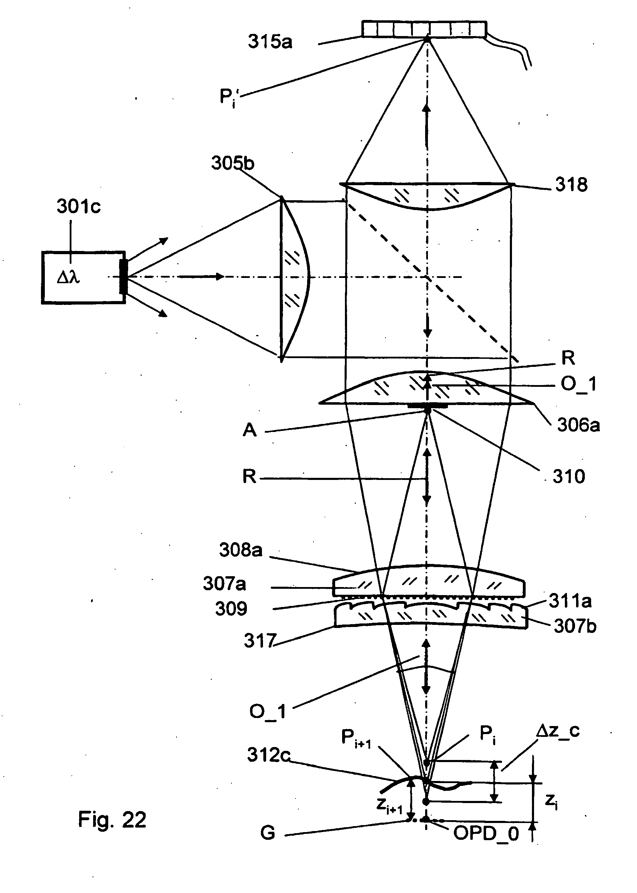

**Figur 22** zeigt eine Anordnung für einen Profilschnitt oder für flächige Objektfelder, die in den wesentliche Komponenten Figur 21 entspricht, jedoch kein Spektrometer aufweist. Mit dieser Anordnung wird eine flächenhafte Messung des Objekts 312c ermöglicht. Diese Anordnung kann auch sehr gut mit einem Immersions-Abbildungssystem für Wasser genutzt werden. Dabei wird eine spektral feinsteinstellbare, durchstimmbare, schmalbandige Laser-Quelle 1c eingesetzt, so daß mittels Wellenlängen-Durchstimmung der Laser-Quelle 1 c die Profiltiefe oder auch hochaufgelöst die 3D-Mikroform des Objekts 312c mittels Flächenkamera 315a erfaßt werden kann.

Eine bevorzugte Ausführungsform der Erfindung betrifft ein interferometrisches Multispektral-Verfahren zur hochdynamischen Objekt-Tiefenabtastung oder -Profilerfassung mit einem Zweistrahl-Interferometer, welches mit einer punktförmigen Multiwellenlängen-Quelle elektromagnetischer Strahlung oder einer Vielzahl von punktförmigen Multiwellenlängen-Quellen am Eingang des Zweistrahl-Interferometers zur Objektbeleuchtung und mit mikroskopischer Abbildung des Objekts auf einen Empfänger elektromagnetischer Strahlung durchgeführt wird, wobei im Zweistrahl-Interferometer mittels bündelbegrenzender Mittel eine konfokale Diskriminierung erzeugt wird, wobei sich die bündelbegrenzenden Mittel zumindest näherungsweise in einer optisch konjugierten Ebene der mindestens einen der punktförmigen Multiwellenlängen-Quelle elektromagnetischer Strahlung am Eingang des Zweistrahl-Interferometers befinden. Dabei kann eine punktförmige Multiwellenlängen-Quelle als eine fasergekoppelte Superlumineszenz-Diode ausgebildet sein, wobei auch mehrere fasergekoppelte Superlumineszenz-Dioden zur Anwendung kommen können. Die konfokale Diskriminierung kann jedoch auch durch nahezu punktförmige Empfänger elektromagnetischer Strahlung erfolgen.

Im Zweistrahl-Interferometer werden ein Referenzstrahlenbündel und ein Objektstrahlenbündel elektromagnetischer Strahlung erzeugt. Dabei ist im Zweistrahl-Interferometer ein diffraktiv-optisches Zonenelement (DOZE) angeordnet, mittels dessen erfindungsgemäß sowohl elektromagnetische Strahlung in der nullten als auch in der ersten Beugungsordnung oder gegebenenfalls einer höheren Beugungsordnung N oder einer tieferen Beugungsordnung als der nullten in Transmission oder in Reflexion erzeugt wird. So passiert das Referenzstrahlenbündel des Zweistrahl-Interferometers im Hin- und Rücklauf das diffraktiv-optische Zonenelement stets in der nullten Beugungsordnung und dagegen passiert das Objektstrahlenbündel im Hin- und Rücklauf in Bezug zum Objekt das diffraktiv-optische Zonenelement jeweils in der ersten Beugungsordnung oder gegebenenfalls auch jeweils einer höheren Beugungsordnung N, wodurch im Objektraum durch Beugung der elektromagnetischen Strahlung eine chromatische - also eine über der Wellenlänge entstehende - Längsaufspaltung des Objektbündels in Foki erfolgt. Die elektromagnetische Strahlung zumindest näherungsweise aller Wellenlängen vom Referenzstrahlenbündel R_0_0, das keine Beugung am diffraktiv-optischen Zonenelement erfährt, durchsetzt nach dem Passieren des diffraktiv-optischen Zonenelements die bündelbegrenzenden Mittel zur konfokalen Diskriminierung, während nach dem diffraktiv-optischen Zonenelement für das vom Objekt kommende Objektstrahlenbündel O_1_1 oder O_N_N nur die Anteile die begrenzenden Mittel zur konfokalen Diskriminierung am Interferometerausgang oder im Zweistrahl-Interferometer passieren, die zumindest näherungsweise scharf auf das Objekt abgebildet waren. Zwischen den konfokal diskriminierten Strahlungsanteilen des Referenz- und des Objektstrahlenbündels findet zumindest näherungsweise Zweistrahl-Interferenz statt. Die interferierende elektromagnetische Strahlung der beiden Strahlenbündel wird anschließend zur Analyse in ein Spektrometer oder in ein weiteres Interferometer geführt und auf dem Empfänger elektromagnetischer Strahlung abgebildet, wobei die Empfängerebene sich zumindest näherungsweise in einer optisch konjugierten Ebene zu mindestens einer der punktförmigen Multiwellenlängen-Quellen am Eingang des ZweistrahlInterferometers befindet. Aus den mittels Spektrometer spektroskopisch analysiert oder einem weiteren Interferometer interferometrisch analysiert erzeugten optischen Signalen wird der Abstand oder das Profil des Objekts mittels bekannter Algorithmen zur Wavelet- oder Interferogramm-Analyse bestimmt.

Es wird also ein multispektrales interferometrisches Verfahren mit Zweistrahl-Interferometrie zur hochdynamischen Objekttiefenabtastung und -profilmessung mit einem diffraktiv-optischen Zonenelement zur Beeinflussung des Objektstrahlenbündels in einer gebeugten Ordnung beschrieben, wobei das Referenzstrahlenbündel jeweils in der nullten Ordnung das diffraktiv-optische Zonenelement passiert. Dieses Verfahren nutzt als objektabbildendes Zweistrahl-Interferometer vorzugsweise ein objektabbildendes Fizeau-Interferometer, welches eine Referenzspiegelfläche enthält. Diese Referenzspiegelfläche ist als Teilspiegel ausgebildet, der also auch einen Teil des auftreffenden Lichtes hindurchlässt und dessen Reflexion und Transmission so ausgebildet sind, dass bevorzugt Zweistrahlinterferenz entstehen kann. Das zu prüfende Objekt ist dabei ebenfalls optischer Bestandteil des Interferometers. Dabei ist dem Fizeau-Interferometer entweder eine spektral breitbandige, welche eine Halbwertsbreite von mindestens einigen Nanometern aufweist, oder eine Wellenlängen-durchstimmbare Quelle elektromagnetischer Strahlung vorgeordnet, welche im Weiteren als Lichtquelle bezeichnet wird und die ein Eingangsstrahlenbündel für das Fizeau-Interferometer erzeugt. Es kann also, eine spektral breitbandige Quelle elektromagnetischer Strahlung mit einer Halbwertsbreite von einigen Nanometern bis zu mehreren hundert Nanometern im DUV-, im UV-, im VIS-, im NIR- oder im MIR-oder auch im FIR-Bereich dem Sensor zugeordnet sein. Im infraroten Spektralbereich kann die Halbwertsbreite auch einige Mikrometer betragen. Es kann also auch eine Weißlichtquelle im sichtbaren Spektralbereich mit einem Anteil im NIR- oder UV-Bereich eingesetzt werden. Möglich ist aber auch der Einsatz eines Wellenlängen-durchstimmbaren Lasers, beispielsweise ein Ti:Sapphir Laser oder ein Multiwellenlängen-Laser mit Frequenzkamm-Charakteristik oder ein Kontinuums-Weißlichtlaser. Es kann auch eine Laserdiode eingesetzt werden. Beim Einsatz einer monochromatischen Lichtquelle ist diese Lichtquelle jeweils durchstimmbar ausgeführt.

Es wird beim Sensor davon ausgegangen, dass dem zu prüfenden Objekt, in allen hier betrachteten Fällen, eine Linse, beispielsweise auch eine Grinlinse, oder ein Prüfobjektiv direkt oder wenigstens indirekt zugeordnet ist. Diese Linse oder das Prüfobjektiv dient sowohl stets der Beleuchtung der Objektoberfläche als auch der Abbildung der Objektoberfläche, mindestens jedoch der Abbildung eines Punktes derselben.

Das Fizeau-Interferometer ist also mit einem Abbildungsstrahlengang für das zu prüfende Objekt und einem Punktempfänger, einem gerasterten Zeilenempfänger, also beispielsweise eine Zeilenkamera, oder einem gerasterten Flächenempfänger, also eine Flächenkamera, für elektromagnetische Strahlung ausgebildet. Es entsteht im Hinlauf des Lichtes von der Lichtquelle zum Objekt aus dem Eingangsstrahlenbündel mittels Diffraktion in Reflexion oder Transmission an einem statischen oder dynamischen, diffraktiv-optischen Zonenelement (DOZE) ein gebeugtes Strahlenbündel $O\_N$ (N=1, 2, 3... oder -1, -2, -3...) in der ersten oder in einer höheren Beugungsordnung N (N=1, 2, 3) oder in einer niedrigeren Beugungsordnung N als der nullten (N=- 1, -2, -3) Beugungsordnung. Das gebeugte Strahlenbündel $O\_N$ ist nach der Fokussierung auf das Objekt im Objektraum chromatisch längs aufgespalten, so dass in Abhängigkeit von der Wellenlänge des Lichts eine Reihe oder eine Vielzahl von Reihen von Fokuspunkten in der Tiefe ausgebildet wird. Nach Reflexion des Teilstrahlenbündels $O\_N$ (N=1, 2, 3... oder -1, - 2, -3...) am Objekt wird ein Objekt-Teilstrahlenbündel vom rückreflektierten Strahlenbündel durch Beugung am diffraktiv-optischen Zonenelement in derselben Ordnung wie im Hinlauf, nämlich $O\_N\_N$ (N=1, 2, 3... oder -1, -2, -3...) gebildet, so dass das Objektteilstrahlenbündel $O\_N\_N$ (N=1,2, 3... oder-1, -2, -3...) nach dem Passieren des diffraktiv-optischen Zonenelements wieder im Strahlengang zurückläuft. Aus dem Eingangsstrahlenbündel wird im Hinlauf auf die Referenzspiegelfläche auch noch ein Teilstrahlenbündel, welches im Weiteren als Referenzstrahlenbündel $R\_0$ dient, durch Beugung in der nullten Ordnung am diffraktiv-optischen Zonenelement gebildet. Dabei durchsetzt das Teilstrahlenbündel $O\_N$ (N=1, 2, 3... oder -1, -2, -3...) auf dem Weg zum Objekt und auch zurück die Referenzspiegelfläche, wobei eine Abschwächung des Teilstrahlenbündels in Kauf genommen werden muss.

Im Rücklauf von der Referenzspiegelfläche, auf welche das Referenzstrahlenbündel $R\_0$ fokussiert wird, wird durch Beugung und auch genau wieder in der nullten Ordnung am diffraktiv-optischen Zonenelement ein Strahlenbündel $R\_0\_0$ gebildet, so dass das Referenzteilstrahlenbündel $R\_0\_0$ nach dem Passieren des diffraktiv-optischen Zonenelements wieder im Strahlengang zurückläuft. Das Licht im Referenzstrahlenbündel erfährt keine chromatische Längsaufspaltung.

Dabei bildet sich auf der Referenzspiegelfläche bei einer Punktlichtquelle wellenlängenunabhängig jeweils nur ein Fokusfleck aus, der jedoch das Licht aller Wellenlängen enthalten kann. Oder bei Anwendung einer Multipunktlichtquelle bildet sich eine Vielzahl von lateral verteilten Fokusflecken aus.

Außerdem ist zwischen dem Objekt-Teilstrahlenbündel $O\_N\_N$ (N=1, 2, 3... oder -1, -2, -3...) und dem Referenzstrahlenbündel $R\_0\_0$ der optische Gangunterschied ungleich null, wenn auf einen Objektpunkt fokussiert ist.

Des Weiteren verlaufen das Objekt-Teilstrahlenbündel $O\_N\_N$ (N=1, 2, 3... oder-1,-2, -3...) und das Referenzstrahlenbündel $R\_0\_0$ nach dem diffraktiv-optische Zonenelement parallel und kommen zur Interferenz, wobei stets ein optischer Gangunterschied ungleich null besteht. Anschließend werden die beiden miteinander interferierenden Teilstrahlenbündel in den Detektionsstrahlengang eingekoppelt.

Des Weiteren wird eine konfokale Diskriminierung des Lichts der interferierenden Teilstrahlenbündel durchgeführt.

Die konfokal diskriminierten interferierenden Bündel $O\_N\_N$ (N=1, 2, 3... oder -1, -2, -3...) und $R\_0\_0$ erfahren zur spektroskopischen Analyse eine Lateralaufspaltung mittels Dispersion oder Diffraktion. Es kann eine schnelle Fourier-Transformation (FFT) des sich ergebenden Intensitätsverlaufs durchgeführt werden, um aus dem sich mittels FFT ergebenden Wertes nach Kalibrierung die Tiefenposition eines Objektpunktes zu bestimmen.

Andererseits ist es auch möglich, dass die interferierenden Bündel $O\_N\_N$ (N=1, 2, 3... oder -1, -2, -3...) und $R\_0\_0$ eine weitere Interferenzbildung mittels scannenden Zweistrahl-Interferometer zur optischen Analyse erfahren, also mit zeitlichem Variieren des optischen Gangunterschieds, und der im scannenden Zweistrahl-Interferometer beim Scan durchfahrene optische Gangunterschied mindestens einmal genau auch dem im Interferometer zwischen Objekt-Teilstrahlenbündel $O\_N\_N$ (N=1, 2, 3... oder -1, -2, -3...) und Referenzstrahlenbündel $R\_0\_0$ bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird. Dabei kann die optische Analyse mittels scannenden Michelson-Interferometer für einen Objektpunkt oder auch gleichzeitig für mehrere Objektpunkte durchgeführt werden. Das scannende Zweistrahl-Interferometer arbeitet sozusagen "ersatzweise" für das objektabbildende Zweistrahl-Interferometer, da dieses aus Gründen der Miniaturisierung nicht über einen eigenen Scanner verfügen kann.

Des Weiteren ist es möglich, dass die interferierenden Bündel O_N_N (N=1, 2, 3...oder -1, -2, -3...) und R_0_0 eine weitere Interferenzbildung mittels statischem Zweistrahl-Interferometer zur optischen Analyse erfahren, also einem Zweistrahl-Interferometer mit lateraler Variation des optischen Gangunterschieds, wobei die Orte unterschiedlichen Gangunterschieds Pixeln einer Zeilen- oder Bildempfängers zugeordnet sind.

Dabei entspricht der im Zweistrahl-Interferometer auftretende optische Gangunterschied dem im Interferometer zwischen Objekt-Teilstrahlenbündel O_N_N (N=1, 2, 3... oder -1, -2, -3...) und Referenzstrahlenbündel R_0_0 bestehenden optischen Gangunterschied in mindestens einem Element des Zeilen- oder Flächenkameras zumindest näherungsweise. Dann wird das Maximum eines optischen Weißlicht-Interferogramms detektiert.

Andererseits wird im Fall der Anwendung einer Wellenlängen-durchstimmbaren Quelle elektromagnetischer Strahlung beim Scan der Wellenlänge ein optisches, moduliertes, periodisches Signal erzeugt und mittels Punktempfänger, gerastertem Zeilenempfänger oder gerastertem Flächenempfänger über der Zeit detektiert und anschließend dieses Signal über der Wellenlänge ausgewertet.

Vorzugsweise erfolgt beim multispektralen interferometrischen Verfahren zur hochdynamischen Objekttiefenabtastung und -Profilerfassung eine Beugung in Transmission in der ersten Ordnung an einer diffraktiv-optischen Phasengitter-Zonen-Linse, so dass zumindest näherungsweise nur die nullte und die erste Beugungsordnung entstehen und zwar als Referenz- und als Objektstrahlenbündel, die sich koaxial zueinander ausbreiten.

Vorzugsweise wirkt beim multispektralen interferometrischen Verfahren zur hochdynamischen Objekttiefenabtastung oder -Profilerfassung die diffraktiv-optische Phasengitter-Zonen-Linse als zerstreuende Linse.

Vorzugsweise erfolgt beim multispektralen interferometrischen Verfahren zur hochdynamischen Objekttiefenabtastung eine konfokale Diskriminierung der interferierenden Strahlenbündel O_N_N (N=1, 2, 3... oder -1, -2, -3...) und R oder R_0_0.

Dieses multispektrale interferometrische Verfahren zur hochdynamischen Objekttiefenabtastung und -Profilmessung kann aber auch vorzugsweise mit einem Mirau-Interferometer ausgeführt werden. Dabei ist im Mirau-Interferometer auch ein diffraktiv-optisches Zonenelement (DOZE) dem Interferometer Strahlteiler für das Eingangstrahlenbündel vorgeordnet, mittels dessen sowohl elektromagnetische Strahlung in der nullten als auch in der ersten Beugungsordnung oder gegebenenfalls einer höheren Beugungsordnung N in Transmission oder in Reflexion erzeugt wird. Das gebeugte Strahlenbündel O_N ist nach der Fokussierung auf das Objekt im Objektraum chromatisch längs aufgespalten, so dass in Abhängigkeit von der Wellenlänge des Lichts eine Reihe oder eine Vielzahl von Reihen von Fokuspunkten in der Tiefe ausgebildet wird. Nach Reflexion des Teilstrahlenbündels O_N (N=1, 2, 3... oder -1, -2, -3...) am Objekt wird ein Objekt-Teilstrahlenbündel vom rückreflektierten Strahlenbündel durch Beugung am diffraktiv-optischen Zonenelement in derselben Ordnung wie im Hinlauf, nämlich O_N_N (N=1, 2, 3... oder -1, -2, -3...) gebildet, so dass das Objektteilstrahlenbündel O_N_N (N=1, 2, 3... oder -1, -2, -3...) nach dem Passieren des diffraktiv-optischen Zonenelements wieder im Strahlengang zurückläuft. Das Referenzstrahlenbündel des Mirau-Interferometers passiert im Hin- und Rücklauf das diffraktiv-optische Zonenelement stets in der nullten Beugungsordnung und dagegen passiert das Objektstrahlenbündel im Hin- und Rücklauf in Bezug zum Objekt das diffraktiv-optische Zonenelement jeweils vorzugsweise in der ersten Beugungsordnung oder gegebenenfalls vorzugsweise auch jeweils einer höheren Beugungsordnung N, wodurch im Objektraum durch Beugung der elektromagnetischen Strahlung eine chromatische - also eine über der Wellenlänge entstehende - Längsaufspaltung des Objektbündels in Foki erfolgt. Des Weiteren verlaufen das Objekt-Teilstrahlenbündel O_N_N (N=1, 2, 3... oder -1, -2, -3...) und das Referenzstrahlenbündel R_0_0 nach dem zweiten Passieren des diffraktiv-optischen Zonenelements parallel und kommen zur Interferenz, wobei durch die Objektposition ein optischer Gangunterschied ungleich null besteht. Anschließend werden die beiden miteinander interferierenden Teilstrahlenbündel in den Detektionsstrahlengang eingekoppelt. Dabei erfolgt die der Signale in der bereits beschriebenen Art und Weise.

Dieses multispektrale interferometrische Verfahren zur hochdynamischen Objekttiefenabtastung und Objekt-Profilmessung kann aber auch vorzugsweise mit einem Michelson-Interferometer ausgeführt werden. Das diffraktiv-optische Phasengitter-Zonenelement ist in diesem Fall dem Interferometer-Strahlteiler für das Eingangstrahlenbündel vorgeordnet. Das Referenzstrahlenbündel R_0 wird auf die Referenzspiegelfläche fokussiert und das Objektstrahlenbündel O_N, wobei hier vorzugsweise N=1 realisiert ist, gelangt in den Objektraum und wird chromatisch längs aufgespalten. Dabei erfolgt die Entstehung der Interferenz und Auswertung der Signale in der bereits für das Zweistrahlinterferometer beschriebenen Art und Weise.

Gemäß einer bevorzugten Ausführungsform der Erfindung wird ein multispektrales interferometrisches Verfahren zur hochdynamischen Objekttiefenabtastung oder - Profilerfassung bereitgestellt. Dieses Verfahren nutzt ein objektabbildendes Mirau-Interferometer mit einem internen Strahlteiler und mit einer Referenzspiegelfläche. Dem Mirau-Interferometer ist das zu prüfende Objekt zugeordnet und das Objekt ist somit auch optisch aktiver Bestandteil des Mirau-Interferometers.

Dabei ist dem Mirau-Interferometer eine spektral breitbandige oder eine Wellenlängen-durchstimmbare Quelle elektromagnetischer Strahlung im DUV-, im UV-, im VIS-, im NIR- oder im MIR- oder auch im FIR-Bereich zugeordnet, die im Weiteren als Lichtquelle bezeichnet wird und welche ein Eingangsstrahlenbündel für das Mirau-Interferometer erzeugt. Das Mirau-Interferometer ist mit einem Abbildungsstrahlengang für das zu prüfende Objekt und mindestens einem einzigen Punktempfänger, oder einem gerasterten Zeilenempfänger oder einem gerasterten Flächenempfänger für elektromagnetische Strahlung ausgebildet.

Es entsteht bei diesem Verfahren im Hinlauf des Lichtes auf dem Weg zum Objekt nach der letzten Linse des Mirau-Interferometers und nach dem Strahlteiler des Mirau-Interferometers aus dem diesen Strahlteiler passierenden Teilstrahlenbündel mittels Diffraktion in Transmission an einer statischen oder einer dynamischen, diffraktiv-optischen Zonenlinse (DOZL) ein gebeugtes Strahlenbündel O_N (N=1, 2, 3... oder -1, -2, -3...) in der ersten oder einer höheren Beugungsordnung N (N=1, 2, 3) oder niedrigeren Beugungsordnung N als der nullten (N=-1, -2, -3). Dieses gebeugte Strahlenbündel O_N (N=1, 2, 3... oder -1, -2, -3...) ist nach Fokussierung auf das Objekt im Objektraum chromatisch längs aufgespalten, so dass in Abhängigkeit von der Wellenlänge des Lichts eine Reihe oder eine Vielzahl von Reihen von Fokuspunkten in der Tiefe ausgebildet wird. Dabei erfolgt bei einer spektral breitbandigen Quelle die Aufspaltung von Fokuspunkten gleichzeitig und bei einer Wellenlängen-durchstimmbaren Quelle zeitsequenziell. Nach Reflexion des Teilstrahlenbündels O_N (N=1, 2, 3... oder -1,-2, -3...) wird am Objekt ein Objekt-Teilstrahlenbündel vom rückreflektierten Strahlenbündel durch Beugung an der diffraktiv-optischen Zonenlinse in derselben Ordnung wie im Hinlauf, nämlich O_N_N (N=1, 2, 3... oder -1, -2, -3...) gebildet, so dass das Objektteilstrahlenbündel O_N_N (N=1, 2, 3... oder -1, -2, -3...) nach dem Passieren der diffraktiv-optischen Zonenlinse wieder im Strahlengang zurückläuft.

Dabei wird aus dem Eingangsstrahlenbündel im Hinlauf auf die Fläche des Strahlteilers, dessen Strahlteilerfläche parallel zu der diffraktiv-optischen Zonenlinse liegt, auch noch ein Teilstrahlenbündel gebildet, welches im Weiteren als Referenzstrahlenbündel R dient.

Im Rücklauf von der Referenzspiegelfläche, auf welchen das Referenzstrahlenbündel R fokussiert wird, läuft das Referenzteilstrahlenbündel R nach dem Passieren des Strahlteilers in Reflexion wieder im Strahlengang zurück. Dabei bildet sich auf der Referenzspiegelfläche jeweils nur ein Fokusfleck oder eine Vielzahl von lateralen Fokusflecken - bei einer Multipunktlichtquelle - aus.

Dabei ist zwischen dem Objekt-Teilstrahlenbündel O_N_N (N=1, 2, 3... oder -1, -2,-3...) und dem Referenzstrahlenbündel R der optische Gangunterschied ungleich null gemacht, wenn auf einen Objektpunkt fokussiert ist. Weiterhin verlaufen das Objekt-Teilstrahlenbündel O_N_N (N=1, 2, 3... oder -1, -2, -3...) und das Referenzstrahlenbündel R nach der diffraktiv-optischen Zonenlinse parallel und kommen zur Interferenz und anschließend werden mittels Teilung der Amplitude der Wellenfronten die beiden miteinander interferierenden Teilstrahlenbündel aus dem Strahlengang ausgekoppelt. Anschließend wird eine konfokale Diskriminierung des Lichts der interferierenden Teilstrahlenbündel durchgeführt.

Dabei erfahren die interferierenden Bündel O_N_N (N=1, 2, 3... oder -1, -2, -3...) und R zur spektroskopischen Analyse eine Lateralaufspaltung mittels Dispersion oder Diffraktion. Es kann eine FFT des Intensitätsverlaufs erfolgen, um aus dem sich mittels FFT ergebenden Wertes nach Kalibrierung die Tiefenposition eines Objektpunktes zu bestimmen.

Andererseits ist es auch möglich, dass die interferierenden Bündel O_N_N (N=1, 2, 3... oder -1, -2, -3...) und R eine weitere Interferenzbildung mittels scannendem Zweistrahl-Interferometer, also mit zeitlichem Variieren des optischen Gangunterschieds zur optische Analyse, erfahren können, wobei der im scannenden Zweistrahl-Interferometer beim Scan durchfahrene optische Gangunterschied mindestens einmal genau auch dem im Interferometer zwischen Objekt-Teilstrahlenbündel O_N_N (N=1, 2, 3... oder -1, -2, -3...) und Referenzstrahlenbündel R bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird. Dies ermöglicht, dass ein Objekt flächig erfasst werden kann. Dabei kann das scannende Zweistrahl-Interferometer als Michelson-Inteferometer mit Optiken am Ein- und am Ausgang des Michelson-Inteferometers ausgebildet sein.

Andererseits ist es auch möglich, dass die interferierenden Bündel Objekt-Teilstrahlenbündel O_N_N (N=1, 2, 3... oder -1, -2, -3...) und Referenzstrahlenbündel R eine weitere Interferenzbildung mittels statischen Zweistrahl-Interferometer zur Objekt-Teilstrahlenbündel optischen Analyse erfahren, also einem Zweistrahl-Interferometer mit lateraler Variation des optischen Gangunterschieds, beispielsweise durch einen verkippten oder treppenförmig ausgebildeten Interferometerspiegel, wobei die Orte unterschiedlichen Gangunterschieds Pixeln einer Zeilen- oder Flächenkamera zugeordnet sind. Dies ermöglicht, beim Messen des Abstandes eines Objektpunktes auf das mechanische Scannen zu verzichten.

Dabei entspricht zumindest näherungsweise der im Zweistrahl-Interferometer auftretende optische Gangunterschied dem Betrag des - im Mirau-Interferometer zwischen O_N_N (N=1, 2, 3... oder -1, -2, -3...) und Referenzstrahlenbündel R - bestehenden optischen Gangunterschied in mindestens einem Objektpunkt, der einem Element der Zeilen- oder Flächenkamera zugeordnet ist.

Andererseits ist es im Fall der Anwendung einer Wellenlängen-durchstimmbaren Quelle elektromagnetischer Strahlung auch möglich, dass beim Scan der Wellenlänge ein moduliertes periodisches optisches Signal erzeugt wird und mittels Punktempfänger das Signal über der Zeit detektiert wird und dieses über der Wellenlänge ausgewertet wird. Vorteilhafterweise kann gemäß der vorliegenden Erfindung ein größerer Arbeitsabstand der letzten Fläche der Sensoranordnung, also der Referenzspiegelfläche, von der Objektoberfläche erhalten werden, als beispielsweise 200 μm.

Gemäß einer bevorzugten Ausführungsform der Erfindung ist ein fasergekoppeltes Michelson-Interferometer einen Strahlteiler, der als x-Koppler ausgebildet ist, und einen Referenzspiegel oder eine Referenzspiegelfläche für ein weiteres multispektrales interferometrisches Verfahren zur hochdynamischen Objekttiefenabtastung. Dabei ist dem Michelson-Interferometer eine spektral breitbandige oder eine Wellenlängen-durchstimmbare Quelle elektromagnetischer Strahlung im DUV-, im UV-; im VIS-, im NIR- oder im MIR- oder auch im FIR-Bereich zugeordnet, die im Weiteren als Lichtquelle bezeichnet wird und welche ein Eingangsstrahlenbündel für das Michelson-Interferometer erzeugt. Das Michelson-Interferometer ist im Objektstrahlengang mit einem Abbildungsstrahlengang für das zu prüfende Objekt und mindestens einem einzigen Punktempfänger, oder einem gerasterten Zeilenempfänger oder einem gerasterten Flächenempfänger für elektromagnetische Strahlung ausgebildet.

Es entsteht bei diesem Verfahren im Hinlauf des lichtes auf dem Weg zum Objekt nach dem x-Koppler des Michelson-Interferometers mittels Diffraktion in Transmission an einer statischen oder einer dynamischen, diffraktiv-optischen Zonenlinse (DOZL) ein gebeugtes Strahlenbündel O_N (N=1, 2, 3... oder-1, -2, -3...) in der ersten oder einer höheren Beugungsordnung N (N=1, 2, 3) oder niedrigeren Beugungsordnung N als der nullten (N=-1, -2, -3). Dieses gebeugte Strahlenbündel O_N (N=1, 2, 3... oder -1, -2, -3...) ist nach Fokussierung auf das Objekt im Objektraum chromatisch längs aufgespalten, so dass in Abhängigkeit von der Wellenlänge des Lichts eine Reihe oder eine Vielzahl von Reihen von Fokuspunkten in der Tiefe ausgebildet wird. Dabei erfolgt bei einer spektral breitbandigen Quelle die Aufspaltung von Fokuspunkten gleichzeitig und bei einer Wellenlängen-durchstimmbaren Quelle zeitsequenziell. Nach Reflexion des Teilstrahlenbündels O_N (N=1, 2, 3... oder -1, -2, -3...) wird am Objekt ein Objekt-Teilstrahlenbündel vom rückreflektierten Strahlenbündel durch Beugung an der diffraktiv-optischen Zonenlinse in derselben Ordnung wie im Hinlauf, nämlich O_N_N (N=1, 2, 3... oder -1, -2, -3...) gebildet, so dass das Objektteilstrahlenbündel O_N_N (N=1, 2, 3... oder -1, -2, -3...) nach dem Passieren der diffraktiv-optischen Zonenlinse wieder im Strahlengang zurückläuft, konfokal mittels Faserende diskriminiert wird, anschließend den x-Koppler passiert und in eine Auskoppelfaser gelangt. Das Referenzbündel R entsteht im Hinlauf durch Teilung am x-Koppler und wird am Ende der Faser direkt an einer Referenzspiegelfläche oder am Ende einer der Faser nachgeordneten Fokussieroptik an einer Referenzspiegelfläche reflektiert. Das in der Faser zurücklaufende Licht passiert den x-Koppler und gelangt ebenfalls in die Auskoppelfaser, wo das Licht aus dem Objektstrahlengang sich mit dem aus dem Referenzstrahlengang überlagert und zur Interferenz kommt.

Dabei erfahren die interferierenden Bündel O_N_N (N=1, 2, 3... oder -1, -2, -3...) und R zur spektroskopischen Analyse eine Lateralaufspaltung mittels Dispersion oder Diffraktion. Es kann eine FFT des Intensitätsverlaufs erfolgen, um aus dem sich mittels FFT ergebenden Wertes nach Kalibrierung die Tiefenposition eines Objektpunktes zu bestimmen.

Andererseits ist es auch möglich, dass die interferierenden Bündel O_N_N (N=1, 2, 3 ... oder -1, -2, -3...) und R eine weitere Interferenzbildung mittels scannendem Zweistrahl-Interferometer, also mit zeitlichem Variieren des optischen Gangunterschieds zur optischen Analyse, erfahren, wobei der im scannenden Zweistrahl-Interferometer beim Scan durchfahrene optische Gangunterschied mindestens einmal genau auch dem im Michelson-Interferometer zwischen Objekt-Teilstrahlenbündel O_N_N (N=1, 2, 3... oder -1, -2, -3...) und Referenzstrahlenbündel R bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird. Dies ermöglicht, dass ein Objekt flächig erfasst werden kann. Dabei kann das scannende Zweistrahl-Interferometer als Michelson-Inteferometer mit Optiken am Ein- und am Ausgang des Michelson-Inteferometers ausgebildet sein.

Andererseits ist es auch möglich, dass die interferierenden Bündel, Objekt-Teilstrahlenbündel O_N_N (N=1, 2, 3... oder -1, -2, -3...) und Referenzstrahlenbündel R, eine weitere Interferenzbildung mittels statischem Zweistrahl-Interferometer zur optischen Analyse erfahren, also einem Zweistrahl-Interferometer mit lateraler Variation des optischen Gangunterschieds, beispielsweise durch einen verkippten oder treppenförmig ausgebildeten Interferometerspiegel, wobei die Orte unterschiedlichen Gangunterschieds Pixeln einer Zeilen- oder Flächenkamera zugeordnet sind. Dies ermöglicht beim Messen des Abstandes eines Objektpunktes auf das mechanische Scannen zu verzichten. Dabei entspricht zumindest näherungsweise der im Zweistrahl-Interferometer auftretende optische Gangunterschied dem Betrag des - im Michelson-Interferometer zwischen O_N_N (N=1, 2, 3... oder -1, -2, -3...) und Referenzstrahlenbündel R - bestehenden optischen Gangunterschied in mindestens einem Objektpunkt, der einem Element der Zeilen- oder Flächenkamera zugeordnet ist.

Andererseits ist es im Fall der Anwendung einer Wellenlängen-durchstimmbaren Quelle elektromagnetischer Strahlung auch möglich, dass beim Scan der Wellenlänge ein moduliertes periodisches optisches Signal erzeugt wird, und mittels Punktempfänger das Signal über der Zeit detektiert wird und dieses über der Wellenlänge ausgewertet wird.

Ein weiteres Beispiel, das zum Verständnis der Erfindung hilfreich ist, betrifft eine multispektrale interferometrische Anordnung zur hochdynamischen Objekttiefenabtastung oder -Profilerfassung mit einem objektabbildenden Zweistrahl-Interferometer mit einer Referenzspiegelfläche und mit einem zu prüfenden Objekt, wobei das Zweistrahl-Interferometer also mit einem Abbildungsstrahlengang für das zu prüfende Objekt und einem Punktempfänger, einem gerasterten Linienempfänger oder einem gerasterten Flächenempfänger für elektromagnetische Strahlung ausgebildet ist. Dabei ist dem Zweistrahl-Interferometer eine spektral breitbandige oder Wellenlängen-durchstimmbare Quelle elektromagnetischer Strahlung im UV, VIS oder IR-Bereich vorgeordnet, die im Weiteren als Lichtquelle bezeichnet wird, welche ein Eingangsstrahlenbündel für das Zweistrahl-Interferometer erzeugt.

Dabei ist das Zweistrahl-Interferometer mit einer vorzugsweise lichtzerstreuenden, diffraktiv-optischen Zonenlinse ausgebildet. Es kann aber auch eine lichtsammelnde diffraktiv-optische Zonenlinse angeordnet sein. Der diffraktiv-optischen Zonenlinse, die vorzugsweise lichtzerstreuend ausgebildet ist, ist vorzugsweise eine abbildende Linse oder ein Objektiv mit Referenzspiegelfläche mit oder ohne Strahlteilerschicht des Zweistrahl-Interferometers in Richtung des Verlaufs des Eingangsstrahlenbündels nachgeordnet. Die abbildende Linse mit Referenzspiegelfläche ist als Grinlinse mit Frontfläche mit Strahlteilerschicht ausgebildet, wobei die Frontfläche als teildurchlässige Referenzspiegelfläche des Zweistrahl-Interferometers wirkt. Dabei befindet sich vorzugsweise die diffraktiv-optische Zonenlinse zumindest näherungsweise in der Brennebene der Grinlinse, also der Brennebene auf der objektabgewandten Seite der Grinlinse. Dies führt vorteilhafterweise dazu, dass der Abbildungsmaßstab sich trotz lichtbeugender diffraktiv-optischer Zonenlinse im Objektraum nicht in Abhängigkeit von der Wellenlänge ändert.

Vorzugsweise ist dem Zweistrahl-Interferometer ein Spektrometer und ein gerasterter Linien- oder Flächenempfänger zur Detektion des Lichts nachgeordnet. Hierbei handelt es sich um eine Common-path-Zweistrahl-interferometer Anordnung, die sich gegenüber Vibrationen als robust erweist. Für die Kohärenz-Tomografie (Optical coherence tomography, OCT) an biologischen Proben kann eine beispielsweise wässrige Probe direkt der abbildenden Grinlinse mit teilreflektierender Frontfläche als teildurchlässige Referenzspiegelfläche, also beispielsweise einer Frontfläche mit Strahlteilerschicht, vorgeordnet sein. So können auch bewegte biologische Partikel in einer wässrigen Probe detektiert werden.

Das objektabbildende Zweistrahl-Interferometer kann vorzugsweise aber auch mit einer Mikrolinse oder einem Mikroobjektiv ausgebildet sein, deren objektzugewandte Frontfläche als teildurchlässige Referenzspiegelfläche des Zweistrahl-Interferometers wirkt.

Am Ausgang des Zweistrahl-Interferometers befinden sich bündelbegrenzende Mittel wie Blenden (Pinholes) oder Lichtleitfaserenden, die eine konfokale Diskriminierung des interferierenden Lichtes ermöglichen.

Der Referenzstrahlengang des Zweistrahl-Interferometers ist durch die Gestaltung der Grinlinse als achromatische Komponente zumindest näherungsweise auch achromatisch ausgebildet. So kann für das Licht aller Wellenlängen grundsätzlich eine kontrastreiche Interferenz bei der Detektion auftreten, da das Licht aller Wellenlängen im Referenzstrahlengang in gleicher Weise auf die Referenzspiegelfläche scharf fokussiert abgebildet werden kann.

Durch den im Zweistrahl-Interferometer von null verschiedenen optischen Gangunterschied - aufgrund der Objektentfemung von der Referenzspiegelfläche - sind die Lichtwellen unterschiedlicher Lichtwellenlänge bei der Detektion der Interferenz nicht in Phase und so ergibt sich über der Wellenzahl $(1/\lambda)$ - als reziprokem Wert der Lichtwellenlänge $\lambda$ - bei Verwendung einer Weißlichtquelle ein Signalverlauf, der ein Wavelet darstellt. Dazu wird vor der Detektion eine spektrale Aufspaltung des Lichtes vorgenommen. Andererseits kann die Wellenlänge der Lichtquelle auch zeitlich variiert werden, beispielsweise bei Verwendung eines Wellenlängen-durchstimmbaren Lasers. In diesem Fall kann eine grauwertempfindliche Zeilen- oder Flächenkamera zur Detektion eingesetzt werden.

Bei Verwendung eines Multiwellenlängenlasers mit einer Frequenzkamm-Charakteristik kann auch ein größerer optischer Gangunterschied im Zweistrahl-Interferometer zugelassen werden. Die Auswertung kann auf den bekannten FFT-Auswerte-Algorithmen basieren.

Die Beleuchtung der Oberfläche des Objektes kann auch mit einer größeren Anzahl von lateral in einer Linie angeordneten Foki erfolgen. Dann ist einer Kamera ein dispersives Spektrometermodul mit einem Eingangsspalt vorgeordnet, auf welchen die Linie der Foki scharf abgebildet wird. Dieses Spektrometermodul mit einer dem Eingangsspalt nachgeordneten internen Abbildungsstufe kann beispielsweise 100 spektrale Kanäle aufweisen, indem die spektrale Zerlegung des Lichtes auf ebenso 100 Pixel und senkrecht zur Ausbildung des Lichtbandbildes auf der Kamerafläche erfolgt. Betrachtet man einen spektralen Bereich von beispielsweise 200 nm von etwa 450 bis 650 nm, so beträgt die spektrale Auflösung in diesem Fall 2 nm. Vorteilhaft ist, wenn dem Pixel-Pitch etwa ein Achtel Streifen des periodischen Signals entspricht. Das Spektrometermodul kann dabei vorzugsweise als Geradsichtkeil oder mittels Gitter ausgebildet sein. Die spektrale Auflösung kann dabei auch 0,1 nm betragen.

Andererseits ist es auch vorzugsweise möglich, dass anstelle des Spektrometermoduls dem objektabbildenden Zweistrahl-Interferometer ein scannendes Zweistrahl-Interferometer nachgeordnet ist, welches eine optische Analyse des Interferenzlichts am Ausgang des objektabbildenden Zweistrahl-Interferometers ermöglicht. In Abhängigkeit von der Feinstruktur des Spektrums des Interferenzlichts eines Objektpunktes entsteht neben dem Weißlicht-Interfefogramm-Maximum-Peak am optischen Gangunterschied null ein weiteres Weißlicht-Interferogramm und zwar genau bei dem Betrag des optischen Gangunterschieds xM, der im objektabbildenden Zweistrahl-Interferometer für diesen Objektpunkt auch besteht. Das scannende Zweistrahl-Interferometer kann dabei hochdynamisch ausgebildet sein und kann beispielsweise mehr als 100 Scans pro Sekunde ausführen, um Weißlicht-Interferogramme in kurzer Zeit zu gewinnen. Dafür muss der Linien- oder Flächenempfänger entsprechend ausgelegt werden.

Andererseits ist es auch vorzugsweise möglich, dass anstelle des Spektrometermoduls dem objektabbildenden Zweistrahl-Interferometer ein statisches Zweistrahl-Interferometer nachgeordnet ist, welches eine optische Analyse des Interferenzlichts am Ausgang des objektabbildenden Zweistrahl-Interferometers durch eine laterale Aufspaltung des Lichts eines Objektpunktes in eine Vielzahl von Wegen mit variierendem optischen Gangunterschied im statischen, analysierenden Zweistrahl-Interferometer ermöglicht. So kann ein vollständiges Weißlichtinterferogramm auf einem Zeilen- oder einem Flächenempfänger in einem Frame aufgenommen werden.

Vorzugsweise ist bei einer multispektralen interferometrischen Anordnung zur hochdynamischen Objekttiefenabtastung und -Profilerfassung das objektabbildende Zweistrahl-Interferometer als Mirau- Interferometer mit Referenzspiegel ausgebildet. Dabei ist zwischen der Strahlteilerplatte mit Strahlteilerschicht des Mirau-Interferometers und dem Objekt das diffraktiv-optische Zonenelement (DOZE) angeordnet. Dies führt zu einer sehr kompakten Anordnung. Dieses kann vorzugsweise als transmissiv arbeitende diffraktiv-optische Zonenlinse ausgebildet sein. Vorzugsweise ist dabei die transmissiv arbeitende diffraktiv-optische Zonenlinse so ausgebildet, dass diese ihre optische Wirkung vorwiegend in der ersten positiven oder der ersten negativen Beugungsordnung entfaltet. Vorzugsweise ist die transmissiv arbeitende diffraktive Zonenlinse als lichtzerstreuende Zonenlinse ausgebildet. Daraus ergibt sich von derselben ein größerer freier Abstand zum Objekt. Vorzugsweise sind weiterhin die Strahlteilerplatte mit Strahlteilerschicht und die transmissiv arbeitende, diffraktiv-optische Zonenlinse zu einer Kittgruppe vereinigt, wobei die Strahlteilerschicht innerhalb der Kittgruppe angeordnet ist und die Substratdicken und die Substratwerkstoffe der Strahlteilerplatte und der diffraktiv-optischen Zonenlinse gleich gemacht sind. Durch diese Symmetrisierung minimieren sich Störungen wie beispielsweise das Chirping im optischen Signal, was die Signalauswertung erheblich vereinfachen kann. Ein weiteres Beispiel, das zum Verständnis der Erfindung hilfreich ist, umfaßt eine weitere multispektrale interferometrische Anordnung zur hochdynamischen Objekttiefenabtastung ein Michelson-Interferometer mit einem Strahlteiler und mit einem Referenzspiegel, wobei das Michelson-Interferometer fasergekoppelt ist und der Strahlteiler als x-Koppler ausgebildet ist. Dabei ist dem fasergekoppelten Michelson-Interferometer eine spektral breitbandige oder eine Wellenlängen-durchstimmbare Quelle elektromagnetischer Strahlung im DUV-, im UV-, im VIS-, im NIR-oder im MIR- oder auch im FIR-Bereich zugeordnet, die im Weiteren als Lichtquelle bezeichnet wird und welche ein Eingangsstrahlenbündel für das Michelson-Interferometer erzeugt. Das fasergekoppelte Michelson-Interferometer ist im Objektstrahlengang mit einem Abbildungsstrahlengang für das zu prüfende Objekt und mindestens einem einzigen Punktempfänger, oder einem gerasterten Zeilenempfänger oder einem gerasterten Flächenempfänger für elektromagnetische Strahlung ausgebildet.

Es entsteht bei dieser Anordnung im Hinlauf des Lichtes auf dem Weg zum Objekt nach dem x-Koppler des fasergekoppelten Michelson-Interferometers an einer statischen oder einer dynamischen, diffraktiv-optischen Zonenlinse (DOZL), die dem Strahlteiler in Lichtrichtung des Eingangsstrahlenbündels im Abbildungsstrahlengang für das zu prüfende Objekt nachgeordnet ist, vorzugsweise mittels Diffraktion in Transmission ein gebeugtes Strahlenbündel O_N (N=1, 2, 3... oder -1, -2, -3...) in der ersten oder einer höheren Beugungsordnung N (N=1, 2, 3) oder niedrigeren Beugungsordnung N als der nullten (N=-1, -2, -3). Die diffraktiv-optische Zonenlinse ist also für eine einzige Beugungsordnung ausgebildet, vorzugsweise für die erste Beugungsordnung oder auch eine höhere Beugungsordnung. Dieses gebeugte Strahlenbündel O_N (N=1, 2, 3... oder -1, -2, -3...) ist nach Fokussierung auf das Objekt im Objektraum chromatisch längs aufgespalten, so dass in Abhängigkeit von der Wellenlänge des Lichts eine Reihe oder eine Vielzahl von Reihen von Fokuspunkten in der Tiefe ausgebildet wird. Dabei erfolgt bei einer spektral breitbandigen Quelle die Aufspaltung von Fokuspunkten gleichzeitig und bei einer Wellenlängendurchstimmbaren Quelle zeitsequenziell. Nach Reflexion des Teilstrahlenbündels O_N (N=1, 2, 3... oder -1, -2, -3 ...) wird am Objekt ein Objekt-Teilstrahlenbündel vom rückreflektierten Strahlenbündel durch Beugung an der diffraktiv-optischen Zonenlinse in derselben Ordnung wie im Hinlauf, nämlich O_N_N (N=1, 2, 3... oder-1, -2, -3...) gebildet, so dass das Objektteilstrahlenbündel O_N_N (N=1, 2, 3... oder-1, -2, -3...) nach dem Passieren der diffraktiv-optischen Zonenlinse wieder im Strahlengang zurückläuft, konfokal mittels Faserende diskriminiert wird, anschließend den x-Koppler passiert und in eine dem x-Koppler nachgeordnete Auskoppelfaser gelangt. Das Referenzbündel R entsteht im Hinlauf durch Teilung am x-Koppler und wird am Ende der Faser direkt an einem dort angeordneten Referenzspiegel oder am Ende einer der Faser nachgeordneten Fokussieroptik an einer Referenzspiegelfläche reflektiert. Das in der Faser zurücklaufende Licht passiert den x-Koppler und gelangt ebenfalls in die dem x-Koppler nachgeordnete Auskoppelfaser, wo das Licht aus dem Objektstrahlengang sich mit dem aus dem Referenzstrahlengang überlagert und es zur Interferenz kommt.

Dabei erfahren die interferierenden Bündel O_N_N (N=1, 2, 3... oder -1, -2, -3...) und R zur spektroskopischen Analyse mit einem - der Auskoppelfaser nachgeordneten Spektrometer - eine Lateralaufspaltung mittels Dispersion oder Diffraktion. Es kann eine FFT des Intensitätsverlaufs erfolgen, um aus dem sich mittels FFT ergebenden Wertes nach Kalibrierung die Tiefenposition eines Objektpunktes zu bestimmen.

Andererseits ist es auch möglich, dass die interferierenden Bündel O_N_N (N=1, 2, 3... oder -1, -2, -3...) und R eine weitere Interferenzbildung mittels einem nachgeordneten, scannendem Zweistrahl-Interferometer, also mit zeitlichem Variieren des optischen Gangunterschieds zur optischen Analyse, erfahren können, wobei der im scannenden Zweistrahl-Interferometer beim Scan durchfahrene optische Gangunterschied mindestens einmal genau auch dem im Michelson-Interferometer zwischen Objekt-Teilstrahlenbündel O_N_N (N=1, 2, 3... oder -1, -2, -3...) und Referenzstrahlenbündel R bestehenden optischen Gangunterschied im Betrag entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird. Dies ermöglicht, dass ein Objekt flächig erfasst werden kann. Dabei kann das scannende Zweistrahl-Interferometer als Michelson-Interferometer mit Optiken am Ein- und am Ausgang des Michelson-Interferometers ausgebildet sein.

Andererseits ist es auch möglich, dass die interferierenden Bündel Objekt-Teilstrahlenbündel O_N_N (N=1, 2, 3... oder -1, -2, -3...) und Referenzstrahlenbündel R eine weitere Interferenzbildung mittels der Auskoppelfaser nachgeordnetem statischem Zweistrahl-Interferometer zur optischen Analyse erfahren, also einem Zweistrahl-Interferometer mit lateraler Variation des optischen Gangunterschieds, beispielsweise durch einen verkippten oder treppenförmig, siehe oben, ausgebildeten Interferometerspiegel, wobei die Orte unterschiedlichen Gangunterschieds Pixeln einer Zeilen- oder Flächenkamera zugeordnet sind. Dies ermöglicht beim Messen des Abstandes eines Objektpunktes auf das mechanische Scannen zu verzichten.

Dabei entspricht zumindest näherungsweise der im Zweistrahl-Interferometer auftretende optische Gangunterschied dem Betrag des - im fasergekoppelten Michelson-Interferometer zwischen dem Objektstrahlenbündel O_N_N (N=1, 2, 3... oder -1, -2, -3...) und dem Referenzstrahlenbündel R - bestehenden optischen Gangunterschied in mindestens einem Objektpunkt, der einem Element der Zeilen- oder Flächenkamera zugeordnet ist.

Andererseits kann auch eine Wellenlängen-durchstimmbare Quelle elektromagnetischer Strahlung angeordnet sein, so dass beim Scan der Wellenlänge ein moduliertes periodisches optisches Signal erzeugt wird und mittels nachgeordneten Punktempfänger das Signal über der Zeit detektiert wird und dieses über der Wellenlänge ausgewertet wird.

Gemäß der vorliegenden Erfindung erfolgt bei einem Verfahren zur konfokalen Spektral-Interferometrie eine mikroskopische Abbildung der Objektoberfläche und auch des Inneren des Objekts mittels Fokussier-Systems auf einen gerasterten Empfänger elektromagnetischer Strahlung zur Detektion der interferierenden elektromagnetischen Strahlung. Dieses spektral-interferometrische konfokale Verfahren wird erfindungsgemäß insbesondere auch zur Erfassung des Abstandes, des Profils und der Form und zur optischen Kohärenz-Tomografie (OCT) und optischen Kohärenz-Mikroskopie (OCM), insbesondere auch zur dreidimensionalen OCM, von biologischen und technischen Objekten oder Objektdetails Objekten in Auf- und/oder Durchlicht mit

- entweder mindestens einer Multiwellenlängen-Quelle, wobei bei der Multiwellenlängen-Quelle dem gerasterten Empfänger ein dispersives Spektrometer vorgeordnet ist,

- oder mindestens einer Wellenlängen-durchstimmbaren Quelle elektromagnetischer Strahlung angewendet.

Erfindungsgemäß wird die chromatisch-konfokale Technik mit der spektralen Zweistrahl-Interferometrie verfahrensmäßig verbunden, wobei sowohl eine vorbestimmte chromatische Tiefenaufspaltung der elektromagnetischen Strahlung im Fokussier-System erfolgt als auch

1. entweder bei Nutzung der Multiwellenlängen-Quelle eine Spektralanalyse der detektierten interferierenden elektromagnetischen Strahlung mittels dispersivem Spektrometer durchgeführt wird, so daß ein Spektrum zur Verfügung steht

2. oder bei Nutzung der Wellenlängen-durchstimmbaren Quelle eine Wellenlängendurchstimmung vorbestimmt durchgeführt und die interferierende elektromagnetische Strahlung bei der Wellenlängendurchstimmung detektiert wird, so daß über der Zeit ein Spektrum aufgenommen wird. Aus dem Spektrum wird über die Kenntnis der vorbestimmten Tiefenaufspaltung im chromatisch-konfokalen System und die zumindest näherungsweise Kenntnis des Brechungsindexes die z-Position eines jeden Objektdetails und die zugehörige Lichtintensität bestimmt.

Dabei ist es verfahrensmäßig unerheblich, ob bei Einsatz einer spektralbreitbandigen Lichtquelle eine Spektralanalyse mittels eines dispersiven Spektrometers, siehe erste Variante, oder ob bei Einsatz einer spektral schmalbandigen Lichtquelle, beispielsweise eine Wellenlängen-durchstimmbare Laserlichtquelle, eine Wellenlängendurchstimmung, siehe zweite Variante, durchgeführt wird. Für die Gewinnung einer dreidimensionalen Information vom Objekt ergeben sich die folgenden Bedingungen:

Erste Variante: Dieses Verfahren erfordert einen Lateral-Scan, da die spektrale Analyse eines Linienschnitts den Einsatz eines flächenhaften Empfängers elektromagnetischer Strahlung voraussetzt. Der Einsatz eines spektral hinreichend hochauflösenden, flächenhaften Empfängers elektromagnetischer Strahlung mit der Spektralachse in der Tiefe des Sensor-Chips ist in naher Zukunft eher nicht zu erwarten.

Zweite Variante: Dieses Verfahren erfordert einen Wellenlängen-Scan. Jedoch ist bei Einsatz eines flächenhaften Empfängers elektromagnetischer Strahlung kein Lateral-Scan erforderlich.

Die Tiefenaufspaltung der elektromagnetischen Strahlung entspricht der Längsaufspaltung, auch als chromatische Längsaberration oder chromatische Längsaufspaltung von optischen Systemen bekannt. Das erfindungsgemäße Verfahren wird im Weiteren als chromatisch-konfokale Spektral-Interferometrie (chromatic confocal spectral interferometry, CC-SI) bezeichnet.

Dieses Verfahren kann auch zur optischen Kohärenz-Tomografie oder zur optischen Kohärenz-Mikroskopie von transparenten und translucenten Objekten eingesetzt werden. Die Kohärenzverfahren können neben den ein-, zwei- oder dreidimensionalen Koordinaten von Objektdetails im Meßvolumen auch den Wert der Reflexion oder den Wert der Lichtstreuung eines Objektdetails liefern.

Dabei ist der optische Gangunterschied im Interferometer vorzugsweise ungleich null gemacht. Bei dem Vorhandensein eines reflektierenden oder lichtstreuenden Bereiches, also einem Objektdetail im Objektvolumen, wird im Abtastvorgang vorzugsweise mindestens ein Wavelet über der spektralen Achse des Spektrometers erzeugt. Dabei wird unter Wavelet in dieser Schrift ein Signal verstanden, welches periodisch ist, wobei sich die Frequenz oder die Periodenlänge auch ändern kann, und das Signal über eine obere Einhüllenden verfügt, also über seinen Verlauf moduliert ist. Dabei kann die obere Einhüllende des Signals ein Maximum aufweisen. Die Halbwertsbreite des Wavelets ist durch die Größe der chromatischen Tiefenaufspaltung im chromatisch konfokalen System vorzugsweise vorbestimmt einstellbar gemacht, wobei hier auch die Abhängigkeit der Halbwertsbreite von der numerischen Apertur des Fokussier-Systems zu beachten ist. Die Anzahl der Perioden unter der Einhüllenden des Wavelets ist vorzugsweise durch die Dimensionierung von optischem Gangunterschied sowie der Gangunterschiedsänderung über der Wellenlänge ebenfalls vorbestimmt einstellbar gemacht.

Anmerkung: Bei Aufnahme eines Interferogramm über dem optischen Gangunterschied mittels Gangunterschieds-Scan, die hier aber verfahrensmäßig nicht ausgeführt wird, führt eine im Interferometer vorhandene Gangunterschiedsänderung über der Wellenlänge, beispielsweise durch Dispersion, zum bekannten Chirping im Interferogramm, also zu einer Asymmetrie in der Einhüllenden des Interferogramms und zu einer Variation der Frequenz über dem optischen Gangunterschied.

Dabei ist es für die Bestimmung des Schwerpunktes des Wavelets auf der spektralen Achse des Spektrometers von Vorteil, wenn mindestens drei bis fünf Perioden mit Amplituden von wenigstens 50% der Maximalamplitude sich unter der Einhüllenden befinden. Die Maximalamplitude des Wavelets oder ein vergleichbarer Wert wie das Maximum der Einhüllenden wird vorzugsweise ebenfalls bestimmt, um ein Maß für die Reflexion oder die Lichtstreuung des jeweiligen Objektdetails im Objektvolumen zu erhalten. Beispielsweise kann die Größe des Maximums der oberen Einhüllenden des Wavelets bestimmt werden.

Der Schwerpunkt der Einhüllenden des Wavelets kann mit den in der Literatur bereits beschriebenen Auswerteverfahren bestimmt werden, siehe beispielsweise in Applied Optics, Vol. 39, No. 8, vom 10. März 2000, Seite 1290 bis 1295. Wird das spektral-interferometrische, konfokale Verfahren mit gekoppelter chromatischkonfokaler mikroskopischer Technik beispielsweise an nicht streuenden, also mehr transparenten Volumenobjekten oder an Oberflächen angewendet, kann beim Nichtauftreten von Speckels oder Speckels mit hinreichend geringem Kontrast mit Vorteil für die Tiefenmeßgenauigkeit auch die Phase des Wavelets ausgewertet werden.

Speckels reduzieren sich in Ihrem Kontrast, wenn die numerische Apertur zur Objektbeleuchtung bekannterweise hinreichend groß gemacht ist. Diese Phase trägt also bei hinreichend kleinem Speckle-Kontrast eine Information über die wellenoptische Weglänge im Interferometer und damit auch eine Abstands- oder Tiefeninformation über ein Objektdetail. Somit kann die Phaseninformation unter diesen Voraussetzungen auch zu einer hochgenauen Bestimmung der Tiefenposition des optisch angetasteten Objektdetails genutzt werden.

Das Fokussier-System wird neben der Abbildung des Objekts auch zur Erzeugung der konfokalen Foki verwendet, die vorzugsweise Fokusflecken oder Fokuspunkte darstellen.

Es kann aber auch die Ausbildung von linienhaften Fokusflecken oder Fokuslinien durchgeführt werden. Dies kann für die Lichtausbeute von Vorteil sein, wenn die Genauigkeitsanforderungen bei der Objekterfassung nicht sehr hoch sind.

Für das Fokussier-System soll Folgendes gelten: Das Fokussier-System enthält mindestens ein Fokussier-Objektiv. Dieses Fokussier-Objektiv kann sowohl eine Mikrolinse, die am besten asphärisch ausgebildet ist, als auch ein handelsübliches Mikroskopobjektiv darstellen. Das Fokussier-System kann also mit dem Fokussier-Objektiv identisch sein, wenn dieses chromatisch ausgebildet ist. In dieser Schrift wird in der Regel davon ausgegangen, daß das Fokussier-System mindestens die folgenden Komponenten aufweist:

- ein zumindest nahezu achromatisches Fokussier-Objektiv
- und eine chromatische Komponente, welche diesem Fokussier-Objektiv zugeordnet ist.

Die chromatische Komponente kann ein diffraktiv-optisches Element sein, welches sich in der Fourier-Ebene des Fokussier-Objektivs befindet.

Beim Objektvolumen kann es sich in der Regel um mikroskopisch kleine Bereiche, auch in der Ausdehnung von wenigen Wellenlängen des verwendeten Lichts, mit eher geringen Reflexionsgradunterschieden im eher trüben Material handeln. Dieses kann insbesondere auch Biomaterial sein, welches mikroskopisch kleine Streuzentren oder Reflexionszentren aufweist. Diese Bereiche sollen mittels optischen Tastkopfs auch erfaßt werden können.

Das hier vorgeschlagene, erfinderische Verfahren kann auch zur Bestimmung der Art des biologischen Gewebes, also zur Unterscheidung von Weichteil- oder Knochengewebe, indem die geometrisch-räumliche Struktur der Biomaterialien sichtbar gemacht wird, angewendet werden.

Das hier vorgeschlagene, erfinderische Verfahren verbindet also die bekannte chromatisch-konfokale Technik zur Tiefenerfassung Profil- oder 3D-Erfassung, im mikroskopischen Maßstab auch als chromatisch-konfokale Mikroskopie (CCM) bezeichnet, mit der bekannten Weißlicht-interferometrie (WLI) mit spektraler Analyse, auch als Fourier-Domain-WLI, Fourier-Domain OCT, als Spektral-Radar oder auch als Spektral-Interferometrie bekannt, s. a. [1], S. 335-350.

Bei der chromatisch-konfokalen Technik zur Tiefenerfassung erfolgt eine Tiefenaufspaltung der Foki im Objektvolumen durch eine chromatische Längsaberration. Das Verfahren kann im Auflicht oder auch im Durchlicht angewendet werden, wobei bei der weiteren Darstellung stets der Auflichtansatz dargestellt wird.

Bei dem Verfahren erfolgt der Einsatz einer im Vergleich zu einem Laser breitbandigen Quelle elektromagnetischer Strahlung. Es kann vorzugsweise auch eine Weißlichtquelle, beispielsweise ein Weißlicht-Kontinuums-Laser, eingesetzt werden.

Bei dem Verfahren zur konfokalen Spektral-Interferometrie, insbesondere auch zur Erfassung des Abstandes, des Profils und der Form und zur zwei- oder dreidimensionalen Tomografie und -Mikroskopie (OCT, OCM) von biologischen Objekten in Auf- und/oder Durchlicht wird eine Multiwellenlängen-Quelle elektromagnetischer Strahlung oder einer Vielzahl von Multiwellenlängen-Quellen eingesetzt. Die Multiwellenlängen-Quellen können punktförmig ausgebildet sein, beziehungsweise es werden mittels Faserenden oder Mikroblenden eine oder mehrere punktförmige Multiwellenlängen-Quellen dargestellt. Mit dem Fokussier-System erfolgt die Beleuchtung des Objektvolumens. Außerdem kann mit dem Fokussier-System bei Bedarf auch eine mikroskopische Abbildung des Inneren des Objektvolumens zumindest punktweise erfolgen. Die Abbildung dieses Inneren erfolgt auf einen möglichst hochsensitiven und möglichst schnellen, gerasterten Empfänger elektromagnetischer Strahlung, wobei dem gerasterten Empfänger in Lichtrichtung ein Spektrometer vorgeordnet ist. Dieses Spektrometer kann als Punkt-oder Linienspektrometer ausgebildet sein. Dabei ist auch eine Faserkopplung des Spektrometers sehr von Vorteil.

Dabei wird vorzugsweise bei einem optischen Gangunterschied ungleich null im Zweistrahl-Interferometer gearbeitet. Bei einem reflektierenden oder lichtstreuenden Bereich auch in einem eher trüben Medium wird so vorzugsweise mindestens ein Wavelet über der spektralen Achse erzeugt.

Vorzugsweise wird bei dem Verfahren zur konfokalen Spektral-Interferometrie zur simultanen Auslesung von Details des Objektvolumens an der Grenzschicht desselben sowie bei Bedarf auch in verschiedener Tiefe desselben zum einen die Signalamplitude von Wavelets ausgewertet. Es soll aber auch die Tiefenposition von Details des Objektvolumens bestimmt werden. Dieses kann andererseits durch die Bestimmung des Maximums der Einhüllenden des Wavelets auf der Wellenlängenachse erfolgen.

Es erfolgt hier bei Anwendung der chromatisch-konfokalen Spektral-Interferometrie mittels optischen Fokussier-Systems also sowohl eine Beleuchtung als auch eine mikroskopische Abbildung des Inneren des zumindest teilweise transparenten oder auch trüben Objektvolumens in verschiedenen Tiefen gleichzeitig. Mittels einer chromatischen Längsaberration im optischen Fokussier-System des Abtastkopfes erfolgt eine Separierung der Foki vorzugsweise und zumindest näherungsweise entlang einer Geraden im Objektvolumen. Die Längsaberration wird auch als Tiefenaufspaltung bezeichnet.

Dabei kann das verwendete Interferometer vom Michelson- oder auch vom Linnik-Typ sein. Ein Linnik-Interferometer kann aus technischen Gründen eine Fokussierung mit einer recht hohen numerischen Apertur ermöglichen, beispielsweise einer numerischen Apertur von 0,8. Die mikroskopischen Immersionsverfahren können hier ebenfalls angewendet werden.

Das Zweistrahl-Interferometer kann aber auch als zumindest teilweise ausgebildetes Common-path-Interferometer gestaltet sein. Dann kann das Zweistrahl-Interferometer also auch als ein Fizeau- oder auch ein Mirau-Interferometer ausgebildet sein.

Andererseits ist auch die Ausbildung des Zweistrahl-Interferometers als Mach-Zehnder-Interferometer (MZI) möglich. Dieses kann auch fasergekoppelt sein, wobei sich beim Mach-Zehnder-Interferometer die konfokale Diskriminierung vorzugsweise im Objektstrahlengang befindet. Die Anwendung eines Mach-Zehnder-Interferometers ermöglicht recht einfach die Realisierung Folgenden:

1. Die numerische Apertur für den Beleuchtungsstrahlengang kann in einfacher Weise vorbestimmt kleiner gemacht werden als die numerische Apertur für den Detektionsstrahlengang. Der Referenzstrahlengang kann auch beim Mach-Zehnder-Interferometer mittels Faser realisiert werden. Somit erlaubt die Anwendung des Mach-Zehnder-Interferometers auch die Miniaturisierung des optischen Tastkopfes.

2. Die Anwendung eines Mach-Zehnder Interferometers ermöglicht weiterhin eine besonders hohe Sensitivität bei der Messung, da es nur vergleichsweise geringe Störeinflüsse gibt, beispielsweise wenig Einflüsse durch unerwünschte Reflexionen an einem Faserende.

3. Die Nutzung eines Mach-Zehnder-Interferometers gestattet die Verwendung des zweiten Inerferometerausganges, der zumindest näherungsweise gegenphasig arbeitet. So kann durch Subtraktion der mit den beiden Inerferometerausgängen gewonnenen Signale die Signalamplitude verdoppelt und der Gleichanteil eliminiert werden. Dies verbessert das Signal-Rausch-Verhältnis.

Weiterhin kann im optischen Fokussier-System des optischen Abtastkopfes die chromatische Tiefenaufspaltung von Foki im Objektraum in verschiedener Art und Weise mit einer im Optikdesign des Fokussier-Systems exakt quantifizierten chromatischen Längsaberration erfolgen:

Erstens durch eine spezielle chromatische Ausbildung einer rein dispersiven Fokussier-Optik,
oder zweitens durch eine dispersive Fokussier-Optik mit eher geringer chromatischer Längsaberration, jedoch zusätzlich mit mindestens einem diffraktiv-optischen Element, also durch ein Hybridsystem, wobei das diffraktiv-optische Element in einer von null verschiedenen Beugungsordnung genutzt wird, oder
drittens durch die Ausbildung der Fokussier-Optik als rein diffraktives System wobei auch hier das diffraktiv-optische Element in einer von null verschiedenen Beugungsordnung genutzt wird.

Dabei ist die Größe der chromatischen Längsaberration der Fokussier-Optik des genutzten Wellenlängenbereiches vorzugsweise so eingestellt, daß eine Tiefenaufspaltung der Foki durchgeführt wird, die mindestens der gewünschten Abtasttiefe im Volumen des Objekts entspricht. Darüber hinaus ist der genutzte chromatische Bereich der spektralen Auflösung dem verwendeten Spektrometer angepaßt. Der Vorteil des Prinzips der chromatischen Längsaberration oder Längsaufspaltung, die eine Tiefenaufspaltung darstellt, in der Fokussier-Optik besteht darin, daß auch bei einer Verschiebung des Abtastkopfes in der Tiefe bei einem Stoß auf das System, dennoch Licht zum Zeitpunkt nach dem Stoß - jedoch mit etwas anderer Wellenlänge als vor dem Stoß - einen Fokus scharf auf denselben Objektpunkt ausbilden kann. Die Auswerteprozessoren werten genau dieses Licht, welches die Information über das Tiefenprofil an dieser Stelle trägt, aus. Mittels Informationen aus Messungen des Umfeldes dieses Meßpunktes kann gegebenenfalls eine Korrektur des durch den Stoß verfälschten Tiefenwertes erfolgen.
Licht wird hier in der Schrift stets im weitgefaßten Sinn von elektromagnetischer Strahlung im UV-, VIS- oder Infrarotbereich verwendet.
Das diffraktiv-optische Element im Fokussier-System kann dabei vorzugsweise als positive oder negative Zonenlinse ausgebildet sein. Es gibt technische Argumente für beide Varianten. Langwelliges Licht durchdringt jedoch ein biologisches Objekt in der Regel besser. Außerdem ist die Frequenzvariation der Wavelets über der Spektralachse geringer, was die Auswertung vereinfachen kann. Diese Gründe sprechen für eine negative Zonenlinse.
Die diffraktiv-optische Zonenlinse kann vorzugsweise auch durch einen Räumlichen Lichtmodulator, Spatial Light Modulator (SLM), dargestellt sein, dessen Brechkraft vorbestimmt variabel eingestellt werden kann.
Um vorzugsweise mehrere Objektpunkte lateral im Objektvolumen gleichzeitig auslesen zu können, ist die Zonenlinse vorzugsweise in der Fourier-Ebene des Fokussier-Objektivs angeordnet, wo sich zumindest näherungsweise auch die Pupille des optischen Fokussier-Systems befindet, wodurch sich im Objektvolumen ein telezentrischer Strahlengang und damit ein von der Wellenlänge unabhängiger Abbildungsmaßstab ergibt. Ein zumindest näherungsweise konstanter Äbbildungsmaßstab ist für das Erfassen eines räumlichen Objekts von Vorteil.
Weiterhin ist es aber auch möglich, daß die diffraktiv-optische Zonenlinse zum Zweck des Durchführens eines Lateral-Scans, wodurch eine dreidimensionale Abtastung ermöglicht wird, sehr schnell dezentriert wird. Dabei ist der laterale Abbildungsmaßstab jedoch wellenlängenabhängig, da die bei einer Dezentrierung erfolgende Bündelablenkung wellenlängenabhängig ist. Jedoch kann dies numerisch korrigiert werden, auch wenn der Rechenaufwand nicht unerheblich ist. Diese Dezentrierung der Zonenlinse kann bei Verwendung eines DOEs mechanisch erfolgen oder beim Einsatz eines elektronischen SLMs auch durch eine Rechner-Steuerung.
Die für die Interferenz jeweils benötigte kohärente Referenzwelle wird vorzugsweise mittels des Referenzstrahlengangs eines fasergekoppelten Michelson-Interferometers, fasergekoppelten Linnik-Interferometers oder eines fasergekoppelten Mach-Zehnder-Interferometers gewonnen.

Dabei sind vorzugsweise im Referenzstrahlengang den genannten Zweistrahl-Interferometern dem Faserende ein miniaturisierter Kollimator und ein miniaturisierter Retroreflektor nachgeordnet. So kann in einem ersten Fall durch einmaliges Verschieben des Retroreflektors bei der Justierung des Abtastkopfes der benötigte optische Gangunterschied im Interferometer abgestimmt werden. Dieser Retroreflektor ist vorzugsweise als miniaturisierter, hier in der Regel glasloser Tripelspiegelreflektor ausgebildet. Der Kollimator muß gut achromatisiert sein. In diesem Fall kann das gesamte Objektvolumen gleichzeitig detektiert werden, da aus dem Referenzstrahlengang interferenzfähiges Licht aller verwendeten Wellenlängen geliefert wird.

Es kann beim Einsatz eines Mach-Zehnder-Interferometers nach dem ersten Y-Koppler Licht aus der Faser ausgekoppelt und kollimiert werden, in einen Retroreflektor seitlich versetzt zur Hauptachse eintreten, parallel versetzt wieder austreten und mittels Fokussier-Objektivs in eine weitere Faser gelangen, die dann zum zweiten Y-Koppler führt. Der Retroreflektor kann als Tripelreflektor ausgebildet sein. Durch Parallelverschieben des Retroreflektors kann eine Änderung des Gangunterschieds erfolgen, so daß der optische Gangunterschied im Mach-Zehnder-Interferometer in weiten Grenzen einstellbar ist und auch ein Wert nahe dem optischen Gangunterschied null einstellbar ist. Dieser vergleichsweise geringe Gangunterschied wird benötigt, damit die Frequenz der Wavelets nicht so hoch ist, was die Auswertung unmöglich machen oder technisch doch sehr erschweren würde. Diese hier beschriebene Anordnung kann beim Mach-Zehnder-Interferometer sowohl im Referenzarm als auch im Objektarm angeordnet sein, wobei der Referenzarm dafür bevorzugt werden kann.

Zur Erzielung von Speckeln mit hohem Interferenzkontrast wird ein Lichtbündel deutlich geringerer Apertur als die Apertur der Beobachtung koaxial in den Strahlengang zur Beobachtung eingekoppelt s. a. [1], S. 335-357. Die chromatisch-konfokale Diskriminierung erfolgt vorzugsweise nur im Beobachtungs- bzw. Objektstrahlengang am Faserende zur Wiedereinkopplung. Diese Faser führt dann zum zweiten Y- Koppler zur Vereinigung und zum fasergekoppelten Spektrometer mit hochempfindlicher Fotodiodenzeile. Die Einkopplung des Beleuchtungsbündels in den Strahlengang zur Beleuchtung erfolgt vorzugsweise mittels flächenhaften Strahlteilers, der die gesamte Pupillenfläche überdeckt. Es sind jedoch grundsätzlich auch andere Wirkprinzipien einsetzbar, welche den optischen Gangunterschied im Referenzarm oder auch im Objektarm des Interferometers verändern können.

Reflektierende oder lichtstreuende Bereiche oder Objektdetails im Objektvolumen liefern also auswertbare Wavelets. Ausgewertet wird also die Signalamplitude des Wavelets oder ein Wert, der sich aus der Signalamplitude ableitet. Dabei wird jedoch nur Licht, bzw. elektromagnetische Strahlung ausgewertet, das oder die einen Fokus auf einem Objektpunkt bildet, der sich jeweils im wellenoptischen Schärfentiefebereich, der symmetrisch einen Objektpunkt umgibt, befindet. Licht von Wellenlängen, das einen Fokus bildet, der mehr als die wellenoptische Schärfentiefe von einem Objektpunkt entfernt liegt, wird von der Verarbeitung durch konfokale Diskriminierung ausgeschlossen.

Dabei kann durch die Verrechnung von fünf Intensitäten $I_1$ bis $I_5$ aus direkt benachbarten Sensorelementen, die eine Phasendifferenz von zumindest näherungsweise 90° zueinander aufweisen zum Beispiel die folgende Gleichung zur Bestimmung der Signalamplitude A, verwendet werden:

$$A = \sqrt{(I_1 - 2I_3 + I_5)^2 + (2I_2 - 2I_4)^2} \,. \qquad (1)$$

Andere Gleichungen zur Bestimmung der Signalamplitude A sind bekannt und ebenfalls anwendbar. Diese Berechnung der Signalamplitude A kann mittels Hardware-Prozessoren, die parallel auf die Sensorelemente zugreifen, extrem schnell ausgeführt werden.

Die Signalamplitude kann jedoch auch über eine größere Anzahl von Intensitäten aus direkt benachbarten Sensorelementen mittels FFT errechnet werden, wobei die FFT vorzugsweise stückweise über dem Spektralbereich ausgeführt wird, nämlich dort, wo ein Wavelet erwartet wird und das auf der Spektrometerzeile ausgewertete Stück vorzugsweise maximal etwa dem halben Abstand zwischen zwei Wavelets entspricht. Auch Sub-Nyquist-Verfahren können gegebenenfalls zur Berechnung der Signalamplitude angewendet werden.

Der Ort des Maximums der Einhüllenden auf der Wellenlängenachse wird über eine Bestimmung des Schwerpunktes des Wavelets ermittelt, um so die Tiefeninformation über den ausgewerteten Objektpunkt zu erhalten. Dabei kann eine Kalibrierung notwendig sein.

Es ist vorzugsweise auch möglich, daß die jeweils benötigte kohärente Referenzwelle aus einer Wellenfront, die beim Durchgang oder der Reflexion in der nullten Beugungsordnung an der diffraktiv-optischen Zonenlinse entsteht, gebildet wird. Damit kann ein Common-path-Strahlengang erreicht werden. Dabei ist dieser Zonenlinse eine zumindest teilweise reflektierende Referenzspiegelfläche im Common-path-Strahlengang zugeordnet, die zur Reflexion der zumindest näherungsweise gut fokussierten Referenzwellen aller Wellenlängen dient. Der Referenzstrahlengang sollte möglichst gut achromatisch ausgebildet sein. Das bedeutet, daß auch der refraktive Teil des Fokussier-Systems, über welchen ja auch das Referenzbündel abgebildet wird, vorzugsweise gut achromatisiert sein muß. Dieser Ansatz ist sehr kompakt und volumensparend, ist jedoch zunächst für ein eher wenig tiefes Objektvolumen wegen der Probleme mit der Eliminierung der sphärischen Aberration im Common-path-Strahlengang geeignet. Beispielsweise kann hierbei ein Optimum für ein nur 200 μm tiefes Objektvolumen und eine numerische Apertur von etwa 0,5 gegeben sein.

Bei Ausbildung eines diffraktiv-optischen Systems mit einer, zwei oder mehr Zonenlinsen zur chromatischen Tiefenaufspaltung in einer vergleichsweise großen Pupille des Fokussier-Systems, die vorzugsweise in der Fourier-Ebene desselben angeordnet ist, kann auch eine höhere numerische Apertur als 0,5 für das Fokussier-System erreicht werden. In diesem Fall muß jedoch mittels diffraktiv-optischen Systems in der Pupille für den chromatischen Strahlengang eine hinreichend gute Korrektur der sphärischen Aberration erfolgen - und dies für den gesamten genutzten Spektralbereich.

Zur Eliminierung der tiefenabhängigen sphärischen Aberration wird eine vorbestimmte wellenlängenabhängige Wellenfrontformung mittels Diffraktion oder Refraktion und Dispersion oder einer Kombination derselben durchgeführt. Dazu kommen also diffraktive oder refraktive optische Elemente oder eine Kombination derselben im chromatisch längsaufspaltenden Fokussier-System zum Einsatz.

Dabei bilden sich durch die vorbestimmt eingeführte chromatische Längsaberration, beziehungsweise die chromatische Tiefenaufspaltung, die Foki in unterschiedlichen Tiefen des Objektvolumens aus, wobei für jeden Fokus jeder beliebigen Wellenlänge im genutzten Spektralbereich, die sphärische Aberration vorzugsweise durch eine vorbestimmte Einstellung im Fokussier-System ein Minimum aufweist, so daß für den Fokusfleck in jeder vom Design des Abtastsystems berücksichtigten Tiefe im Objektvolumen in guter Näherung eine beugungsbegrenzte Abbildung besteht.

Es ist aber auch möglich, daß die sphärische Aberrations-Korrektur an verschiedene Tiefen elektronisch gesteuert anpaßbar gemacht ist, damit verschiedene Tiefen des Objekts auch mit Licht verschiedener Wellenlängen erfaßbar ist. Dies kann wegen einer gegebenenfalls hohen Abhängigkeit der Transparenz des Objekts von der Wellenlänge, sehr sinnvoll sein. Dies führt zu einem Maximum an Adaptivität. Diese Adaption wird jedoch nicht im Meßvorgang, sondern vor Beginn der Messung durchgeführt. Dies bedeutet, daß der Tastkopf und das Verfahren für eine spezielle Aufgabe einmalig konditioniert werden. Dies kann erreicht werden, indem auch die Größe der chromatischen Tiefenaufspaltung im chromatisch-konfokalen System vorzugsweise elektronisch steuerbar durch die vorzugsweise Anwendung von SLMs gemacht ist.

Weiterhin wird im optischen System mittels bündelbegrenzender Mittel stets eine konfokale Diskriminierung für die aus dem Objektvolumen kommenden Wellenfronten durchgeführt. Das kann durch das Ende einer Monomode-Faser oder durch Mikroblenden geschehen.

Bei einem hochaperturigen Fokussier-Objektiv, beispielsweise mit einer numerischen Apertur von 0,7 und einer besonders großen Tiefe eines eher kooperativen Objektvolumens, kann die sich stark ändernde Periodizität über der Wellenlänge in den Wavelets ein erhebliches Problem für die Detektion darstellen.

Deshalb werden vorzugsweise bei dem Verfahren zur konfokalen Spektral-Interferometrie, insbesondere auch zur Erfassung des Abstandes, des Profils und der Form und zur Optischen Kohärenz-Tomografie (OCT) sowie auch zur dreidimensionalen Optischen Kohärenz-Mikroskopie (OCM) von biologischen und technischen Objekten, Wavelets mit Frequenzanpassung erzeugt, indem in mindestens einem Arm des Interferometers durch Dispersion und/oder Diffraktion der optische Gangunterschied vorbestimmt wellenlängenabhängig gemacht ist. Dies ist von großem Vorteil für eine schnelle Auswertung der Wavelets auf der Spektrometerachse, insbesondere dann, wenn ein eher tief ausgedehntes Volumen auszuwerten ist. Bei einem tief ausgedehnten Volumen ändert sich im Interferometer der optische Gangunterschied über der Wellenlänge vergleichsweise stark. Beispielsweise, wenn es sich um einen Tiefenbereich von mehr als 200 μm handelt. Dies kann zu einer unerwünscht großen Variation der Frequenz der Wavelets führen. Die Spektrometerzeile müßte dann Sensorelemente mit variablem Abstand aufweisen oder es wird eine sehr große Anzahl von Sensorelementen benötigt, die wiederum eine extrem schnelle Auswertung aufwändiger machen. Die Abhängigkeit des optischen Gangunterschieds im Interferometer von der Wellenlängen wird deshalb so eingestellt, daß diese somit eine sich insgesamt wenig variierende Variation der Frequenz der Wavelets auf der Spektrometerachse, beziehungsweise Spektrometerzeile ergibt. Beispielsweise werden vorzugsweise so jeder Periode unter der Einhüllenden des Wavelets, 3 bis 6 Sensorelemente zugeordnet. Bei Sub-Nyquist-Auswertungen und optimierter Abhängigkeit des optischen Gangunterschieds im Interferometer von der Wellenlängen können auch weniger Sensorelemente eingesetzt werden, die dann jedoch einen Füllfaktor in Richtung der Spektrometerachse von beispielsweise höchstens 50% aufweisen, um eine hohe Signalmodulation zu erreichen. Dies bedeutet aber eine nicht effiziente Nutzung der eingesetzten Lichtenergie.

Es werden also beim Verfahren zur konfokalen Spektral-Interferometrie, insbesondere auch zur Erfassung des Abstandes, des Profils und der Form und zur Optischen Kohärenz-Tomografie (OCT) sowie auch zur dreidimensionalen Optischen Kohärenz-Mikroskopie (OCM) von biologischen Objekten vorzugsweise Wavelets mit Frequenzanpassung erzeugt. Dies bedeutet: Für Licht unterschiedlicher Wellenlängen gibt es einen unterschiedlichen Gangunterschied im Interferometer. Dazu ist mindestens in einem Arm des Interferometers der optische Gangunterschied wellenlängenabhängig gemacht. Vorzugsweise erfolgt dies im Referenzarm des Interferometers. Diese Anpassung der Frequenz der Wavelets über der Wellenlänge, also auf der Spektrometerzeile, erleichtert die extrem schnelle Erkennung und Auswertung der Wavelets. Ziel ist hierbei eine möglichst geringe Variation der Frequenz der Wavelets. Dieser Ansatz kann somit ein axiales Nachstellen des Retroreflektors beim Wechsel des zu erfassenden Tiefenbereiches vollständig überflüssig machen.

Die Variation des optischen Gangunterschieds über der Wellenlänge kann im Interferometer mittels Dispersion, aber auch mittels Diffraktion oder mittels einer Kombination von beiden in einem oder auch in beiden Interferometerarmen erzeugt werden. Dieses kann gleichzeitig in beiden Interferometerarmen oder auch nur in jeweils einem verursacht werden, wobei auch das optische Abbildungssystem bereits etwas Abhängigkeit des optischen Gangunterschieds von der Wellenlänge einbringen kann, jedoch in der Regel in eher geringer Größe. Die Größe der Diffraktion kann durch Einsatz von SLMs vorzugsweise elektronisch gesteuert werden. Es kann aber auch der von der Wellenlänge abhängige optische Gangunterschied im Interferometer durch die Variation der Dicke einer optischen Platte elektronisch gesteuert werden, da zwei Prismen elektronisch gesteuert gegeneinander verschoben werden können.

Weiterhin ist es auch vorzugsweise möglich, daß durch eine vorbestimmte Variation des optischen Gangunterschieds über der Wellenlänge im umgekehrten Sinne wie vorab beschrieben eine charakteristische und gut auswertbare Verschiebung der Mittenfrequenz der Wavelets ergibt. Diese Änderung der Mittenfrequenz der Wavelets kann bereits bei zwei benachbarten Wavelets festgestellt werden. Diese Änderung der Mittenfrequenz kann vorzugsweise mittels FFT ausgewertet werden. Dabei wird vorzugsweise eine - durch die vorbestimmte Variation des optischen Gangunterschieds über der Wellenlänge gezielt mitinduzierte - stetig fallende oder stetig steigende Mittenfrequenz der Wavelets erzeugt. Diese Variation des optischen Gangunterschieds beruht auf der Dispersion und/oder Diffraktion in mindestens einem der beiden Arme des Interferometers. Da die Frequenzvariation bei effizienter Nutzung der Sensorelemente wegen des Abtast-Theorems in der Regel nur in einem eher begrenzten Rahmen möglich ist, es sei denn bei Anwendung von Sub-Nyquist-Kriterien, wird dieser Ansatz eher für ein begrenzt tiefes Objektvolumen und damit auszuwertenden Wavelets eingesetzt werden können.

Darüber hinaus können mit der schnellen Bestimmung der mittleren Frequenz der Wavelets, beispielsweise mittels FFT-Prozessoren, auch Trends im Gesamtsystem, beispielsweise Abstandsänderungen der Fokussieroptik zum Objekt durch Stöße sehr gut erkannt und damit auch zumindest teilweise korrigiert werden.

Bei dem Verfahren zur konfokalen Spektral-Interferometrie wird vorzugsweise eine Phasenschiebung im Interferometer erzeugt.

Diese Phasenschiebung ist dabei vorzugsweise achromatisch. In jedem Sensorelement des Spektrometers kann so mit wenigen achromatischen Phasenschiebungen die Amplitude und Phase des spektral aufgespalteten Interferenzsignals mittels der bekannten Gleichungen für die phasenschiebende Interferometrie vergleichsweise genau ermittelt werden. Dabei werden mindestens zwei achromatische Phasenschiebungen, beispielsweise von 120 Altgrad (2/3 p), durchgeführt, so daß sich drei Intensitätswerte pro Sensorelement ergeben. Die Auswertung der Intensitätswerte führt zu mehr Informationen über das Objekt, da die spektrale Auflösung erhöht wird. Das Signal-Rausch-Verhältnis wird ebenfalls verbessert. So kann bei sehr schwachen Signalen eine sinnvolle Verbesserung der Meßgenauigkeit erreicht werden. Vorzugsweise wird dabei die achromatische Phasenschiebung mehrfach erzeugt, beispielsweise auch viermal zu je 90 Altgrad, entsprechend p/2, so daß fünf Intensitätswerte zur Verfügung stehen. Nach jeder achromatischen Phasenschiebung erfolgt mindestens eine weitere Aufnahme des Spektrums. Der Vorteil der mehrfachen, achromatischen Phasenschiebung ist hierbei letztlich die Erhöhung der Sensitivität des Verfahrens und die Erhöhung der spektralen Auflösung für den gesamten Spektralbereich mit.

Wird bei dem Verfahren zur konfokalen Spektral-Interferometrie nach einer Messung eines Objektdetails vorzugsweise eine einfache oder mehrfache Veränderung des Abstandes des Abtastkopfes zum Objekt durchgeführt und nach der Veränderung erneut gemessen, ist auch für die optische Tomografie mit dem chromatisch-konfokalen Ansatz ein Verfahren gegeben, welches Informationen über die Änderung von Absorption, Rückstreuung und Reflexion sowohl über der Tiefe als auch der Wellenlänge des Objektdetails, also spektrale Informationen, gewinnen läßt. Es können so für die Absorption, Rückstreuung und Reflexion die spektralen Abhängigkeiten zumindest näherungsweise ermittelt werden. So kann erreicht werden, daß für jede Tiefe oder für jedes Objektdetail mehr als Licht nur einer Wellenlänge oder eines eng begrenzten Spektralbereiches zur Anwendung kommt.

Es wird bei einem anderen Abstand des Abtastkopfes zum Objekt jeweils auch ein Wavelet mit einer anderen Schwerpunktwellenlänge detektiert. Dieses Vorgehen kann sehr wesentliche spektrale Informationen über das Objektdetail und somit auch über das gesamte Objekt liefern, wobei die Nutzung eines Modells der räumlich verteilten spektralen Eigenschaften des Objekts sehr nützlich ist. Ein Anwendungsbeispiel dafür kann die Bestimmung des Grades der Durchblutung eines Gewebebereiches bei einem Menschen während einer medizinischen Behandlung sein.

Weiterhin kann bei dem Verfahren zur konfokalen Spektral-Interferometrie auch durch achromatische adaptive Optik im Fokussier-System, beispielsweise durch einen in das Fokussier-System integrierten, vorbestimmt steuerbaren Membranspiegel, das Ensemble der aufgespalteten Foki in der Tiefe einmal oder mehrfach - wie bei der vordem beschriebenen, mechanisch verursachten Abstandsänderung - verschoben werden und nach jeder Verschiebung ein Datensatz aufgenommen und ausgewertet werden. Dabei ist die Krümmung des Membranspiegels vorbestimmt einstellbar gemacht. Auch dies ermöglicht, vergleichbar mit der Änderung des Abstandes des Tastkopfes, die Erfassung von Objektdetails mit unterschiedlichen Schwerpunktwellenlängen im Wavelet. Dadurch wird wie vordem beschrieben eine Aussage über die spektralen Eigenschaften von Objektdetails und des gesamten Objekts möglich.

Darüber hinaus ist bei dem Verfahren zur konfokalen Spektral-Interferometrie vorzugsweise der Grad der chromatischen Tiefenaufspaltung vorbestimmt veränderbar gemacht. Dies erfolgt vorzugsweise elektronisch gesteuert. Damit besteht die Möglichkeit, das Verfahren an ein ausgewähltes Objekt oder Objektdetail optimal hinsichtlich der Tiefenauflösung und der spektralen Analyse anzupassen.

Ein Aspekt der vorliegenden Erfindung betrifft eine Anordnung zur konfokale Spektral-Interferometrie gemäß Anspruch 16.

Erfingdungsgemäß wird dem spektralen Zweistrahl-Interferometer eine chromatisch-konfokale Anordnung im Abbildungsstrahlengang des Fokussier-Systems zur

Objektbeleuchtung und Objektdetektion zugeordnet , und das Interferometer ist als Common-path-Interferometer ausgebildet.

Dabei sind im Referenzstrahlengang keine Komponenten angeordnet, die eine chromatisch-konfokale Tiefenaufspaltung bewirken, denn über den Referenzstrahlengang soll das Licht von allen Wellenlängen des nutzbaren Spektralbereiches für die Interferenz mit dem Objektlicht zur Verfügung gestellt werden. Dagegen überdeckt das Objektlicht, also das aus dem Objektstrahlengang zurückkommende Licht, den nutzbaren Spektralbereich wegen der konfokalen Diskriminierung in der Regel nicht vollständig.

Als Weißlichtquelle wird beispielsweise ein fasergekoppelter Weißlicht-Kontinuumslaser eingesetzt. Auch Weißlicht-Laser mit einer spektralen Kamm-Charakteristik sind einsetzbar, wenn nur hinreichend viele Emissions-Linien im genutzten Spektrum vorhanden sind. Es können aber auch eine oder mehrere fasergekoppelte Superlumineszenz-LEDs (SLDs) zur Anwendung kommen.

Die Halbwertsbreite des konfokalen Signals FWHM folgt bekannterweise aus der Gleichung (2)

$$FWHM = \frac{0,45\lambda}{n(1-\cos(\alpha))} \tag{2}$$

mit der jeweiligen Wellenlänge 1 und dem Aperturwinkel a der numerischen Apertur sin a des Fokussier-Systems sowie dem Brechungsindex n.

Bei Fokussier-Systemen mit einer hohen numerischen Apertur NA, NA > 0,5, und bei elektromagnetischer Strahlung im sichtbaren- oder nahen infraroten Spektralbereich kann so eine Tiefendiskriminierung von deutlich besser als 10 μm für Objektdetails erreicht werden. Das absolute Minimum kann unter 1 μm liegen, s. a. Gleichung (2). Insbesondere kann im blauen oder violetten Spektralbereich, einschließlich des ultravioletten Spektralbereichs, wegen der kleinen Wellenlänge eine hohe Tiefenauflösung ermöglicht werden. Jedoch muß dies auch stets im Zusammenhang mit der eingesetzten Algorithmik zur Auswertung der optischen Daten und den dabei auftretenden Einflüssen des Umfeldes betrachtet werden.

Dabei muß folgende Bedingung für die Generierung zuverlässig auswertbarer optischer Signale eingehalten sein:

Der Abstand des Fokussier-Systems zur optischen Grenzfläche oder zur Oberfläche des Objektvolumens sowie der Brechungsindex sollten nicht zu stark von den Werten abweichen, welche dem Design des optischen Abtastkopfes zu Grunde liegen. Insbesondere sollte bei einem Brechungsindex n größer 1, also wenn eine Flüssigkeit die Oberfläche des Objektvolumens bedeckt, der optische Weg zumindest näherungsweise den Nennwerten des optischen Abtastkopfes entsprechen. Dies ist wegen der sonst nicht eliminierten sphärischen Aberration ein Problem, da dadurch die Modulation im Signal stark absinken kann. Eine Möglichkeit zur Reduzierung dieser Fehlanpassung stellt hier auch das gesteuerte Verringern der numerischen Apertur des Fokussier-Systems im Anfahrvorgang dar, beispielsweise durch eine den Durchmesser der Pupille des Fokussier-Systems steuernde Blende, welche auch den Durchmesser der Pupille verringern kann.

Das optische Design des Fokussier-Systems ist also vorzugsweise so durchgeführt, daß bei der Ausbildung von Fokusflecken jeder Wellenlänge der für die Auslesung genutzten elektromagnetischen Strahlung im Objektvolumen die sphärische Aberration hinreichend klein gemacht ist, damit in jeder Tiefe des Objektvolumens reflektierte oder gestreute fokussierte elektromagnetische Strahlung eine hinreichend gering aberrierte Wellenfront liefern kann, die dadurch mit einer im Interferometer kohärent erzeugten Referenzwelle zur signaltechnisch hinreichend gut auswertbaren Interferenz gelangen kann.

Bei hinreichend kleiner sphärischer Aberration für alle im Fokussier-System genutzten Wellenlängen kann ein hinreichend gut moduliertes Interferenzsignal auch für alle - jeweils zumindest näherungsweise entlang einer Geraden in der Tiefe des Objektvolumens durch chromatische Tiefenaufspaltung separierten - Fokusflecken, die reflektiert oder gestreut werden, gebildet werden. Die zumindest näherungsweise Eliminierung der sphärischen Aberration für alle genutzten Wellenlängen ermöglicht die Überwindung des bekannten Problems des recht stark begrenzten Tiefenerfassungsbereiches von beispielsweise nur +/- 100 $\mu$m bei hochaperturigen Fokussier-Systemen, z. B. bei einer numerischen Apertur von 0,55 und einer Wellenlänge des Lichts von 780 nm, aufgrund der sphärischen Aberration ohne die Nutzung aktiver Optik wie beispielsweise steuerbare Membranspiegel.

Bei dem Ansatz ist also für jede Wellenlänge die sphärische Aberration durch das Design des chromatisch längsaufspaltenden Fokussier-Systems hinreichend klein gemacht, so daß ein hinreichend großer Tiefenerfassungsbereich erreichbar ist. Dieser kann beispielsweise +/- 300 $\mu$m bei einer numerischen Apertur von 0,7 betragen. Dabei kommen im chromatisch längsaufspaltenden Fokussier-System vorzugsweise diffraktive oder refraktive optische Elemente oder eine Kombination derselben zum Einsatz.

Beim Einsatz einer Weißlicht-Kontinuums-Quelle, beispielsweise ein Weißlicht - Kontinuums-Laser, und einer Sensorzeile mit einer hoher Anzahl von Sensorelementen, deren Sensorelemente parallel und damit sehr schnell ausgelesen werden, kann die Forderung nach der genauen Abstandsposition des Abtastkopfes etwas entschärft werden, da in jedem Fall ein Objektdetail von einem Fokusfleck getroffen wird, auch wenn die Wellenlänge des jeweils zumindest näherungsweise beugungsbegrenzten Fokusflecks für dieses Objektdetail etwas variieren kann. Die Wellenlänge des Fokusflecks ist also bei Änderung des Abstandes zum Objektdetail, durch welche Gründe auch immer, innerhalb bestimmter Grenzen gleitend. Das verbessert die Robustheit der Messung beispielsweise gegenüber Vibrationen erheblich.

Die Auswertung des Interferenzsignals erfolgt vorzugsweise mittels Gitter-Spektrometers mit mindestens einer Sensorzeile für elektromagnetische Strahlung, vorzugsweise einer schnellen Fotodiodenzeile, die im Millisekunden-, Sub-Millisekunden- Mikrosekunden- oder auch im Sub-Mikrosekundenbereich auswertbare Signale liefern kann, sowie sehr hoher Sensitivität. Es kann aber auch ein Linienspektrometer mit einer hochempfindlichen, gekühlten Flächenkamera oder auch mit einer Photomultiplier-Kamera eingesetzt werden. Bei Einsatz eines Linienspektrometers wird das Licht aus dem Objektvolumen entlang einer Linie erfaßt.

Dabei stellt der Zeilensensor, also die spektrale Achse, die $\lambda$-Achse dar, wobei $\lambda$ die Wellenlänge der elektromagnetischen Strahlung darstellt. Der Chip der Fotodiodenzeile kann auch eine Intelligence-on-Chip-Funktionalität aufweisen. Mit einem Parallelzugriff auf die einzelnen Sensorelemente kann so mittels festverdrahteten Prozessoren eine extrem schnelle Auswertung der Spektren, also hier der Wavelets, durchgeführt werden.

Für jede Tiefe eines Objektdetails im Objektvolumen oder an der Oberfläche des Objekts innerhalb des auswertbaren Bereiches des Objektvolumens gibt es also Licht einer genau passenden Wellenlänge, die genau am Objektdetail einen Fokus bildet, so daß sich stets ein zumindest näherungsweise beugungsbegrenzter Fokusfleck ausbilden kann, wobei die Wellenlänge des Lichtes etwas variieren kann. Genau in diesem Wellenlängenbereich entsteht auf diesem Zeilensensor jeweils ein Wavelet, wobei die genau passende Wellenlänge zumindest näherungsweise die Schwerpunktwellenlänge des Wavelets darstellt. Im Minimum genügen drei Sensorelemente geeigneten lateralen Abstandes, um die Existenz eines Wavelet mit zumindest näherungsweise bekannter Frequenz zu erkennen. Besser ist es jedoch, vier bis acht Sensorelemente geeigneten lateralen Abstandes zu verwenden, um die Existenz eines Wavelets mit zumindest näherungsweise bekannter Frequenz sicher zu erkennen. Dabei kann es von Vorteil sein, wenn die Phasendifferenz zwischen zwei - von direkt benachbarten Sensorelementen - abgetasteten Bereichen des Wavelets zumindest näherungsweise 90 Altgrad, entsprechend p/2, beträgt. Bestimmt wird die Signalamplitude und die Position des Wavelets auf der Wellenlängenachse, welche mittels bekannten Zusammenhangs zwischen Wellenlänge und Tiefenposition der Foki die Tiefeninformation liefert.

Bei einer Anordnung zur konfokalen Spektral-Interferometrie mit einem spektralen Zweistrahl-Interferometer, insbesondere auch zur Erfassung des Abstandes, des Profils und der Form und zur dreidimensionalen optischen Kohärenz-Mikroskopie (3D-OCM) von biologischen Objekten kann die diffraktiv-optische Zonenlinse in der Pupille in der abgewandten Brennebene des Fokussier-Objektivs angeordnet sein. Damit ist die numerische Apertur des Lichtes aller Wellenlängen gleich. Es kann die Pupille aber auch für längerwelliges Licht durch ein chromatisches Filter mit radialer Abhängigkeit, auch als chromatische Apodisation bekannt, etwas größer gemacht sein, da so die beugungsbegrenzte laterale Ausdehnung für Foki längerwelligen Lichts der des kürzerweiligen Lichts angeglichen werden kann.

Die diffraktiv-optische Zonenlinse ist vorzugsweise elektronisch steuerbar ausgebildet. Dies kann beispielsweise durch ein Phase-mostly-LCD oder ein Phaseonly-Digital-Micro-Mirror-Device (DMD) geschehen.

Weiterhin ist bei der Anordnung zur konfokalen Spektral-Interferometrie dem Interferometer ein vorzugsweise fasergekoppeltes Zeilen-Spektrometer nachgeordnet.

Weiterhin ist vorzugsweise bei der Anordnung zur konfokalen Spektral-Interferometrie mindestens ein optisches Element im kollimierten Referenzstrahlengang mit dispersivem Material angeordnet. Dies ermöglicht eine Gangunterschiedsanpassung zur Anpassung der Frequenz der Wavelets über der Wellenlänge. Dabei ist das optische Element mit dispersivem Material im kollimierten Referenzstrahlengang vorzugsweise als eine Planparallelplatte ausgebildet.

Weiterhin ist vorzugsweise bei der Anordnung zur konfokalen Spektral-Interferometrie im kollimierten Referenzstrahlengang ein Diffraktionsmodul mit mindestens zwei parallel zueinander positionierten Phasengittem angeordnet.

Bei Anwendung eines Mach-Zehnder-Interferometers kann der optische Gangunterschied sowohl im kollimierten Referenz- als auch im kollimierten Objektstrahlengang mittels mindestens eines verschiebbaren Tripelreflektors, der vorzugsweise außeraxial genutzt wird, eingestellt werden.

Bei Anwendung eines Mach-Zehnder-Interferometers kann auch die Gangunterschiedsvariation über der Wellenlänge sowohl im kollimierten Referenzals auch im kollimierten Objektstrahlengang mittels mindestens des verschiebbaren Tripelreflektors und vier parallel zueinander angeordneten und verschiebbaren Phasengittem, die vorzugsweise in der ersten Beugungsordnung genutzt werden, vorbestimmt eingestellt werden.

Vorteilhaft kann für die CC-SI bei der Applikation für die Detektion des Inneren eines Objekts die Einhaltung folgender Bedingung sein:

Der mittlere optische Gangunterschied im Referenzarm eines Interferometers soll vorzugsweise so eingestellt sein, daß sich - beim Auftreffen eines Bündels mit der Wellenlänge $\lambda + \Delta\lambda$l auf ein streuendes Detail im Objektinneren - eine Änderung der Phase von zumindest näherungsweise p/2 ergibt im Vergleich zu einem Bündel mit der Wellenlänge $\lambda$, welches auf dasselbe streuende Detail trifft, wobei das Licht mit der Wellenlänge $\lambda + \Delta\lambda$l vom benachbarten Sensorelement des Spektrometers detektiert wird.

Es kann die Periode eines Wavelets unter der Einhüllenden bereits mit vier Sensorelementen schon recht gut abgetastet werden und die vorhandene Signalamplitude des Wavelets somit sicher bestimmt werden. Diese Einstellung des optischen Gangunterschieds kann beispielsweise mittels axial verschiebbaren Retroreflektors im Referenzarm des Interferometers erfolgen. Weiterhin wird die Position des Mittenmaximums der Einhüllenden auf der Wellenlängenskala bestimmt, um die Tiefenposition des lichtstreuenden Objektelements bestimmen zu können.

Es kann der Spektralbereich von 500 nm bis ca. 700 nm genutzt werden. Eine Vergrößerung der Tiefe des detektierbaren Volumens im sichtbaren Spektralbereich setzt auch eine Erhöhung der spektralen Auflösung voraus. Dies macht den Einsatz hinreichend starker, vorzugsweise fasergekoppelter Weißlichtquellen und hochempfindlicher Sensorelemente sehr zweckmäßig.

Die Brennweite des Fokussier-Systems ist in weiten Grenzen frei wählbar. Je größer die Brennweite gemacht ist, um so geringer ist die - für einen bestimmten Tiefenbereich - durch die Diffraktion aufzubringende Brechkraft. Grundsätzlich einfacher ist es deshalb, das Fokussier-System mit nicht zu kurzer Brennweite zu gestalten. Das führt jedoch andererseits zu einem großen Durchmesser des Fokussier-Systems und damit großem Bauvolumen des optischen Tastkopfes.

Dem Abtastkopf kann zur Erzeugung einer Relativbewegung des Abtastbündels zum Objekt - wie bei der OCM üblich - ein lateral arbeitender 1D- oder auch 2D-Scanner in Beleuchtungsrichtung zugeordnet sein, der sich dann vorzugsweise mit seinen Spiegeln zumindest näherungsweise in der Fourier-Ebene des Fokussier-Objektivs des Fokussier-Systems oder in einer zu dieser optisch konjugierten Ebene befindet.

Bei der Anordnung zur konfokalen Spektral-Interferometrie sind vorzugsweise zur Eliminierung der tiefenabhängigen sphärischen Aberration durch eine wellenlängenabhängige Wellenfrontformung diffraktive oder refraktive optische Elemente oder eine Kombination derselben im chromatisch tiefenaufspaltenden Fokussier-System angeordnet.

Bei der Anordnung zur konfokalen Spektral-Interferometrie sind vorzugsweise zur Erzeugung einer achromatischen Phasenschiebung diffraktive oder refraktive optische Elemente oder eine Kombination derselben im chromatisch tiefenaufspaltenden Fokussier-System angeordnet.

Weiterhin kann bei der Anordnung zur konfokalen Spektral-Interferometrie nach einer Messung eines Objektdetails vorzugsweise eine Veränderung des Abstandes des Abtastkopfes zum Objekt mit geeigneten mechanischen Mitteln durchgeführt werden. Anschließend erfolgt eine Messung. So können Informationen über die spektrale Änderung von Absorption, Rückstreuung und Reflexion über der Tiefe und der Wellenlänge des Objektdetails gewonnen werden.

Andererseits kann bei der Anordnung zur konfokalen Spektral-Interferometrie dem Fokussier-System eine achromatische adaptive Optik zugeordnet sein. Diese adaptive Optik kann beispielsweise durch einen in das Fokussier-System integrierten, vorbestimmt steuerbaren Membranspiegel das Ensemble der aufgespalteten Foki in der Tiefe einmal oder mehrfach - wie bei einer mechanisch verursachten Abstandsänderung - verschoben werden. Nach jeder Verschiebung kann ein Datensatz aufgenommen und ausgewertet werden. Dabei ist die Krümmung des Membranspiegels vorbestimmt einstellbar gemacht. Auch dies ermöglicht, vergleichbar mit einer Änderung des Abstandes des Tastkopfes, die Erfassung von Objektdetails mit unterschiedlichen Schwerpunktwellenlängen im Wavelet. Dadurch wird wie vordem beschrieben auch eine Aussage über die spektralen Eigenschaften von Objektdetails möglich.

Weiterhin kann bei der Anordnung zur konfokalen Spektral-Interferometrie dem Fokussier-System eine achromatische adaptive Optik zugeordnet sein. Diese ist vorzugsweise als vorbestimmt steuerbarer Wölb- oder Hohlspiegel, jedoch mit hochdynamischer Änderung der lateralen Position seiner optischen Achse, ausgebildet. Der Wölb- oder Hohlspiegel wird also hochdynamisch dezentriert und ist dabei zumindest näherungsweise in der Fourier-Ebene des Fokussier-Objektivs des Fokussier-Systems oder in einer zu dieser Fourier-Ebene optisch konjugierten Ebene angeordnet. So kann ein schneller Lateral-Scan im Objektraum erzeugt werden. Dieser Lateral-Scan kann in X- und in Y-Richtung erfolgen, so daß ein Feld abgetastet werden kann. Dieser Wölb- oder Hohlspiegel kann als vorbestimmt steuerbares, achromatisches Spiegel-Array ausgebildet sein, wobei die laterale Lage der optischen Achse desselben vorbestimmt steuerbar gemacht ist. Die vorbestimmt steuerbare Veränderung der Brechkraft des Wölb- oder Hohlspiegels kann zur Tiefenanpassung der aufgespalteten Foki genutzt werden. So können von einem Objektdetail Wavelets mit unterschiedlicher Schwerpunktwellenlänge gebildet werden, da sich durch die Veränderung der Brechkraft auch die zu einem Objektdetail bestimmter Tiefe gehörende Schwerpunktwellenlänge des Wavelets verschiebt.

Sowohl die gleichzeitige Generierung von mehreren Punktlichtquellen als auch die konfokale Diskriminierung des vom Objekt kommenden Lichts kann durch eine bewegte Mikrolinsenscheibe im konfokalen System erzeugt werden.

Mit der Anordnung zur konfokalen Spektral-Interferometrie besteht die Möglichkeit mittels elektromagnetischer Strahlung im Nahen Infrarotbereich (NIR) auch an elektronischen oder mikromechanischen Systemen, wie MEMS, MOEMS, die von Halbleiterwerkstoffen umgeben und mit Halbleiterwerkstoffen aufgebaut sind, optische Tomografie zu betreiben. Dabei kann zusätzlich auch die äußere Form der Systemkomponenten ermittelt werden.

Mit der Anordnung zur konfokalen Spektral-Interferometrie kann auch die Topografie von Zähnen im Mund eines Menschen beim Zahnarzt zumindest in Teilbereichen hochgenau erfaßt werden, da hierbei durch die Applikation der Interferometrie sowohl eine hohe Sensitivität erreicht werden kann als auch das Streulicht aus dem Zahninneren durch die konfokale, Diskriminierung wirkungsvoll unterdrückt wird.

Außerdem kann bei der Hochpräzisions-Chirurgie mit der Anordnung zur konfokalen Spektral-Interferometrie auch eine Kavität, beispielsweise das Innenohr eines Menschen oder zumindest Bereiche desselben, zumindest teilweise hinsichtlich der Form der Kavität optisch hochgenau vermessen werden. Dies dient beispielsweise der Erhöhung der geometrischen Genauigkeit bei der Roboter-assistierten Implantation von einer oder mehreren Komponenten im Innenohr zur Verbesserung des Hörvermögens. Durch die Anwendung des erfindungsgemäßen Verfahrens kann dabei auch eine Unterscheidung der Art des optisch erfaßten Gewebes vorgenommen werden, beispielsweise zur sicheren Unterscheidung zwischen Weichteil- oder Knochengewebe.

Weitere Anwendungen zur dreidimensionalen Erfassung von Topografien bestehen für die erfindungsgemäße Anordnung zur konfokalen Spektral-Interferometrie aufgrund der hohen Sensitivität und der Vielfalt der gewinnbaren Informationen in der roboterassistierten Hirnchirurgie.

**Patentansprüche**

1. Verfahren zur konfokalen Spektral-Interferometrie, insbesondere auch zur Erfassung des Abstandes, des Profils und der Form und auch zur Optischen Kohärenz-Tomografie und/oder optischen Kohärenz-Mikroskopie von biologischen und technischen Objekten oder Objektdetails in Auf- und/oder Durchlicht, wobei in dem Verfahren verwendet wird:

   entweder mindestens einer Multiwellenlängen-Quelle elektromagnetischer Strahlung (1, 1a), wobei bei Nutzung der Multiwellenlängen-Quelle dem gerasterten Empfänger (111a) ein dispersives Spektrometer (100) vorgeordnet ist,
   - oder mindestens einer Wellenlängen-durchstimmbaren Quelle elektromagnetischer Strahlung
   und mit mikroskopischer Abbildung der Objektoberfläche und/oder des Inneren des Objektsvolumens mittels Fokussier-Systems auf einen gerasterten Empfänger (111a) elektromagnetischer Strahlung zur Detektion der interferierenden elektromagnetischen Strahlung,
   **gekennzeichnet dadurch,**
   **daß** die chromatisch-konfokale Technik mit der spektralen Zweistrahl-Interferometrie verfahrensmäßig verbunden wird, wobei sowohl eine vorbestimmte chromatische Tiefenaufspaltung der elektromagnetischen Strahlung im Fokussier-System erfolgt als auch
   - entweder bei Nutzung der Multiwellenlängen-Quelle eine Spektralanalyse der detektierten interferierenden elektromagnetischen Strahlung mittels dispersivem Spektrometer durchgeführt wird, so daß ein Spektrum zur Verfügung steht,
   - oder bei Nutzung der Wellenlängen-durchstimmbaren Quelle eine Wellenlängendurchstimmung vorbestimmt durchgeführt wird und die interferierende elektromagnetische Strahlung bei der Wellenlängendurchstimmung detektiert wird, so daß über der Zeit ein Spektrum aufgenommen wird,

   und aus dem Spektrum über die Kenntnis der vorbestimmten Tiefenaufspaltung im chromatisch-konfokalen System und die zumindest näherungsweise Kenntnis des Brechungsindexes die z-Position eines jeden Objektdetails bestimmt wird.

2. Verfahren zur konfokalen Spektral-Interferometrie nach Anspruch 1,

EP 1 805 477 B1

**gekennzeichnet dadurch,**

**daß** bei Vorhandenseins eines reflektierenden oder lichtstreuenden Bereiches, also einem Objektdetail, im Objektvolumen (208) im Abtastvorgang mindestens ein Wavelet über der spektralen Achse des Spektrometers (100) erzeugt wird, dessen Halbwertsbreite durch die Dimensionierung der chromatischen Tiefenaufspaltung vorbestimmt ist, und die Anzahl der Perioden unter der Einhüllenden des Wavelets, die durch die Dimensionierung von optischem Gangunterschied und von der Gangunterschiedsänderung über der Wellenlänge vorbestimmt ist, einstellbar gemacht ist.

3.  Verfahren zur konfokalen Spektral-Interferometrie nach mindestens einem der Ansprüche 1 oder 2,
    **gekennzeichnet dadurch, daß** das Verfahren bei einem optischen Gangunterschied Dx ungleich null im Interferometer durchgeführt wird.

4.  Verfahren zur konfokalen Spektral-Interferometrie nach mindestens einem der Ansprüche 1 bis 3,
    **gekennzeichnet dadurch,**
    **daß** sich durch eine chromatische Längsaberration, das heißt chromatische Tiefenaufspaltung, Foki in unterschiedlichen Tiefen des Objektvolumens (208) ausbilden, wobei für jeden Fokus jeder beliebigen Wellenlänge im genutzten Spektralbereich, die sphärische Aberration durch eine vorbestimmte Einstellung im Fokussier-System ein Minimum aufweist, so daß für einen Fokusfleck in jeder berücksichtigten Tiefe im Objektvolumen (208) in guter Näherung eine beugungsbegrenzte Abbildung besteht.

5.  Verfahren zur konfokalen Spektral-Interferometrie nach mindestens einem der Ansprüche 1 bis 4,
    **gekennzeichnet dadurch,**
    **daß** die Signalamplitude von Wavelets ausgewertet wird.

6.  Verfahren zur konfokalen Spektral-Interferometrie nach mindestens einem der Ansprüche 1 bis 5.
    **gekennzeichnet dadurch,**
    **daß** der Schwerpunkt der Einhüllenden von Wavelets ausgewertet wird.

7.  Verfahren zur konfokalen Spektral-Interferometrie nach mindestens einem der Ansprüche 1 bis 6,
    **gekennzeichnet dadurch,**
    **daß** die Größe des Maximums der Einhüllenden des Wavelets ausgewertet wird.

8.  Verfahren zur konfokalen Spektral-Interferometrie nach mindestens einem der Ansprüche 1 bis 7,
    **gekennzeichnet dadurch,**
    **daß** die Phase eines Wavelets ausgewertet wird.

9.  Verfahren nach Anspruch 1, insbesondere ein interferometrisches Multispektral-Verfahren zur hochdynamischen Objekt-Tiefenabtastung oder -Profilerfassung, wobei die zumindest eine Quelle elektromagnetischer Strahlung eine punktförmige Quelle elektromagnetischer Strahlung ist und wobei sich bündelbegrenzende Mittel zumindest näherungsweise in einer optisch konjugierten Ebene der mindestens einen der punktförmigen Multiwellenlängen-Quelle elektromagnetischer Strahlung am Eingang eines Zweistrahl-Interferometers befinden und im Zweistrahl-Interferometer ein Referenzstrahlenbündel und ein Objektstrahlenbündel elektromagnetischer Strahlung erzeugt wird,
    **gekennzeichnet dadurch,**
    **dass** im Zweistrahl-Interferometer ein diffraktiv-optisches Zonenelement angeordnet ist, mittels dessen sowohl elektromagnetische Strahlung in der nullten als auch in der ersten Beugungsordnung oder gegebenenfalls auch einer höheren Beugungsordnung N oder einer tieferen Beugungsordnung als der nullten in Transmission oder in Reflexion erzeugt wird
    und **dass** das Referenzstrahlenbündel des Zweistrahl-Interferometers im Hin- und Rücklauf das diffraktiv-optische Zonenelement stets in der nullten Beugungsordnung passiert und dagegen das Objektstrahlenbündel im Hin- und Rücklauf in Bezug zum Objekt das diffraktiv-optische Zonenelement jeweils in der ersten Beugungsordnung oder gegebenenfalls jeweils einer höheren Beugungsordnung N passiert, wodurch im Objektraum durch Beugung der elektromagnetischen Strahlung eine chromatische - also eine über der Wellenlänge entstehende - Längsaufspaltung des Objektbündels in Foki erfolgt
    und **dass** die elektromagnetische Strahlung zumindest näherungsweise aller Wellenlängen vom Referenzstrahlenbündel R_0_0, das keine Beugung am diffraktiv-optischen Zonenelement erfährt, nach dem Passieren des diffraktiv-optischen Zonenelements die bündelbegrenzenden Mittel zur konfokalen Diskriminierung durchsetzt, während nach dem diffraktiv-optischen Zonenelement für das vom Objekt kommende Objektstrahlenbündel O_1_1 oder O_N_N nur die Anteile die begrenzenden Mittel zur konfokalen Diskriminierung am Interferometerausgang oder im Zwei-

44

strahl-Interferometer passieren, die zumindest näherungsweise scharf auf das Objekt abgebildet waren

und **dass** zwischen konfokal diskriminierten Strahlungsanteilen des Referenz- und des Objektstrahlenbündels zumindest näherungsweise Zweistrahl-Interferenz stattfindet

und **dass** interferierende elektromagnetische Strahlung anschließend zur Analyse in ein Spektrometer oder in ein weiteres Interferometer geführt und auf dem Empfänger elektromagnetischer Strahlung abgebildet wird, wobei eine Empfängerebene sich zumindest näherungsweise in einer optisch konjugierten Ebene zu mindestens einer der punktförmigen Multiwellenlängen-Quellen am Eingang des Zweistrahl-Interferometers befindet

und **dass** aus den mittels Spektrometer spektroskopisch analysiert oder einem weiteren Interferometer interferometrisch analysiert erzeugten optischen Signalen der Abstand oder das Profil des Objekts mittels bekannter Algorithmen zur Wavelet- oder Interferogramm-Analyse bestimmt wird.

10. Verfahren nach Anspruch 9, insbesondere multispektrales interferometrisches Verfahren zur hochdynamischen Objekttiefenabtastung oder -Profilerfassung,
**gekennzeichnet dadurch,**
**dass** das Verfahren mit einem objektabbildenden Fizeau-Interferometer mit einer teildurchlässigen Referenzspiegelfläche und mit einem zu prüfenden Objekt (8) durchgeführt wird,
und **dass** das Fizeau-Interferometer mit einem Abbildungsstrahlengang für das zu prüfende Objekt (8) und einem Punktempfänger, einem gerasterten Zeilenempfänger oder einem gerasterten Flächenempfänger für elektromagnetische Strahlung ausgebildet ist
und **dass** im Hinlauf des Lichtes von der Lichtquelle (1) zum Objekt aus dem Eingangsstrahlenbündel mittels Diffraktion in Reflexion oder Transmission an einem statischen oder dynamischen, diffraktiv-optischen Zonenelement (5, 105) ein gebeugtes Strahlenbündel O_N, N=1, 2, 3...oder -1, -2, -3..., in der ersten oder einer höheren Beugungsordnung N, N=1, 2, 3 oder niedrigeren Beugungsordnung N als der nullten, N=-1, -2, -3 entsteht, wobei das gebeugte Strahlenbündel O_N, N=1, 2, 3...oder -1, -2, -3..., die teildurchlässige Referenzspiegelfläche (7) durchsetzt,
wobei das gebeugte Strahlenbündel O_N nach Fokussierung auf das Objekt (8) im Objektraum chromatisch längs aufgespalten ist, so dass in Abhängigkeit von der Wellenlänge des Lichts eine Reihe oder eine Vielzahl von Reihen von Fokuspunkten in der Tiefe ausgebildet wird,
und **dass** nach Reflexion des Teilstrahlenbündels O_N am Objekt (8) ein Objekt-Teilstrahlenbündel vom rückreflektierten Strahlenbündel durch Beugung am diffraktiv-optischen Zonenelement in derselben Ordnung wie im Hinlauf, nämlich O_N_N, N=1, 2, 3... oder -1, -2, -3..., gebildet wird, so dass das Objektteilstrahlenbündel O_N_N nach dem Passieren des diffraktiv-optischen Zonenelements (5) wieder im Strahlengang zurückläuft, und dass aus dem Eingangsstrahlenbündel im Hinlauf auf die Referenzspiegelfläche (7) auch noch ein Teilstrahlenbündel, welches im Weiteren als Referenzstrahlenbündel R_0 dient, durch Beugung in der nullten Ordnung am diffraktiv-optischen Zonenelement (5, 105) gebildet wird und im Rücklauf von der Referenzspiegelfläche (7a), auf welche das Referenzstrahlenbündel R_0 fokussiert wird,
durch Beugung auch genau wieder in der nullten Ordnung am diffraktiv-optischen Zonenelement (5, 105) ein Strahlenbündel R_0_0 gebildet wird, so dass das Referenzteilstrahtenbündel R_0_0 nach dem Passieren des diffraktiv-optischen Zonenelements wieder im Strahlengang zurückläuft,
und **dass** zwischen dem Objekt-Teilstrahlenbündel O_N_N und dem Referenzstrahlenbündel R_0_0 der optische Gangunterschied ungleich null gemacht ist, wenn auf einen Objektpunkt fokussiert ist,
und **dass** das Objekt-Teilstrahlenbündel O_N_N und das Referenzstrahlenbündel R_0_0 nach dem diffraktiv-optischen Zonenelement (5, 105) parallel verlaufen und zur Interferenz kommen und anschließend die beiden miteinander interferierenden Teilstrahlenbündel in den Detektionsstrahlengang eingekoppelt werden.

11. Verfahren nach Anspruch 9, insbesondere multispektrales interferometrisches Verfahren zur hochdynamischen Objekttiefenabtastung oder -Profilerfassung,
**gekennzeichnet dadurch,**
**dass** das multispektrale interferometrische Verfahren zur hochdynamischen Objekttiefenabtastung und -profilmessung mit einem Mirau-Interferometer ausgeführt wird.

12. Verfahren nach Anspruch 9, insbesondere multispektrales interferometrisches Verfahren zur hochdynamischen Objekttiefenabtastung oder -Profilerfassung,
**gekennzeichnet dadurch,**
**dass** das multispektrale interferometrische Verfahren zur hochdynamischen Objekttiefenabtastung und -profilmessung mit einem Michelson-Interferometer ausgeführt wird.

13. Verfahren nach einem der Ansprüche 9 bis 12, insbesondere multispektrales interferometrisches Verfahren zur hochdynamischen Objekttiefenabtastung oder - Profilerfassung,

**gekennzeichnet dadurch,**

**dass** eine konfokale Diskriminierung des Lichts der interferierenden Teilstrahlenbündel durchgeführt wird und die konfokal diskriminierten interferierenden Bündel O_N_N und R_0_0 zur spektroskopischen Analyse eine Lateralaufspaltung mittels Dispersion oder Diffraktion erfahren und eine FFT des Intensitätsverlaufs erfolgt, um aus dem sich mittels FFT ergebenden Wertes nach Kalibrierung die Tiefenposition eines Objektpunktes zu bestimmen,

oder dass die interferierenden Bündel O_N_N und R_0_0 eine weitere Interferenzbildung mittels scannendem Zweistrahl-Interferometer (AZI) zur optischen Analyse, also mit zeitlichem Variieren des optischen Gangunterschieds, erfahren und der im scannenden Zweistrahl-Interferometer beim Scan durchfahrene optische Gangunterschied mindestens einmal genau auch dem im Interferometer zwischen Objekt-Teilstrahlenbündel O_N_N und Referenzstrahlenbündel R_0_0 bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger (11, 111) über der Zeit detektiert wird

oder dass die interferierenden Bündel O_N_N und R_0_0 eine weitere Interferenzbildung mittels statischem Zweistrahl-Interferometer zur optischen Analyse, erfahren

also einem Zweistrahl-Interferometer mit lateraler Variation des optischen Gangunterschieds, wobei die Orte unterschiedlichen Gangunterschieds Pixeln einer Zeilen- oder Flächenkamera (11, 111) zugeordnet sind,

und **dass** der im Zweistrahl-Interferometer auftretende optische Gangunterschied dem im Interferometer zwischen Objekt-Teilstrahlenbündel O_N_N und Referenzstrahlenbündel R_0_0 bestehenden optischen Gangunterschied in mindestens einem Element der Zeilen- oder Flächenkamera (11, 111) zumindest näherungsweise entspricht

oder dass im Fall der Anwendung einer Wellenlängen-durchstimmbaren Quelle elektromagnetischer Strahlung beim Scan der Wellenlänge ein optisches moduliertes periodisches Signal erzeugt wird und mittels Punktempfänger, gerastertem Zeilenempfänger oder gerastertem Flächenempfänger (11, 111) über der Zeit detektiert wird und dieses über der Wellenlänge ausgewertet wird.

14. Verfahren nach Anspruch 1, insbesondere ein multispektrales interferometrisches Verfahren zur hochdynamischen Objekttiefenabtastung und -Profilerfassung, mit einem objektabbildenden Mirau-Interferometer mit einem internen Strahlteiler, einer internen Referenzspiegelfläche (15) und mit einem zu prüfenden Objekt (8),

wobei dem Mirau-Interferometer eine spektral breitbandige oder Wellenlängen-durchstimmbare Quelle elektromagnetischer Strahlung (als bevorzugte Multiwellenlängen-Quelle elektromagnetischer Strahlung bzw. als bevorzugte Wellenlängen-durchstimmbare Quelle elektromagnetischer Strahlung) vorgeordnet ist, im Weiteren als Lichtquelle (1) bezeichnet, welche ein Eingangsstrahlenbündel für das Mirau-Interferometer erzeugt,

also das Mirau-Interferometer mit einem Abbildungsstrahlengang für das zu prüfende Objekt (8) und mindestens einem einzigen Punktempfänge bzw. einem gerastertem Zeilenempfänger oder einem gerastertem Flächenempfänger (11, 111) (als bevorzugten gerasterten Empfänger) für elektromagnetische Strahlung ausgebildet ist,

**gekennzeichnet dadurch,**

**dass** im Hinlauf des Lichtes auf dem Weg zum Objekt (8) nach der letzten Linse des Mirau-Interferometers nach dem Strahlteiler des Mirau-Interferometers aus dem diesen Strahlteiler passierenden Teilstrahlenbündel mittels Diffraktion in Transmission an einer statischen oder dynamischen, diffraktiv-optischen Zonenlinse (5a) ein gebeugtes Strahlenbündel O_N, N=1, 2, 3... oder -1, -2, -3..., in der ersten oder einer höheren Beugungsordnung N, N=1, 2, 3, oder einer niedrigeren Beugungsordnung N als der nullten, N=-1, -2, -3, N entsteht,

welches nach Fokussierung auf das Objekt (8) im Objektraum chromatisch längs aufgespalten ist, so dass in Abhängigkeit von der Wellenlänge des Lichts eine Reihe oder eine Vielzahl von Reihen von Fokuspunkten in der Tiefe ausgebildet wird,

und nach Reflexion des Teilstrahlenbündels O_N am Objekt (8) ein Objekt-Teilstrahlenbündel vom rückreflektierten Strahlenbündel durch Beugung an der diffraktiv-optischen Zonenlinse (5a) in derselben Ordnung wie im Hinlauf, nämlich O_N_N gebildet wird, so dass das Objektteilstrahlenbündel O_N_N nach dem Passieren der diffraktiv-optischen Zonenlinse (5a) wieder im Strahlengang zurückläuft,

und aus dem Eingangsstrahlenbündel im Hinlauf auf die Fläche des Strahlteilers, dessen Strahlteilerfläche (7) parallel zu der diffraktiv-optischen Zonenlinse (5a) liegt, auch noch ein Teilstrahlenbündel,

welches im Weiteren als Referenzstrahlenbündel R dient,

gebildet wird und im Rücklauf von der Referenzspiegelfläche (15), auf welche das Referenzstrahlenbündel R fokussiert wird,

und **dass** das Referenzteilstrahlenbündel R nach dem Passieren des Strahlteilers in Reflexion wieder im Strahlengang zurückläuft,

und zwischen dem Objekt-Teilstrahlenbündel O_N_N und dem Referenzstrahlenbündel R der optische Gangunterschied ungleich null gemacht ist, wenn auf einen Objektpunkt fokussiert ist,

und **dass** das Objekt-Teilstrahlenbündel O_N_N und das Referenzstrahlenbündel R nach der diffraktiv-optischen Zonenlinse (5a) parallel verlaufen und zur Interferenz kommen und anschließend mittels Teilung der Amplitude der Wellenfronten die beiden miteinander interferierenden Teilstrahlenbündel in den Detektionsstrahlengang eingekoppelt werden

und eine konfokale Diskriminierung des Lichts der interferierenden Teilstrahlenbündel durchgeführt wird und die interferierenden Bündel Objekt-Teilstrahlenbündel O_N_N und Referenzstrahlenbündel R zur spektroskopischen Analyse eine Lateralaufspaltung mittels Dispersion oder Diffraktion erfahren und eine FFT des Intensitätsverlaufs durchgeführt wird, um aus dem sich mittels FFT ergebenden Wertes nach Kalibrierung die Tiefenposition eines Objektpunktes zu bestimmen,

oder dass die interferierenden Bündel Objekt-Teilstrahlenbündel O_N_N und R eine weitere Interferenzbildung mittels scannendem Zweistrahl-Interferometer, also mit zeitlichem Variieren des optischen Gangunterschieds zur optischen Analyse, erfahren und der im scannenden Zweistrahl-Interferometer beim Scan durchfahrene optische Gangunterschied mindestens einmal genau auch dem im Interferometer zwischen Objekt-Teilstrahlenbündel O_N_N und Referenzstrahlenbündel R bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird

oder dass die interferierenden Bündel O_N_N und R eine weitere Interferenzbildung mittels statischem Zweistrahl-Interferometer zur optischen Analyse, erfahren

also einem Zweistrahl-Interferometer, mit lateraler Variation des optischen Gangunterschieds, wobei die Orte unterschiedlichen Gangunterschieds Pixeln einer Zeilen- oder Flächenkamera zugeordnet sind

und **dass** der im Zweistrahl-Interferometer auftretende optische Gangunterschied dem im Mirau-Interferometer zwischen Objekt-Teilstrahlenbündel O_N_N und Referenzstrahlenbündel R bestehenden optischen Gangunterschied in mindestens einem Element der Zeilen- oder Flächenkamera zumindest näherungsweise entspricht

oder dass im Fall der Anwendung einer Wellenlängen-durchstimmbaren Quelle elektromagnetischer Strahlung beim Scan der Wellenlänge ein moduliertes periodisches optisches Signal erzeugt wird und mittels Punktempfänger über der Zeit detektiert wird und dieses über der Wellenlänge ausgewertet wird.

15. Verfahren nach Anspruch 1, insbesondere ein multispektrales interferometrisches Verfahren zur hochdynamischen Objekttiefenabtastung oder -Profilerfassung, mit einem fasergekoppelten Michelson-Interferometer mit einem Strahlteiler, der als x-Koppler (33) ausgebildet ist, und mit einem Referenzspiegel (15a) oder mit einer Referenzspiegelfläche und mit einem zu prüfenden Objekt (8),

wobei dem fasergekoppelten Michelson-Interferometer eine spektral breitbandige oder Wellenlängen-durchstimmbare Quelle elektromagnetischer Strahlung (als bevorzugte Multiwellenlängen-Quelle elektromagnetischer Strahlung bzw. als bevorzugte Wellenlängen-durchstimmbare Quelle elektromagnetischer Strahlung) vorgeordnet ist, im Weiteren als Lichtquelle (1) bezeichnet, welche ein Eingangsstrahlenbündel für das Michelson-Interferometer erzeugt,

also das fasergekoppelte Michelson-Interferometer im Objektstrahlengang mit einem Abbildungsstrahlengang für das zu prüfende Objekt (8) und mindestens einem einzigen Punktempfänger, einem gerastertem Zeilenempfänger oder einem gerastertem Flächenempfänger (11, 111) für elektromagnetische Strahlung (als bevorzugten gerasterten Empfänger für elektromagnetische ) ausgebildet ist,

**gekennzeichnet dadurch,**

**dass** im Hinlauf des Lichtes auf dem Weg zum Objekt nach dem x-Koppler (33) des Michelson-Interferometers mittels Diffraktion in Transmission an einer statischen oder einer dynamischen, diffraktiv-optischen Zonenlinse (5) ein gebeugtes Strahlenbündel O_N, N=1, 2, 3... oder -1, -2, -3..., in der ersten oder einer höheren Beugungsordnung N, N=1, 2, 3, oder niedrigeren Beugungsordnung N als der nullten, N=-1, -2, -3, entsteht und dieses gebeugte Strahlenbündel O_N, nach Fokussierung auf das Objekt (8) im Objektraum chromatisch längs aufgespalten ist, und nach Reflexion des Teilstrahlenbündels O_N am Objekt (8) ein Objekt-Teilstrahlenbündel vom rückreflektierten Strahlenbündel durch Beugung an der diffraktiv-optischen Zonenlinse in derselben Ordnung wie im Hinlauf, nämlich O_N_N gebildet wird, so dass das Objektteilstrahlenbündel O_N_N nach dem Passieren der diffraktiv-optischen Zonenlinse (5) wieder im Strahlengang zurückläuft, konfokal mittels Faserende diskriminiert wird, anschließend den x-Koppler (33) passiert und in eine Auskoppelfaser (9) gelangt

und **dass** das Referenzbündel R im Hinlauf durch Teilung am x-Koppler (33) entsteht und am Ende der Faser direkt an einer Referenzspiegelfläche (7b) oder am Ende einer der Faser nachgeordneten Fokussieroptik an einer Referenzspiegelfläche reflektiert wird

und **dass** das in der Faser zurücklaufende Licht den x-Koppler (33) passiert und ebenfalls in die Auskoppelfaser (9) gelangt, wo das Licht aus dem Objektstrahlengang mit dem aus dem Referenzstrahlengang sich überlagert und zur Interferenz kommt

und **dass** die interferierenden Bündel O_N_N und R dabei zur spektroskopischen Analyse eine Lateralaufspaltung mittels Dispersion oder Diffraktion erfahren

und **dass** eine FFT des Intensitätsverlaufs erfolgen kann, um aus dem sich mittels FFT ergebenden Wertes nach Kalibrierung die Tiefenposition eines Objektpunktes P zu bestimmen

oder dass die interferierenden Bündel O_N_N und R eine weitere Interferenzbildung mittels scannendem Zweistrahl-Interferometer, also mit zeitlichem Variieren des optischen Gangunterschieds zur optischen Analyse, erfahren, wobei der im scannenden Zweistrahl-Interferometer beim Scan durchfahrene optische Gangunterschied mindestens einmal genau auch dem im Michelson-Interferometer zwischen Objekt-Teilstrahlenbündel O_N_N und Referenzstrahlenbündel R bestehenden optischen Gangunterschied entspricht, so dass beim Variieren des optischen Gangunterschieds ein moduliertes periodisches Signal erzeugt wird und mittels Punktempfänger, gerastertem Zeilenempfänger oder gerastertem Flächenempfänger über der Zeit detektiert wird

oder dass die interferierenden Bündel Objekt-Teilstrahlenbündel O_N_N und Referenzstrahlenbündel R eine weitere Interferenzbildung mittels statischem Zweistrahl-Interferometer zur Objekt-Teilstrahlenbündel optischen Analyse erfahren, also einem Zweistrahl-Interferometer mit lateraler Variation des optischen Gangunterschieds

oder dass im Fall der Anwendung einer Wellenlängen-durchstimmbaren Quelle elektromagnetischer Strahlung beim Scan der Wellenlänge ein moduliertes periodisches optisches Signal erzeugt wird und mittels Punktempfänger über der Zeit detektiert wird und dieses über der Wellenlänge ausgewertet wird.

**16.** Anordnung zur konfokalen Spektral-Interferometrie mit einem spektralen Zweistrahl-Interferometer, insbesondere auch zur Erfassung des Abstandes, des Profils und der Form und auch zur Optischen Kohärenz-Tomografie und/oder-Mikroskopie von biologischen und technischen Objekten in Auf- und/oder Durchlicht, wobei

- die Anordnung eine Multiwellenlängen-Quelle (1, 1a) elektromagnetischer Strahlung oder einer Vielzahl von Multiwellenlängen-Quellen aufweist und wobei
- die Anordnung ein Fokussier-System auf einem gerasterten Empfänger (111a) elektromagnetischer Strahlung aufweist, welches zu einer mikroskopischen Abbildung der Objektoberfläche und/oder des Inneren des Objektvolumens ausgelegt ist, wobei dem gerasterten Empfänger (111a) ein dispersives Spektrometer (100) vorgeordnet ist,
**gekennzeichnet dadurch,**
**daß** dem spektralen Zweistrahl-Interferometer eine chromatisch-konfokale Anordnung im Abbildungsstrahlengang des Fokussier-Systems zur Objektbeleuchtung und Objektdetektion zugeordnet ist und daß
das Interferometer als Common-path-Interferometer ausgebildet ist.

**17.** Anordnung zur konfokalen Spektral-Interferometrie nach Anspruch 16,
**gekennzeichnet dadurch,**
**daß** im Fokussier-System mindestens eine diffraktiv-optische Zonenlinse (5, 55, 105) angeordnet ist.

**18.** Anordnung zur konfokalen Spektral-Interferometrie nach Anspruch 17,
**gekennzeichnet dadurch,**
**daß** die diffraktiv-optische Zonenlinse (5, 55, 105) in der Pupille des Fokussier-Systems angeordnet ist, welche sich in der Fourier-Ebene des Fokussier-Objektivs (6, 12, 12b) befindet.

**19.** Anordnung nach Anspruch 16, insbesondere multispektrale interferometrische Anordnung zur hochdynamischen Objekttiefenabtastung oder -Profilerfassung,
wobei dem Zweistrahl-Interferometer die spektral breitbandige oder Wellenlängen-durchstimmbare Quelle elektromagnetischer Strahlung, als bevorzugte Multiwellenlängen-Quelle elektromagnetischer Strahlung, vorgeordnet ist, im weiteren als Lichtquelle (1) bezeichnet, welche ein Eingangsstrahlenbündel für das Zweistrahl-Interferometer erzeugt,
also das Zweistrahl-Interferometer mit einem Abbildungsstrahlengang für das zu prüfende Objekt (8) und einem Punktempfänger, einem gerasterten Zeilenempfänger oder einem gerasterten Flächenempfänger für elektromagnetische Strahlung (11, 111) (als bevorzugtem gerasterten Empfänger) ausgebildet ist,
**gekennzeichnet dadurch,**
**dass** chromatisch-konfokale Anordnung des Zweistrahl-Interferometers mit einer diffraktiv-optischen Zonenlinse (5, 105) und mittels in Richtung des Verlaufs des Eingangsstrahlenbündels nachgeordneter abbildender Linse (12) oder Objektiv (12a) mit Referenzspiegelfläche (7) mit oder ohne Strahlteilerschicht (7a, 107a) des Zweistrahl-Interferometers ausgebildet ist.

**20.** Anordnung nach Anspruch 19, insbesondere multispektrale interferometrische Anordnung zur hochdynamischen Objekttiefenabtastung oder -Profilerfassung
**gekennzeichnet dadurch,**

**dass** dem Zweistrahl-Interferometer ein Spektrometer (10) nachgeordnet ist.

21. Anordnung nach Anspruch 19, insbesondere multispektrale interferometrische Anordnung zur hochdynamischen Objekttiefenabtastung oder -Profilerfassung

**gekennzeichnet dadurch,**

**dass** dem objektabbildenden Zweistrahl-Interferometer ein Zweistrahl-Interferometer nachgeordnet ist, welches eine optische Analyse des Interferenzlichts am Ausgang des objektabbildenden Zweistrahl-Interferometers ermöglicht.

**Claims**

1. Confocal spectral interferometric method, in particular for recording of separation, profile and form as well as for optical coherence tomography and/or coherence microscopy of biological and technical objects or object details by incident and/or transmitted light, the method using:

   - either at least one multi-wavelength source of electromagnetic radiation (1, 1a), wherein with the use of the multi-wavelength source a dispersive spectrometer is arranged upstream of the scanned receiver (111a),
   - or at least one wavelength-tunable source of electromagnetic radiation

   and with microscopic imaging of the object surface and/or the interior of the object volume on a scanned receiver (111a) of electromagnetic radiation by means of a focussing system to detect the interfering electromagnetic radiation,

   **characterized in that**

   the chromatic-confocal technique is procedurally combined with the spectral two-beam interferometry, wherein a predetermined chromatic depth resolution of the electromagnetic radiation is performed in the focussing system as well as

   - either with the use of the multi-wavelength source a spectral analysis of the detected interfering electromagnetic radiation is performed by means of a dispersive spectrometer so that a spectrum is available
   - or with the use of the wavelength-tunable source a wavelength tuning is performed in a predetermined way and the interfering electromagnetic radiation is detected during the wavelength tuning, so that a spectrum is recorded over time

   and the z-position of each object detail is determined from the spectrum from the knowledge of the predetermined depth resolution in the chromatic confocal system and from at least the approximate knowledge of the refractive index.

2. The confocal spectral interferometric method of claim 1,
   **characterized in that**
   in the presence of a reflective or light dispersive area, i.e. an object detail, at least one wavelet over the spectral axis of the spectrometer (100) is produced in the object volume (208) during the scanning operation, the full width at half maximum of which is predetermined by the dimensioning of the chromatic depth resolution and the number of periods below the envelope of the wavelet that is determined by the dimensioning of the optical path difference and of the change of the path difference over the wavelength is rendered adjustable.

3. The confocal spectral interferometric method according to claim 1 and/or claim 2,
   **characterized in that** the method is performed for an optical path difference Dx unequal to zero in the interferometer.

4. The confocal spectral interferometric method according to at least one of claims 1 to 3,
   **characterized in that**
   by a chromatic longitudinal aberration, i. e. chromatic depth resolution, focuses in different depths of the object volume (208) are formed, wherein for each focus of any wavelength in the used spectral range the spherical aberration comprises a minimum for a predetermined setting in the focussing system, so that for a focus spot in every considered depth in the object volume (208) a diffraction-limited imaging exists in good approximation.

5. The confocal spectral interferometric method according to at least one of claims 1 to 4,
   **characterized in that**
   the signal amplitude of wavelets is evaluated.

6. The confocal spectral interferometric method according to at least one of claims 1 to 5,
   **characterized in that**
   the centre of gravity of the envelope of wavelets is evaluated.

7. The confocal spectral interferometric method according to at least one of claims 1 to 6,
   **characterized in that**
   the size of the maximum of the envelope of the wavelet is evaluated.

8. The confocal spectral interferometric method according to at least one of claims 1 to 7,
   **characterized in that**
   the phase of a wavelet is evaluated.

9. The method of claim 1, in particular an interferometric multi-spectral method for highly dynamic object depth resolution or profile recording, wherein the at least one source of electromagnetic radiation is a point-shaped source of electromagnetic radiation and wherein bundle-limiting means are located at least approximately in an optically conjugated plane of the at least one multi-wavelength source of electromagnetic radiation at the input of a two-beam interferometer and a reference beam bundle and an object beam bundle of electromagnetic radiation are produced in the two-beam interferometer,
   **characterized in that**
   a diffractive optical zone element is located in the two-beam interferometer by means of which electromagnetic radiation is produced in the zero diffraction order as well as in the first diffraction order or maybe also in a higher diffraction order N or in a lower diffraction order than in the zero diffraction order by transmission or reflexion,
   and the reference beam bundle of the two-beam interferometer in the forward and return path always passes the diffractive optical zone element in the zero diffraction order whereas the object beam bundle in the forward and return path passes the diffractive optical zone element in respect to the object in the first diffraction order or maybe in a higher diffraction order N respectively, whereby a chromatic longitudinal resolution - thus a longitudinal resolution emerging with the wavelength - of the object bundle in focuses takes place in the object space by diffraction of the electromagnetic radiation,
   and the electromagnetic radiation of at least approximately all wavelengths of the reference beam bundle $R\_0\_0$ that undergoes no diffraction at the diffractive zone element, after passing the diffractive optical zone element penetrates the bundle-limiting means for confocal discrimination, whereas after the diffractive optical zone element only the fractions of the object beam bundle $O\_1\_1$ or $O\_N\_N$ coming from the object pass the means for confocal discrimination at the output of the interferometer or in the two-beam interferometer that have been imaged on the object at least in approximation in a sharp way,
   two-beam interference takes place between the confocally discriminated radiation fractions of the reference and object beam bundle at least in approximation
   and subsequently the interfering electromagnetic radiation is guided in a spectrometer or another interferometer for analysis and is imaged to the receiver of electromagnetic radiation, where one receiver plane is located at least approximately in an optical conjugated plane of a least one point-shaped multi-wavelength source at the input of the two-beam interferometer,
   the separation or the profile of the object is determined by means of known algorithms for wavelet or interferogram analysis from the optical signals, which are produced in a spectroscopically analyzed way by means of a spectrometer or in an interferometric analysed way by means of another interferometer.

10. The method of claim 9, in particular a multi-spectral interferometric method for highly dynamic object depth sensing or profile recording,
    **characterized in that**
    the method is performed with an object-imaging Fizeau interferometer with a partially transmitting reference reflecting surface and with an object (8) to be inspected,
    and the Fizeau interferometer is formed with an imaging beam path for the object (8) to be inspected and a point receiver, a scanned line receiver or a scanned area receiver for electromagnetic radiation,
    and in the forward path of light from the light source (1) to the object a diffracted beam bundle $O\_N$, N = 1, 2, 3 ... or -1, -2, -3 ... in the first or a higher diffraction order N, N = 1, 2, 3 or a lower diffraction order N than the zero order diffraction, N = -1, -2, -3 is generated by means of diffraction by reflexion or transmission at a static or dynamic diffractive optical zone element (5, 105), wherein the diffracted beam bundle $O\_N$, N = 1, 2, 3... or -1, -2, -3 penetrates the partially transmitting reference reflective surface (7),
    wherein the diffracted beam bundle $O\_N$ is chromatically longitudinally resolved after focussing on the object (8) in the object space, so that a row or a plurality of rows of focal points in the depth is formed depending on the wavelength of light,

and after reflexion of a partial beam bundle O_N at the object (8) a partial object beam bundle of the beam bundle reflected backwards is produced by the diffraction at the diffractive optical zone element in the same order as in the forward path, namely O_N_N, N = 1, 2, 3... or -1, -2, -3..., so that the partial object beam bundle O_N_N after passing the diffractive optical zone element (5) goes back in the beam path,

and also another partial beam bundle, which serves as a reference beam bundle R_0 in the following, is formed by means of zero order diffraction at the diffractive optical zone element (5, 105) from the input beam bundle in the forward path on the reference reflective surface (7) and in the back path from the reference reflecting surface (7a), on which the reference beam bundle R_0 is focussed, a beam bundle R_0_0 is formed by diffraction at the diffractive optical zone element (5, 105) again exactly in the zero order, so that the partial reference beam bundle R_0_0 after passing the diffractive optical zone element goes back in the beam path,

and the optical path difference between the partial object beam bundle O_N_N and the reference beam bundle R_0_0 is made unequal to zero, if focussed on an object point,

and the partial object beam bundle O_N_N and the reference beam bundle R_0_0 are parallel after the diffractive optical zone element (5, 105) and interfere and subsequently, the two partial beam bundles interfering with each other are coupled into the detection beam path.

11. The method of claim 9, in particular a multi-spectral interferometric method for highly dynamic object depth sensing or profile recording,

**characterized in that**

the multi-spectral interferometric method for highly dynamic object depth sensing and profile measuring is performed with a Mirau interferometer.

12. The method of claim 9, in particular a multi-spectral interferometric method for highly dynamic object depth sensing or profile recording,

**characterized in that**

the multi-spectral interferometric method for highly dynamic object depth sensing and profile measuring is performed with a Michelson interferometer.

13. The method according to any of claims 9 to 12, in particular a multi-spectral interferometric method for highly dynamic object depth sensing or profile recording,

**characterized in that**

a confocal discrimination of the light of the interfering partial beam bundles is performed and the confocally discriminated interfering bundles O_N_N and R_0_0 undergo a lateral resolution by means of dispersion or diffraction for spectroscopic analysis and a FFT of the intensity characteristics is performed to determine the depth position of an object point from the value resulting by means of FFT after calibration,

or the interfering bundles O_N_N and R_0_0 undergo another forming of interference by means of a scanning two-beam interferometer (AZI) for optical analysis, thus, with varying the optical path difference over time, and the optical path difference undergone in the scanning two-beam interferometer during passing through the scan corresponds at least once exactly to the optical path difference existing in the interferometer between the partial object beam bundle O_N_N and the reference beam bundle R_0_0, so that varying the optical path difference a modulated periodic signal is produced and detected by means of a point receiver, a scanned line receiver or a scanned area receiver (11, 111) over time,

and the interfering bundles O_N_N and R_0_0 undergo another forming of interference by means of a statistical two-beam interferometer for optical analysis,

thus, with a two-beam interferometer with lateral variation of the optical path difference, wherein the locations with different path differences are assigned to pixels of a line or area camera (11, 111),

and the optical path difference occurring in the two-beam interferometer at least approximately corresponds to the optical path difference existing in the interferometer between the partial object beam bundle O_N_N and the reference beam bundle R_0_0 in at least one element of the line or area camera (11, 111),

or in case of the application of a wavelength-tunable source of electromagnetic radiation for the scanning of the wavelength an optical modulated periodic signal is produced and detected over time by means of a point receiver, a scanned line receiver or a scanned area receiver (11, 111) and it is evaluated over the wavelength.

14. The method of claim 1, in particular a multi-spectral interferometric method for highly dynamic object depth sensing or profile recording, with an object-imaging Mirau interferometer with an internal beam splitter, an internal reference reflecting surface (15) and with an object (8) to be inspected,

wherein a spectral broadband or wavelength-tunable source of electromagnetic radiation (as a preferred multi-wavelength source of electromagnetic radiation or as a preferred wavelength-tunable source of electromagnetic radiation) is arranged upstream of the Mirau interferometer, wherein the first is named a light source (1) in the following, which produces an input beam bundle for the Mirau interferometer,

thus, the Mirau interferometer is formed with an imaging beam path for the object (8) to be inspected and at least one single point receiver or scanned line receiver or scanned area receiver (11, 111) (as a preferred scanned receiver) for electromagnetic radiation,

**characterized in that**

a diffracted beam bundle O_N, N = 1, 2, 3... or -1, -2, -3... is produced in the first or a higher diffraction order N, N = 1, 2, 3 or a lower diffraction order than zero, N= -1, -2, -3, in the forward path on the way to the object (8) after the last lens of the Mirau interferometer after the beam splitter of the Mirau interferometer from the partial beam bundle passing this beam splitter by means of transmitting diffraction at a statistical or dynamical diffractive optical zone lens (5a)

which is chromatically longitudinally resolved in the object space after focusing on the object (8), so that dependent on the wavelength of light a row or a plurality of rows of focal points is formed in the depth,

and a partial object beam bundle from the beam bundle reflected backwards by diffraction at the diffractive optical zone lens (5a) having the same order as in the forward path, namely O_N_N, is formed after reflexion of the partial beam bundle O_N at the object (8), so that the partial object bundle O_N_N returns back on the beam path after having passed the diffractive optical zone lens (5a)

and also a partial beam bundle, which serves as the reference beam bundle R in the following, is formed from the input beam bundle in the forward path to the surface of the beam splitter, the beam splitter surface (7) of which is parallel to the diffractive optical zone lens (5a) and

in the back path form the reference reflecting surface (15), on which the reference beam bundle R is focussed, the reference beam bundle R returns back by reflexion in the beam path after having passed the beam splitter,

and the optical path difference between the partial object beam bundle O_N_N and the reference beam bundle R is unequal to zero, if focussed on an object point,

and the partial object beam bundle O_N_N and the reference beam bundle R extend parallel to each other after the diffractive optical zone lens (5a) and interfere and subsequently the two partial beam bundles interfering with each other are coupled into the detection beam path by means of amplitude separation of the wave fronts,

and a confocal discrimination of the light of the interfering partial beam bundles is performed,

and the interfering bundles, the partial object beam bundle O_N_N and reference beam bundle R, undergo a lateral resolution by means of dispersion or diffraction for spectroscopic analysis and a FFT of the intensity characteristics is performed to determine the depth position of an object point from the value resulting by means of FFT after calibration,

or the interfering bundles, the partial object beam bundles O_N_N and R, undergo another forming of interference by means of a scanning two-beam interferometer for optical analysis, thus, with varying the optical path difference over time, and the optical path difference undergone in the scanning two-beam interferometer during passing through the scan corresponds at least once exactly to the optical path difference existing in the interferometer between the partial object beam bundle O_N_N and the reference beam bundle R, so that varying the optical path difference a modulated periodic signal is produced and detected by means of a point receiver, a scanned line receiver or a scanned area receiver over time,

and the interfering bundles O_N_N and R undergo another forming of interference by means of a statistical two-beam interferometer for optical analysis,

thus, with a two-beam interferometer with lateral variation of the optical path difference, wherein the locations with different path difference are assigned to pixels of a line or area camera,

and the optical path difference occurring in the two-beam interferometer at least approximately corresponds to the optical path difference existing in the Mirau interferometer between the partial object beam bundle O_N_N and the reference beam bundle R in at least one element of the line or area camera,

or in case of the application of a wavelength-tunable source of electromagnetic radiation for the scanning of the wavelength an optical modulated periodic signal is produced and detected over time by means of a point receiver and it is evaluated over the wavelength.

15. The method of claim 1, in particular a multi-spectral interferometric method for highly dynamic object depth sensing or profile recording, with a fibre-coupled Michelson interferometer with a beam splitter formed as an x-coupler (33) and with a reference mirror (15a) or with a reference reflective surface und with an object (8) to be inspected,

wherein a spectral broadband or wavelength-tunable source of electromagnetic radiation (as a preferred multi-wavelength source of electromagnetic radiation or as a preferred wavelength-tunable source of electromagnetic radiation) is arranged upstream of the Michelson interferometer, wherein the first is named a light source (1) in the following, which produces an input beam bundle for the Michelson interferometer,

thus, the fibre-coupled Michelson interferometer is formed with an imaging beam path in the object beam path for the object (8) to be inspected and at least one single point receiver, scanned line receiver or a scanned area receiver (11, 111) (as a preferred scanned receiver) for electromagnetic radiation,

**characterized in that**

a diffracted beam bundle O_N, N = 1, 2, 3... or -1, -2, -3... is produced in the first or a higher diffraction order N, N = 1, 2, 3 or a lower diffraction order than zero, N= -1, -2, -3, in the forward path on the way to the object after the x-coupler (33) of the Michelson interferometer by means of transmitting diffraction at a statistical or dynamical diffractive optical zone lens (5a) and this diffracted beam bundle O_N is chromatically longitudinally resolved after focussing on the object (8) in the object space, and after reflexion of the partial beam bundle O_N at the object (8) a partial object beam bundle is formed from the beam bundle reflected back by diffraction at the diffractive optical zone lens in the same order as in the forward path, namely O_N_N, so that the partial object beam bundle O_N_N having passed the diffractive optical zone lens (5) goes back in the beam path again, is discriminated confocally by means of the fibre end, subsequently passes the x-coupler (33) and arrives in an output coupling fibre (9),

and the reference bundle R is produced in the forward path by separation at the x-coupler (33) and is reflected at the fibre end directly at the reference reflecting surface (7b) or a reference reflecting surface at the end of a focussing optics arranged after the fibre,

the light going back in the fibre passes the x-coupler (33) and also arrives in the output coupling fibre (9), where the light from the object beam path superimposes on that from the reference beam path and interferes with it,

the interfering bundles O_N_N and R undergo a lateral resolution by means of dispersion or diffraction for spectroscopic analysis,

and an FTT of the intensity characteristic can be performed to determine the depth position of an object point P from the value resulting by means of the FFT after calibration,

or the interfering bundles O_N_N and R undergo another forming of interference by means of a scanning two-beam interferometer for optical analysis, thus, with varying the optical path difference over time, and wherein the optical path difference undergone in the scanning two-beam interferometer during passing through the scan corresponds at least once exactly to the optical path difference existing in the Michelson interferometer between the partial object beam bundle O_N_N and the reference beam bundle R, so that varying the optical path difference a modulated periodic signal is produced and detected by means of a point receiver, scanned line receiver or a scanned area receiver over time,

or the interfering bundles, the partial object beam bundle O_N_N and the reference beam bundle R, undergo another forming of interference by means of a statistical two-beam interferometer for optical analysis of partial object beam bundles, thus by means of a two-beam interferometer with lateral variation of the optical path difference,

or in case of the application of a wavelength-tunable source of electromagnetic radiation for the scanning of the wavelength a modulated periodic optical signal is produced and detected over time by means of a point receiver and is evaluated over the wavelength.

16. Arrangement for confocal spectral interferometry, in particular for recording of separation, profile and optical form as well as for optical coherence tomography and/or coherence microscopy of biological and technical objects by incident and/or transmitted light, wherein

- the arrangement comprises one multi-wavelength source (1, 1a) of electromagnetic radiation or a plurality of multi-wavelength sources and wherein
- the arrangement comprises a focussing system on a scanned receiver (111a) for electromagnetic radiation, which is designed for a microscopic imaging of the object surface and/or of the interior of the object volume, a dispersive spectrometer being arranged upstream of the scanned receiver (111a),

**characterized in that**

a chromatic confocal arrangement is assigned to the spectral two-beam interferometer in the imaging beam path of the focussing system for object illumination and object detection and

the interferometer is formed as a common-path interferometer.

17. The arrangement for confocal spectral interferometry of claim 16,

**characterized in that**

at least on diffractive optical zone lens (5, 55, 105) is arranged in the focussing system.

18. The arrangement for confocal spectral interferometry of claim 17,

**characterized in that**

the diffractive optical zone lens (5, 55, 105) is arranged in the pupil of the focussing system, which is located in the Fourier plane of the focussing objective (6, 12, 12b).

19. The arrangement of claim 16, in particular a multi-spectral interferometric arrangement for highly dynamic object depth sensing or profile recording,

wherein a spectral broadband or wavelength-tunable source of electromagnetic radiation as a preferred multi-wavelength source of electromagnetic radiation is arranged upstream of the two-beam interferometer, wherein the first is named a light source (1) in the following, which produces an input beam bundle for the two-beam interferometer, thus, the two-beam interferometer is formed with an imaging beam path for the object (8) to be inspected and a point receiver, a scanned line receiver or scanned area receiver (11, 111) (as a preferred scanned receiver) for electromagnetic radiation,

**characterized in that**

the chromatic confocal arrangement of the two-beam interferometer is formed with a diffractive optical zone lens (5, 105) und by means of a downstream imaging lens (12) or objective (12a) with a reference reflecting surface (7) in the direction of the course of the input beam bundle with or without beam splitter layer (7a, 107a) of the two-beam interferometer.

20. The arrangement of claim 19, in particular a multi-spectral interferometric arrangement for highly dynamic object depth sensing or profile recording,

**characterized in that**

a spectrometer (10) is arranged downstream of the two-beam interferometer.

21. The arrangement of claim 19, in particular a multi-spectral interferometric arrangement for highly dynamic object depth sensing or profile recording,

**characterized in that**

a two-beam interferometer is arranged downstream of the object-imaging two-beam interferometer, which enables an optical analysis of the interference light at the output of the object-imaging two-beam interferometer.

**Revendications**

1. Procédé pour l'interférométrie confocale spectrale, en particulier aussi pour la saisie de la distance, du profil et de la forme et aussi pour la tomographie à cohérence optique et/ou la microscopie à cohérence optique des objets biologiques et techniques ou de détails d'objets dans la lumière incidente et/ou la lumière transmise, les composants suivants étant utilisés dans le procédé :

- soit au moins une source de longueurs d'onde multiples à rayonnement électromagnétique (1, 1a), dans laquelle un spectromètre dispersif (100) est agencé en amont du récepteur à trame (111a) lors de l'utilisation de la source de longueurs d'onde multiples,
- ou au moins une source de longueurs d'onde ajustable à rayonnement électromagnétique

et avec représentation microscopique de la surface d'objet et/ou de l'intérieur du volume d'objet au moyen du système de focalisation sur un récepteur à trame (111a) à rayonnement électromagnétique pour la détection du rayonnement électromagnétique qui interfère,

**caractérisé en ce que**

la technique chromatique confocale est reliée à l'interférométrie spectrale à double rayon quant au procédé, sachant qu'aussi bien un dédoublement prédéterminé chromatique en profondeur du rayonnement électromagnétique a lieu dans le système de focalisation que

- soit, lors de l'utilisation de la source de longueurs d'onde multiples, une analyse spectrale du rayonnement électromagnétique détecté qui interfère est exécutée au moyen du spectromètre dispersif de telle sorte qu'un spectre est disponible,
- soit, lors de l'utilisation de la source ajustable en longueur d'onde, un ajustement de la longueur d'onde est exécuté de manière prédéterminée et le rayonnement électromagnétique qui interfère est détecté lors de l'ajustement de la longueur d'onde de telle sorte qu'un spectre est enregistré sur le temps,

et **en ce que** la position z de chaque détail d'objet est déterminée à partir du spectre par l'intermédiaire de la connaissance du dédoublement en profondeur prédéterminé dans le système chromatique confocal et de la connaissance au moins approximative de l'indice de réfraction.

**2.** Procédé pour l'interférométrie confocale spectrale selon la revendication 1,
**caractérisé en ce que**
en présence d'une zone réfléchissante ou qui diffuse de la lumière, c'est-à-dire un détail d'objet, un volume d'objet (208) dans l'opération d'exploration, au moins une ondelette est générée sur l'axe spectral du spectromètre (100) dont la largeur de valeur moyenne est prédéterminée par le dimensionnement du dédoublement chromatique en profondeur, et **en ce que** le nombre de périodes sous l'enveloppe de l'ondelette, qui est prédéterminée par le dimensionnement de la différence optique des chemins et par la modification de la différence des chemins sur la longueur d'onde, est réalisée avec possibilité de réglage.

**3.** Procédé pour l'interférométrie confocale spectrale selon au moins une des revendications 1 ou 2,
**caractérisé en ce que** le procédé est exécuté pour une différence optique des chemins Dx différente de zéro dans l'interféromètre.

**4.** Procédé pour l'interférométrie confocale spectrale selon au moins une des revendications 1 à 3,
**caractérisé en ce que**
des foyers se forment à des profondeurs différentes du volume d'objet (208) suite à une aberration longitudinale chromatique, c'est-à-dire suite à un dédoublement chromatique en profondeur, sachant que l'aberration sphérique présente un minimum par un réglage prédéterminé dans le système de focalisation pour chaque foyer de chaque longueur d'onde quelconque dans la zone spectrale utilisée, de telle sorte qu'il existe une reproduction limitée par la diffraction pour une tache de foyer à chaque profondeur considérée dans le volume d'objet (208) dans une bonne approximation.

**5.** Procédé pour l'interférométrie confocale spectrale selon au moins une des revendications 1 à 4,
**caractérisé en ce que**
l'amplitude de signal des ondelettes est évaluée.

**6.** Procédé pour l'interférométrie confocale spectrale selon au moins une des revendications 1 à 5,
**caractérisé en ce que**
le centre de gravité de l'enveloppe des ondelettes est évalué.

**7.** Procédé pour l'interférométrie confocale spectrale selon au moins une des revendications 1 à 6,
**caractérisé en ce que**
la dimension du maximum de l'enveloppe des ondelettes est évaluée.

**8.** Procédé pour l'interférométrie confocale spectrale selon au moins une des revendications 1 à 7,
**caractérisé en ce que**
la phase d'une ondelette est évaluée.

**9.** Procédé selon la revendication 1, en particulier un procédé interférométrique multispectral pour l'exploration en profondeur ou la saisie de profils d'objets de manière hautement dynamique, dans lequel la au moins une source à rayonnement électromagnétique est une source ponctuelle à rayonnement électromagnétique et dans lequel des moyens limitant le faisceau se trouvent au moins approximativement dans un plan optiquement conjugué de la au moins une source ponctuelle de longueurs d'onde multiples à rayonnement électromagnétique sur l'entrée d'un interféromètre à double rayon et dans lequel un faisceau de rayons de référence et un faisceau de rayons d'objet à rayonnement électromagnétique sont générés dans l'interféromètre à double rayon,
**caractérisé en ce que**
un élément de zone diffractif optique est agencé dans l'interféromètre à double rayon, au moyen duquel le rayonnement électromagnétique est généré aussi bien dans l'ordre de diffraction zéro que dans le premier ordre de diffraction ou le cas échéant, aussi dans un ordre de diffraction plus élevé N ou dans un ordre de diffraction plus bas que l'ordre de diffraction zéro en transmission ou en réflexion,
et **en ce que** le faisceau de rayons de référence de l'interféromètre à double rayon dans les sens de parcours « aller » et « retour » passe toujours par l'élément de zone diffractif optique dans l'ordre de diffraction zéro et par contre le faisceau de rayons d'objet dans les sens de parcours « aller » et « retour » en référence à l'objet passe par l'élément de zone diffractif optique respectivement dans le premier ordre de diffraction ou le cas échéant respectivement dans un ordre de diffraction plus élevé N, à la suite de quoi un dédoublement longitudinal chromatique - c'est-à-dire se produisant sur la longueur d'onde - du faisceau d'objet dans les foyers a lieu dans l'espace d'objet par la diffraction du rayonnement électromagnétique,
et **en ce que** le rayonnement électromagnétique d'au moins approximativement toutes les longueurs d'onde du faisceau de rayons de référence R_0_0, lequel ne subit aucune diffraction sur l'élément de zone diffractif optique, traverse les moyens limitant les faisceaux pour la discrimination confocale après le passage par l'élément de zone diffractif optique, tandis que, après l'élément de zone diffractif optique pour le faisceau de rayons d'objet O_1_1 ou O_N_N en provenance de l'objet, seules les parts passent par les moyens de limitation pour la discrimination confocale sur la sortie de l'interféromètre ou dans l'interféromètre à double rayon, lesquelles étaient reproduites au moins approximativement avec précision sur l'objet,

et **en ce qu'**une interférence à double rayon a lieu au moins approximativement entre des parts de rayonnement à discrimination confocale du faisceau de rayons de référence et du faisceau de rayons d'objet,

et **en ce que** le rayonnement électromagnétique qui interfère est ensuite guidé pour l'analyse dans un spectromètre ou dans un autre interféromètre et **en ce qu'**un rayonnement électromagnétique est reproduit sur le récepteur, sachant qu'un plan de récepteur se trouve au moins approximativement dans un plan optiquement conjugué à au moins une des sources ponctuelles de longueurs d'onde multiples sur l'entrée de l'interféromètre à double rayon,

et **en ce que**, à partir des signaux optiques générés par analyse spectroscopique au moyen d'un spectromètre ou par analyse interférométrique au moyen d'un autre interféromètre, la distance ou le profil de l'objet est déterminé au moyen d'algorithmes connus pour l'analyse des ondelettes ou de l'interférogramme.

10. Procédé selon la revendication 9, en particulier un procédé multispectral interférométrique pour l'exploration en profondeur ou la saisie de profils d'objets de manière hautement dynamique,
   **caractérisé en ce que**
   le procédé est exécuté avec un interféromètre du type « Fizeau » qui reproduit les objets avec une surface de miroir de référence en partie diaphane et un objet à examiner (8),
   et **en ce que** l'interféromètre du type « Fizeau » est réalisé avec une trajectoire du faisceau de reproduction pour l'objet à examiner (8) et avec un récepteur ponctuel, un récepteur linéaire à trame ou un récepteur surfacique à trame pour un rayonnement électromagnétique,
   et **en ce que**, dans le sens de parcours « aller » de la lumière depuis la source de lumière (1) vers l'objet depuis le faisceau de rayons d'entrée, un faisceau de rayons diffracté O_N, N = 1, 2, 3 ... ou -1, -2, -3 ..., se génère dans le premier ordre de diffraction ou dans un ordre de diffraction plus élevé N, N = 1, 2, 3, ou dans un ordre de diffraction N plus bas que l'ordre de diffraction zéro, N = -1, -2, -3, au moyen d'une diffraction en réflexion ou en transmission sur un élément de zone statique ou dynamique, diffractif optique (5, 105), sachant que le faisceau de rayons diffracté O_N, N = 1, 2, 3 ... ou -1, -2, -3 ..., traverse la surface de miroir de référence en partie diaphane (7),
   dans lequel le faisceau de rayons diffracté O_N est dédoublé chromatiquement en sens longitudinal dans l'espace d'objet après la focalisation sur l'objet (8), de telle sorte qu'une rangée ou une pluralité de rangées de points de focalisation est réalisée en profondeur en dépendance de la longueur d'onde de la lumière,
   et **en ce que**, après la réflexion du faisceau de rayons partiels O_N sur l'objet (8), un faisceau de rayons partiels d'objet est formé par le faisceau de rayons réfléchi en retour par la diffraction sur l'élément de zone diffractif optique dans le même ordre que dans le sens de parcours « aller », à savoir O_N_N, N = 1, 2, 3 ... ou -1, -2, -3 ..., de telle sorte que le faisceau de rayons partiels d'objet O_N_N retourne à nouveau dans la trajectoire du faisceau après le passage par l'élément de zone diffractif optique (5),
   et **en ce que**, depuis le faisceau de rayons d'entrée dans le sens de parcours « aller » sur la surface de miroir de référence (7), un faisceau de rayons partiel, qui sert par la suite de faisceau de rayons de référence R_0, est aussi encore formé par la diffraction dans l'ordre zéro sur l'élément de zone diffractif optique (5, 105) et dans le sens de parcours « retour » par la surface de miroir de référence (7a) sur laquelle le faisceau de rayons de référence R_0 est focalisé,
   et **en ce que**, exactement un faisceau de rayons R_0_0 est aussi formé à nouveau par la diffraction dans l'ordre zéro sur l'élément de zone diffractif optique (5, 105) de telle sorte que le faisceau de rayons de référence R_0_0 retourne à nouveau dans la trajectoire du faisceau après le passage par l'élément de zone diffractif optique,
   et **en ce que**, entre le faisceau de rayons partiels d'objet O_N_N et le faisceau de rayons de référence R_0_0, la différence optique des chemins est rendue différente de zéro lorsque la focalisation est réalisée sur un point objet,
   et **en ce que** le faisceau de rayons partiels d'objet O_N_N et le faisceau de rayons de référence R_0_0 ont un trajet parallèle après l'élément de zone diffractif optique (5, 105) et viennent en interférence et qu'ensuite, les deux faisceaux de rayon partiel qui s'interfèrent mutuellement sont couplés dans la trajectoire du faisceau de détection.

11. Procédé selon la revendication 9, en particulier un procédé multispectral interférométrique pour l'exploration en profondeur ou la saisie de profils d'objets de manière hautement dynamique,
   **caractérisé en ce que**
   le procédé multispectral interférométrique est exécuté avec un interféromètre du type « Mirau » pour l'exploration en profondeur et la saisie de profils d'objets de manière hautement dynamique.

**12.** Procédé selon la revendication 9, en particulier un procédé multispectral interférométrique pour l'exploration en profondeur ou la saisie de profils d'objets de manière hautement dynamique,
**caractérisé en ce que**
le procédé interférométrique multispectral est exécuté avec un interféromètre du type « Michelson » pour l'exploration en profondeur et la saisie de profils d'objets de manière hautement dynamique.

**13.** Procédé selon l'une des revendications 9 à 12, en particulier un procédé multispectral interférométrique pour l'exploration en profondeur ou la saisie de profils d'objets de manière hautement dynamique,
**caractérisé en ce que**
une discrimination confocale de la lumière du faisceau de rayons partiels qui interfère est exécutée et que les faisceaux qui interfèrent et à discrimination confocale O_N_N et R_0_0 subissent pour l'analyse spectroscopique un dédoublement latéral au moyen d'une dispersion ou d'une diffraction et qu'une transformée de Fourier rapide (FFT) de la courbe d'intensité a lieu afin de déterminer la position en profondeur d'un point objet après le calibrage à partir de la valeur résultante au moyen de la transformée de Fourier rapide (FFT),
ou **en ce que** les faisceaux qui interfèrent O_N_N et R_0_0 subissent une autre formation d'interférence au moyen de l'interféromètre de scanning à double rayon (AZI) pour l'analyse optique, c'est-à-dire avec variation temporelle de la différence optique des chemins, et **en ce que** la différence optique des chemins balayée dans l'interféromètre de scanning à double rayon pendant le scannage correspond au moins une fois exactement aussi à la différence optique des chemins qui existe dans l'interféromètre entre le faisceau de rayons partiels d'objet O_N_N et le faisceau de rayons de référence R_0_0, de telle sorte que, lors de la variation de la différence optique des chemins, un signal périodique modulé est généré et que la détection est effectuée sur le temps au moyen d'un récepteur ponctuel, d'un récepteur linéaire à trame ou d'un récepteur surfacique à trame (11, 111),
ou **en ce que** les faisceaux qui interfèrent O_N_N et R_0_0 subissent une autre formation d'interférence au moyen de l'interféromètre statique à double rayon pour l'analyse optique,
c'est-à-dire au moyen d'un interféromètre à double rayon avec variation latérale de la différence optique des chemins, sachant que les emplacements de la différence des chemins sont attribués à différents pixels d'une caméra linéaire ou d'une caméra surfacique (11, 111),
et **en ce que** la différence optique des chemins qui se produit dans l'interféromètre à double rayon correspond au moins approximativement à la différence optique des chemins qui existe dans l'interféromètre entre le faisceau de rayons partiels d'objet O_N_N et le faisceau de rayons de référence R_0_0 dans au moins un élément de la caméra linéaire ou de la caméra surfacique (11, 111),
ou **en ce que**, dans le cas de l'application d'une source à rayonnement électromagnétique, ajustable en longueur d'onde, pendant le scannage de la longueur d'onde, un signal périodique optique modulé est généré et **en ce que** la détection est effectuée sur le temps au moyen d'un récepteur ponctuel, d'un récepteur linéaire à trame ou d'un récepteur surfacique à trame (11, 111) et **en ce que** celui-ci est évalué par l'intermédiaire de la longueur d'onde.

**14.** Procédé selon la revendication 1, en particulier un procédé multispectral interférométrique pour l'exploration en profondeur ou la saisie de profils d'objets de manière hautement dynamique, comprenant un interféromètre du type « Mirau » qui reproduit les objets, comprenant un diviseur de rayon interne, une surface de miroir de référence (15) et un objet à examiner (8),
dans lequel l'interféromètre du type « Mirau » est agencé en amont d'une source spectrale à bande large ou une d'une source à rayonnement électromagnétique, ajustable en longueur d'onde (comme source préférée de longueurs d'onde multiples à rayonnement électromagnétique ou comme source à rayonnement électromagnétique, ajustable en longueur d'onde), désignée par la suite comme source de lumière (1), laquelle génère un faisceau de rayons d'entrée pour l'interféromètre du type « Mirau »,
c'est-à-dire l'interféromètre du type « Mirau » est réalisé avec une trajectoire de faisceau de reproduction pour l'objet à examiner (8) et au moins un récepteur ponctuel unique ou un récepteur linéaire à trame ou un récepteur surfacique à trame (11, 111) (comme récepteur à trame préféré) pour le rayonnement électromagnétique,
**caractérisé en ce que**
dans le sens de parcours « aller » de la lumière sur le trajet vers l'objet (8) après la dernière lentille de l'interféromètre du type « Mirau » après le diviseur de rayon de l'interféromètre du type « Mirau », à partir du faisceau de rayons partiels qui passe par ce diviseur de rayon au moyen d'une diffraction en transmission sur une lentille de zone statique ou dynamique diffractive optique (5a), un faisceau de rayons diffracté O_N, N = 1, 2, 3 ... ou -1, -2, -3 ..., se génère dans le premier ordre de diffraction ou dans un ordre de diffraction plus élevé N, N = 1, 2, 3, ou dans un ordre de diffraction N plus bas que l'ordre de diffraction zéro, N = -1, -2, -3,
lequel, après la focalisation sur l'objet (8) dans l'espace d'objet, est dédoublé chromatiquement en sens longitudinal de telle sorte qu'une rangée ou une pluralité de rangées de points de focalisation est réalisée en profondeur en dépendance de la longueur d'onde de la lumière,

et après la réflexion du faisceau de rayons partiels O_N sur l'objet (8), un faisceau de rayons partiels d'objet est formé par le faisceau de rayons réfléchi en retour suite à la diffraction sur la lentille de zone diffractive optique (5a) dans le même ordre que dans le sens de parcours « aller », à savoir O_N_N, de telle sorte que le faisceau de rayons partiels d'objet O_N_N retourne à nouveau dans la trajectoire du faisceau après le passage par la lentille de zone diffractive optique (5a),

et, à partir du faisceau de rayons d'entrée dans le sens de parcours « aller » sur la surface du diviseur de rayon, la surface de division de rayon (7) de celui-ci est disposée en parallèle à la lentille de zone diffractive optique (5a), un faisceau de rayons partiel,

lequel sert ci-après de faisceau de rayons de référence R,

est aussi encore formé et est focalisé dans le sens de parcours « retour » par la surface de miroir de référence (15) sur laquelle le faisceau de rayons de référence R est focalisé,

et **en ce que** le faisceau de rayons de référence R retourne à nouveau dans la trajectoire du faisceau après le passage par le diviseur de rayon dans la réflexion,

et, entre le faisceau de rayons partiels d'objet O_N_N et le faisceau de rayons de référence R, la différence optique des chemins est rendue différente de zéro lorsque la focalisation est effectuée sur un point objet,

et **en ce que** le faisceau de rayons partiels d'objet O_N_N et le faisceau de rayons de référence R ont un trajet parallèle après la lentille de zone diffractive optique (5a) et viennent en interférence et qu'ensuite les deux faisceaux de rayons partiels qui s'interfèrent mutuellement sont couplés dans la trajectoire du faisceau de détection au moyen d'une division de l'amplitude des fronts d'ondes,

et une discrimination confocale de la lumière des faisceaux de rayons partiels qui interfèrent est exécutée,

et **en ce que** les faisceaux qui interfèrent - le faisceau de rayons partiels d'objet O_N_N et le faisceau de rayons de référence R - subissent pour l'analyse spectroscopique un dédoublement latéral au moyen d'une dispersion ou d'une diffraction et qu'une transformée de Fourier rapide (FFT) de la courbe d'intensité est exécutée afin de déterminer la position en profondeur d'un point objet après le calibrage à partir de la valeur résultante au moyen de la transformée de Fourier rapide (FFT),

ou **en ce que** les faisceaux qui interfèrent - le faisceau de rayons partiels d'objet O_N_N et le faisceau de rayons de référence R - subissent une autre formation d'interférence au moyen d'un interféromètre de scanning à double rayon, c'est-à-dire avec variation temporelle de la différence optique des chemins pour l'analyse optique, et **en ce que** la différence optique des chemins balayée dans l'interféromètre de scanning à double rayon pendant le scannage correspond au moins une fois exactement aussi à la différence optique des chemins qui existe dans l'interféromètre entre le faisceau de rayons partiels d'objet O_N_N et le faisceau de rayons de référence R, de telle sorte que, lors de la variation de la différence optique des chemins, un signal périodique modulé est généré et que la détection est effectuée sur le temps au moyen d'un récepteur ponctuel, d'un récepteur linéaire à trame ou d'un récepteur surfacique à trame,

ou **en ce que** les faisceaux qui interfèrent O_N_N et R subissent une autre formation d'interférence au moyen d'un interféromètre statique à double rayon pour l'analyse optique,

c'est-à-dire au moyen d'un interféromètre à double rayon avec variation latérale de la différence optique des chemins, sachant que les emplacements de la différence des chemins sont attribués à différents pixels d'une caméra linéaire ou d'une caméra surfacique,

et **en ce que** la différence optique des chemins qui se produit dans l'interféromètre à double rayon correspond au moins approximativement à la différence optique des chemins qui existe dans l'interféromètre du type « Mirau » entre le faisceau de rayons partiels d'objet O_N_N et le faisceau de rayons de référence R dans au moins un élément de la caméra linéaire ou de la caméra surfacique,

ou **en ce que**, dans le cas de l'application d'une source à rayonnement électromagnétique, ajustable en longueur d'onde, pendant le scannage de la longueur d'onde, un signal périodique optique modulé est généré et **en ce que** la détection est effectuée sur le temps au moyen d'un récepteur ponctuel et **en ce que** celui-ci est évalué par l'intermédiaire de la longueur d'onde.

15. Procédé selon la revendication 1, en particulier un procédé multispectral interférométrique pour l'exploration en profondeur ou la saisie de profils d'objets de manière hautement dynamique, comprenant un interféromètre du type « Michelson » qui est couplé à des fibres, comprenant un diviseur de rayon qui est réalisé comme coupleur en X (33), et comprenant un miroir de référence (15a) ou une surface de miroir de référence et un objet à examiner (8), dans lequel l'interféromètre du type « Michelson » couplé à des fibres est agencé en amont d'une source spectrale à bande large ou une d'une source à rayonnement électromagnétique, ajustable en longueur d'onde (comme source préférée de longueurs d'onde multiples à rayonnement électromagnétique ou comme source à rayonnement électromagnétique, ajustable en longueur d'onde), désignée par la suite comme source de lumière (1), laquelle génère un faisceau de rayons d'entrée pour l'interféromètre du type « Michelson »,

c'est-à-dire l'interféromètre du type « Michelson » couplé à des fibres est réalisé dans la trajectoire de faisceau d'objet avec une trajectoire de faisceau de reproduction pour l'objet à examiner (8) et au moins un récepteur ponctuel unique, un récepteur linéaire à trame ou un récepteur surfacique à trame (11, 111) pour le rayonnement électromagnétique (comme récepteur à trame préféré pour le rayonnement électromagnétique),

**caractérisé en ce que**

dans le sens de parcours « aller » de la lumière sur le trajet vers l'objet après le coupleur en X (33) de l'interféromètre du type « Michelson », au moyen d'une diffraction en transmission sur une lentille de zone statique ou dynamique diffractive optique (5), un faisceau de rayons diffracté O_N, N = 1, 2, 3 ... ou -1, -2, -3 ..., se génère dans le premier ordre de diffraction ou dans un ordre de diffraction plus élevé N, N = 1, 2, 3, ou dans un ordre de diffraction N plus bas que l'ordre de diffraction zéro, N = -1, -2, -3, et **en ce que** ce faisceau de rayons diffracté O_N est dédoublé chromatiquement en sens longitudinal après la focalisation sur l'objet (8) dans l'espace d'objet, et après la réflexion du faisceau de rayons partiels O_N sur l'objet (8), un faisceau de rayons partiels d'objet est formé par le faisceau de rayons réfléchi en retour suite à la diffraction sur la lentille de zone diffractive optique dans le même ordre que dans le sens de parcours « aller », à savoir O_N_N, de telle sorte que le faisceau de rayons partiels d'objet O_N_ N retourne à nouveau dans la trajectoire du faisceau après le passage par la lentille de zone diffractive optique (5), est discriminé de manière confocale au moyen de l'extrémité de fibre, passe ensuite par le coupleur en X (33) et parvient dans une fibre de découplage (9),

et **en ce que** le faisceau de référence R se génère dans le sens de parcours « aller » par une division sur le coupleur en X (33) et est réfléchi sur l'extrémité de la fibre directement sur une surface de miroir de référence (7b) ou sur l'extrémité d'une des fibres du système optique à focalisation agencé en aval sur une surface de miroir de référence,

et **en ce que** la lumière qui retourne dans la fibre passe par le coupleur en X (33) et parvient également dans la fibre de découplage (9) où la lumière en provenance de la trajectoire du faisceau d'objet se superpose avec la lumière en provenance de la trajectoire du faisceau de référence et vient en interférence,

et **en ce qu'**une transformée de Fourier rapide (FFT) de la courbe d'intensité peut avoir lieu afin de déterminer la position en profondeur d'un point objet P après le calibrage à partir de la valeur résultante au moyen de la transformée de Fourier rapide (FFT),

ou **en ce que** les faisceaux qui interfèrent O_N_N et R subissent une autre formation d'interférence au moyen de l'interféromètre de scanning à double rayon, c'est-à-dire avec variation temporelle de la différence optique des chemins pour l'analyse optique, sachant que la différence optique des chemins balayée dans l'interféromètre de scanning à double rayon pendant le scannage correspond au moins une fois exactement aussi à la différence optique des chemins qui existe dans l'interféromètre du type « Michelson » entre le faisceau de rayons partiels d'objet O_N_N et le faisceau de rayons de référence R, de telle sorte que, lors de la variation de la différence optique des chemins, un signal périodique modulé est généré et que la détection est effectuée sur le temps au moyen d'un récepteur ponctuel, d'un récepteur linéaire à trame ou d'un récepteur surfacique à trame,

ou **en ce que** les faisceaux qui interfèrent - le faisceau de rayons partiels d'objet O_N_N et le faisceau de rayons de référence R - subissent une autre formation d'interférence au moyen de l'interféromètre statique à double rayon pour l'analyse optique du faisceau de rayons partiels d'objet, c'est-à-dire au moyen d'un interféromètre à double rayon avec variation latérale de la différence optique des chemins,

ou **en ce que**, dans le cas de l'application d'une source à rayonnement électromagnétique, ajustable en longueur d'onde, pendant le scannage de la longueur d'onde, un signal périodique optique modulé est généré et **en ce que** la détection est effectuée sur le temps au moyen d'un récepteur ponctuel et **en ce que** celui-ci est évalué par l'intermédiaire de la longueur d'onde.

16. Système pour l'interférométrie spectrale confocale, comprenant un interféromètre spectral à double rayon, en particulier aussi pour la saisie de la distance, du profil et de la forme et aussi pour la tomographie à cohérence optique et/ou la microscopie à cohérence optique des objets biologiques et techniques dans la lumière incidente et/ou la lumière transmise, dans lequel

- le système présente une source de longueurs d'onde multiples à rayonnement électromagnétique (1, 1a) ou une pluralité de sources de longueurs d'onde multiples et dans lequel
- le système présente un système de focalisation sur un récepteur à trame (111a) à rayonnement électromagnétique, lequel est conçu pour une reproduction microscopique de la surface d'objet et/ou de l'intérieur du volume d'objet, et dans lequel un spectromètre dispersif (100) est agencé en amont du récepteur à trame (111a),

**caractérisé en ce que**

un système chromatique confocal est attribué à l'interféromètre spectral à double rayon dans la trajectoire du faisceau de reproduction du système de focalisation pour l'éclairage de l'objet et la détection de l'objet, et **en ce que** l'interféromètre est réalisé comme interféromètre du type « à chemin commun » (« common path »).

**17.** Système pour l'interférométrie spectrale confocale selon la revendication 16,
**caractérisé en ce que**
au moins une lentille de zone diffractive optique (5, 55, 105) est agencée dans le système de focalisation.

**18.** Système pour l'interférométrie spectrale confocale selon la revendication 17,
**caractérisé en ce que**
la lentille de zone diffractive optique (5, 55, 105) est agencée dans la pupille du système de focalisation, laquelle se trouve dans le plan de Fourier de l'objectif de focalisation (6, 12 12b).

**19.** Système selon la revendication 16, en particulier un système multispectral interférométrique pour l'exploration en profondeur ou la saisie de profils d'objets de manière hautement dynamique,
dans lequel l'interféromètre à double rayon est agencé en amont de la source spectrale à bande large ou de la source à rayonnement électromagnétique, ajustable en longueur d'onde, comme source préférée de longueurs d'onde multiples à rayonnement électromagnétique, désignée par la suite comme source de lumière (1), laquelle génère un faisceau de rayons d'entrée pour l'interféromètre à double rayon,
c'est-à-dire l'interféromètre à double rayon est réalisé avec une trajectoire de faisceau de reproduction pour l'objet à examiner (8) et avec un récepteur ponctuel, un récepteur linéaire à trame ou un récepteur surfacique à trame pour le rayonnement électromagnétique (11, 111) (comme récepteur à trame préféré),
**caractérisé en ce que**
le système chromatique confocal de l'interféromètre à double rayon est réalisé avec une lentille de zone diffractive optique (5, 105) et, au moyen de la lentille de reproduction (12) ou de l'objectif (12a), agencé(e) en aval dans la direction du passage du faisceau de rayons d'entrée, avec une surface de miroir de référence (7) avec ou sans couche de division de rayon (7a, 107a) de l'interféromètre à double rayon.

**20.** Système selon la revendication 19, en particulier un système multispectral interférométrique pour l'exploration en profondeur ou la saisie de profils d'objets de manière hautement dynamique,
**caractérisé en ce que**
un spectromètre (10) est agencé en aval de l'interféromètre à double rayon.

**21.** Système selon la revendication 19, en particulier un système multispectral interférométrique pour l'exploration en profondeur ou la saisie de profils d'objets de manière hautement dynamique,
**caractérisé en ce que**
un interféromètre à double rayon est agencé en aval de l'interféromètre à double rayon qui reproduit l'objet, lequel permet une analyse optique de la lumière d'interférence sur la sortie de l'interféromètre à double rayon qui reproduit l'objet.

Konfokale
Diskriminierung

P'

R_0_0, O_1_1

4

5

6

R_0

O_1

7, 7a

A

8

P

Fig. 1

P

A

$\lambda_1$

$\lambda_2$

$\lambda_3$

1. Beugungsordnung

$\lambda 1 < \lambda 2 < \lambda 3$

Fig. 2

Fig. 3

FFT

Fig. 4

Fig. 5

Fig. 6

1a 2 9 10 11

33

2a

P'

2b

4

O_1_1

5

12, 12a

P

O_1

8

R

15a

Fig. 7

$r = r\,(\lambda,\, z)$ bei O_1_1

Fig. 8

Fig. 9

Fig. 11

Fig. 10

Fig. 12

Fig. 13

1a  2  9  100  111a

215

Fig. 14a

33

2a

confokal
discrimination

2b

R

4

116  217a

55

12b

B209
TB209
209
208

blue
red

red

217b  215

blue

Fig. 14

123a  124a

red

122a

125

215

blue

stop

118

Δx blue

Δx red

Fig. 14b

Fig. 15

1, 1a

Interferometer

Spektrometer 100, 111a

Datenverarbeitung

Fokussier-System mit chromat. Komponente

12b

209

208

Z /depth)

λ

Wavelets

Fig. 16

Fig. 17

Fig. 18

Fig. 19

Fig. 20a

Fig. 20b

Fig. 20c

Fig. 20d

Fig. 20e

Fig. 20f

Fig. 20g

Fig. 21

Fig. 22

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4340306 A
- DE 10321895 A1
- GB 2355310 A
- US 6480285 B1


**In der Beschreibung aufgeführte Nicht-Patentliteratur**

- Spectral Radar: Optical Coherence tomography in the Fourier Domain. **M. W. LINDNER ; P. ANDRETZKY ; F. KIESEWETTER ; G. HÄUSLER.** Handbook of Optical Coherence Tomography. Marcel Dekker, Inc, 2002, 335-345
- **STEPHEN R. CHINN ; ERIC A. SWANSON.** Optical Coherence Tomography for High-Density Data Storage. vol. 1, 408
- **VON H. J. TIZIANI ; H.-M. UHDE.** Fachartikel Three-dimensional image sensing by chromatic confocal microscopy. *Applied Optics,* April 1994, vol. 33 (1), 1838-1843
- **KINO GS ; CHIM S.** Mirau correlation microscope. *Appl. Opt.,* 1990, vol. 29, 3775-3783
- **BYRON SL ; TIMOTHY CS.** Profilometry with a coherence scanning microscope. *Appl. Opt.,* 1990, vol. 29, 3784-3788
- **DRESEL TH ; HÄUSLER G ; VENZKE H.** Three-dimensional sensing of rough surfaces by coherence radar. *Appl. Opt.,* 1992, vol. 31, 919-925
- **DECK L ; DE GROOT P.** High-speed noncontact profiler based on scanning white-light interferometry. *Appl. Opt.,* 1994, vol. 33, 7334-7338
- **WINDECKER R ; HAIBLE P ; TIZIANI H J.** Fast coherence scanning interferometry for measuring smooth, rough and spherical surfaces. *J. Opt. Soc. Am,* 1995, vol. 42, 2059-2069
- **HEGE G.** Speckleverfahren zur Abstandsmessung. *Dissertation, in Berichte aus dem Institut für Technische Optik,* 1984, vol. 4, 20-25
- **VON J. SCHWIDER ; L. ZHOU.** Dispersive interferometric profilometer. *Opt. Lett.,* 1994, vol. 19 (13
- **VON G. LI ; P.-CH. SUN ; P. C. LIN ; Y. FEINMAN.** *Optics Letters 15. Okt.,* 2000, vol. 25 (20), 1505-1507
- **VON PETER HLUBINA.** Measuring distances and displacements using dispersive white-light spectral interferometry. *SPIE,* vol. 5144, 628-636
- **C. BOSBACH ; F. DEPRIEUX ; T. PFEIFER ; B. MICHELT.** *Proc. SPIE,* 2002, vol. 4900, 408-415
- **VON PAVEL PAVLICEK ; GERD HÄUSLER.** Accurate fiber-optic sensor for measurement of the distance based on white-light interferometry with dispersion. *ICO Tokyo,* 15. Juli 2004
- **VON MASATO OHMI ; AKIHIRO KAKIMOTO ; MASAMITSU HARUNA.** Fiber-optic in-focus OCT. *ICO Tokyo,* 13. Juli 2004
- **VON MEHTA, D., S. ; SAITO, S. ; HINOSUGI, H. ; TAKEDA, M. ; KUROKAWA, T.** Spectral interference Mirau microscope. *Applied Optics,* 01. Marz 2003, vol. 42 (7), 1296-1305
- **VON M. PAWLOWSKI ; Y. SAKANO ; Y. MIYAMOTO ; M. TAKEDA ; K. OBAYASHI.** Spatio-spectral digital holography for full-field tomografic imaging with adaptive focusing. *Proc. Of SPIE,* vol. 5531, 121-126
- Chromatic Confocal Spectral Interferometry. **VON E. PAPASTATHOPOULOS ; K. KÖRNER ; W. OSTEN.** Proceedings of Fringe 2005. Springerverlag, 11. September 2005
- *Applied Optics,* 10. Marz 2000, vol. 39 (8), 1290-1295
- Signalverarbeitung bei tiefen-scannenden 3D-Sensoren für neue industrielle Anwendungen. *Fachzeitschrift Technisches Messen,* 2002, vol. 69, 5
- *Applied Optics,* 10. Marz 2000, vol. 39, 1290-1295